# EUROPEAN PATENT APPLICATION

(11) **EP 3 903 789 A1**
(43) Date of publication of application: **03.11.2021**
(21) Application number: 19903001.6
(22) Date of filing: 27.12.2019
(51) Int. Cl.: A61K 31/573, A61K 45/00, A61P 27/02, A61K 35/30

(54) **THERAPEUTIC DRUG FOR DISEASE ACCOMPANIED BY DISORDERS IN RETINAL SYSTEM CELLS OR RETINAL TISSUE**

(30) Priority: 28.12.2018 JP 2018248350; 11.10.2019 JP 2019187999
(71) Applicant: RIKEN, Wako-shi Saitama 351-0198 (JP); Sumitomo Dainippon Pharma Co., Ltd., Chuo-ku Osaka-shi Osaka 541-8524 (JP); SUMITOMO CHEMICAL COMPANY LIMITED, Chuo-ku Tokyo 104-8260 (JP)
(72) Inventor: SUGITA Sunao, Wako-shi, Saitama 351-0198 (JP); MANDAI Michiko, Wako-shi, Saitama 351-0198 (JP); TAKAHASHI Masayo, Wako-shi, Saitama 351-0198 (JP); YAMASAKI Suguru, Kobe-shi, Hyogo 650-0047 (JP)
(74) Representative: Vossius & Partner Patentanwälte Rechtsanwälte mbB
(86) International application number: PCT/JP2019/051468
(87) International publication number: WO 2020/138430

(57) **Abstract**

The present invention relates to a therapeutic drug for a disease accompanied by a disorder in retinal cells or a retinal tissue, the drug including a retinal tissue, the retinal tissue being of an allogeneic origin and having a three-dimensional structure, wherein an intended patient to be given the therapeutic drug is a patient affected by a disease accompanied by a disorder of retinal cells or a retinal tissue, to be given systemically, for 1 month or more after the administration of the therapeutic drug, no immunosuppressive agent aimed at preventing graft rejection.

## Description

### Technical Field

The present invention relates to a therapeutic drug for a disease accompanied by a disorder in retinal cells or a retinal tissue. The present invention also relates to a method, a retinal tissue, and a kit for treating a disease accompanied by a disorder of retinal cells or a retinal tissue.

### Background Art

The intraocular region is considered to be an immune-privileged site, where excessive immune responses seldom occur to maintain the vision and the eye is recognized to be an organ where immunorejection seldom occurs. It is considered that the function as an immune-privileged site is accomplished by the blood-retinal barrier (BRB) composed of the inner blood-retinal barrier (inner BRB), which is substantially consist of retinal microvascular endothelial cells, and the outer blood-retinal barrier (outer BRB), which is substantially consist of retinal pigment epithelium (RPE) cells, separating the retina from the circulating blood and cytokines such as TGFβ secreted by the RPE cells. On the other hand, it is thought that the function as an immune-privileged site is weakened and immunorejection is prone to be evoked when the blood retina barrier is impaired by a disease of the retina, a grafting procedure, and the like, but details thereof are not known (Non Patent Literature 1).

In the case of graft of retinal pigment epithelial(RPE) cells, it has been proved in recent years that immune responses to a graft of retinal pigment epithelial(RPE) cells can be prevented without using any immunosuppressive agent when the major histocompatibility complex antigens (also referred to as MHC antigens, human leukocyte antigens (HLAs)) of the donor and the recipient match, while it has also been reported that immune responses are evoked by a graft of retinal pigment epithelial cells and the graft are rejected, in spite of the graft being performed in an immune-privileged site, when the MHC antigens (HLAs) of the donor and the recipient do not match (Non Patent Literature 2, 3).

Moreover, immunosuppressive agents such as tacrolimus (Non-Patent Literature 4) or prednisolone (Non-Patent Literature 5) are systemically administered by oral administration in graft of allogeneic retinal pigment epithelial cells with unmatched HLAs to humans (Non Patent Literature 4, 5).

### Citation List

### Non Patent Literature

Non Patent Literature 1: J. Wayne Streilein et al., Vision Research, Vol.42(2002), pp.487-495
Non Patent Literature 2: Sunao Sugita et al., Stem Cell Reports, Vol.7, pp.635-648, October 11, 2016
Non Patent Literature 3: Sunao Sugita et al., Stem Cell Reports, Vol.7, pp.619-634, October 11, 2016
Non Patent Literature 4: Steven D Schwartz et al., LANCET Vol.385, Issue.9967, 7-13 February 2015, pp.509-516
Non Patent Literature 5: Lyndon da Cruz et al., Nature biotechnology, Vol.36, pp.328-337 (2018)

### Summary of Invention

### Technical Problem

As described above, it is considered that the administration of an immunosuppressive agent is necessary even when a retinal tissue of an allogeneic origin (donor) is grafted to a human. However, systemic administration of an immunosuppressive agent particularly has possibilities of serious side effects such as increasing the risk of oncogenic transformation and causing a serious infection and such administration to elderly persons and cancer patients with lowered immunity particularly has been considered problematic. Moreover, a long-term use of immunosuppressive agent increases the risk of the aforementioned side effects and the like and markedly lowers the quality of life (QOL) of patients.

Therefore, the present invention is directed to providing a therapeutic drug for a disease accompanied by a disorder in retinal cells or a retinal tissue, wherein the drug includes a retinal tissue being of an allogeneic origin and unlikely to cause rejection.

### Solution to Problem

The present inventors found that retinal tissues forming cell aggregates and having a three-dimensional structure induce no immune responses in a recipient whose MHC (HLA) type does not match that of the donor, but rather suppress the immune activation, while dispersed retinal cells forming no aggregates induce immune responses in the recipient. The present invention is based on these novel findings.

Accordingly, the present invention relates to the following inventions.
[1] A therapeutic drug for a disease accompanied by a disorder in retinal cells or a retinal tissue, wherein the drug includes a retinal tissue being of an allogeneic origin and having a three-dimensional structure, wherein an intended patient to be given the therapeutic drug is a patient affected by a disease accompanied by a disorder of retinal cells or a retinal tissue, to be given systemically no immunosuppressive agent other than a steroidal anti-inflammatory drug and/or a calcineurin inhibitor (CNI) aimed at preventing graft rejection, before, during, and/or after the administration of the therapeutic drug.
[2] The therapeutic drug according to [1], wherein the intended patient is a patient to be given systemically none of a steroidal anti-inflammatory drug and/or a calcineurin inhibitor, aimed at preventing graft rejection at least 1 month after the administration of the therapeutic drug.
[3] A therapeutic drug for a disease accompanied by a disorder in retinal cells or a retinal tissue, wherein the drug includes a retinal tissue being of an allogeneic origin and having a three-dimensional structure, wherein an intended patient to be given the therapeutic drug is a patient affected by a disease accompanied by a disorder of retinal cells or a retinal tissue, to be given systemically no immunosuppressive agent aimed at preventing graft rejection for a long period of time (for example, for 1 month or more after the administration of the therapeutic drug), before, during, and/or after the administration of the therapeutic drug,.
[4] The therapeutic drug according to any one of [1] to [3], wherein the intended patient is a patient to be given systemically no immunosuppressive agent aimed at preventing graft rejection, before, during, and/or after the administration of the therapeutic drug.
[5] The therapeutic drug according to any one of [1] to [4], wherein the intended patient is a patient to be given locally no immunosuppressive agent (preferably, a steroidal anti-inflammatory drug) other than a steroidal anti-inflammatory drug and a calcineurin inhibitor aimed at preventing graft rejection, before, during, and/or after the administration of the therapeutic drug.
[6] The therapeutic drug according to any one of [1] to [5], wherein the intended patient is a patient to be given locally one or more immunosuppressive agents selected from the group consisting of triamcinolone, fluocinolone, and a calcineurin inhibitor aimed at preventing graft rejection, before, during, and/or after the administration of the therapeutic drug.
[7] The therapeutic drug according to [5], wherein the intended patient is a patient to be given locally none of a steroidal anti-inflammatory drug and/or a calcineurin inhibitor aimed at preventing graft rejection, at least 1 month after the administration of the therapeutic drug,.
[8] The therapeutic drug according to any one of [1] to [3], wherein the intended patient is a patient to be given no immunosuppressive agent aimed at preventing graft rejection, before, during, and/or after the administration of the therapeutic drug.
[9] The therapeutic drug according to any one of [1] to [8], wherein the therapeutic drug includes substantially no retinal pigment epithelial cells.
[10] The therapeutic drug according to any one of [1] to [9], wherein TGFβ is present at least on the surface of the retinal tissue.
[11] The therapeutic drug according to any one of [1] to [10], wherein the retinal tissue has a diameter in the major axis direction of 0.2 mm or more.
[12] The therapeutic drug according to any one of [1] to [11], wherein the total number of cells contained in the retinal tissue is 1 × 10⁴ cells or more per tissue.
[13] The therapeutic drug according to any one of [1] to [12], wherein the surface of the retinal tissue has 50% or more by area of a continuous epithelial structure.
[14] The therapeutic drug according to any one of [1] to [13], wherein 50% or more of the total number of cells contained in the retinal tissue are cells expressing at least one of PAX6, Chx10, and Crx.
[15] The therapeutic drug according to any one of [1] to [14], wherein the retinal tissue is derived from a pluripotent stem cell.

### Advantageous Effects of Invention

According to the present invention, it becomes possible to provide a therapeutic drug for a disease accompanied by a disorder in retinal cells or a retinal tissue, wherein the drug includes a retinal tissue being of an allogeneic origin, but unlikely to cause rejection because of exhibiting immunosuppressive properties. Thereby, it becomes possible to select a method that imposes less burden of a combined application of immunosuppressive agents in graft on the patient, for example, a method not involving systemic administration of an immunosuppressive agent or the like.

### Brief Description of Drawings

[Figure 1] Figure 1 is a figure showing photomicrographs of neural retina, each differentiated from a human ES cell line (khES-1) and a human iPS cell line (TLHD2) in Example 1.
[Figure 2A] Figure 2A is graphs showing results of HLA expression analysis in neural retina differentiated from human ES cells in Example 2.
[Figure 2B] Figure 2B is graphs showing results of HLA expression analysis in neural retina differentiated from human iPS cells in Example 2.
[Figure 3] Figure 3 is photographs showing results of the observation of the expression of HLA class I in neural retina differentiated from human ES cells in Example 3.
[Figure 4] Figure 4 is photographs showing results of the observation of the expression of HLA class II in neural retina differentiated from human iPS cells in Example 3.
[Figure 5A] Figure 5A is photographs showing results of the observation of expression of HLA class I and HLA class II after the graft of neural retina differentiated from human ES cells in Example 4.
[Figure 5B] Figure 5B is photographs showing a result of staining with hGFAP after the graft of neural retina differentiated from human ES cells in Example 4.
[Figure 6] Figure 6 is photographs showing samples for the evaluation of the ability to suppress the activation of immune cells in Example 5. (1) represents a neural retina (Whole 3D retina), as it is, differentiated from human ES cells; (2) represents semi dissociates of the neural retina of (1) (semi dissociate); and (3) represents dispersed single cells (single) of the neural retina of (1).
[Figure 7A] Figure 7A is a figure showing results of immunogenic tests for immune cells using neural retina differentiated from human ES cells in Example 5.
[Figure 7B] Figure 7B is a figure showing results of immunogenic tests for immune cells using neural retina differentiated from human ES cells in Example 5.
[Figure 8] Figure 8 is a figure showing results of immunogenic tests for immune cells using neural retina differentiated from human ES cells in Example 5.
[Figure 9A] Figure 9A is a figure showing results of immunogenic tests for immune cells using neural retina differentiated from human iPS cells in Example 5.
[Figure 9B] Figure 9B is a figure showing results of immunogenic tests for immune cells using neural retina differentiated from human iPS cells in Example 5.
[Figure 10] Figure 10 is a figure showing results of immunogenic tests for immune cells using neural retina differentiated from human iPS cells in Example 5.
[Figure 11A] Figure 11A shows results of the evaluation of the ability of neural retina differentiated from human iPS cells to suppress activated immune cells in Examples 6-7.
[Figure 11B] Figure 11B shows results of the evaluation of the ability of neural retina differentiated from human iPS cells to suppress activated immune cells in Examples 6-7.
[Figure 12] Figure 12 shows results of the evaluation of the ability of neural retina differentiated from human iPS cells to suppress activated immune cells in Examples 6-7.
[Figure 13] Figure 13 shows results of the evaluation of the ability of neural retina differentiated from human ES cells to suppress activated immune cells in Examples 6-7.
[Figure 14] Figure 14 shows results of the evaluation of the ability of neural retina differentiated from human ES cells to suppress activated immune cells in Examples 6-7.
[Figure 15] Figure 15 is a figure showing results of expression analysis of TGFβ in Example 8.
[Figure 16] Figure 16 is photographs showing the expression of TGFβ in a neural retina in Example 9.
[Figure 17] Figure 17 is a figure showing results of the measurement of amount of TGFβ2 secretion by ELISA in Example 10.
[Figure 18] Figure 18 is a figure showing results of the measurement of amount of TGFβ2 secretion in Example 11.
[Figure 19] Figure 19 is photographs showing the localization of TGFβ2 in Example 11.
[Figure 20A] Figure 20A is a figure showing results of MLR tests using neural retina on Day 50, Day 100, Day 160, and Day 200 after starting floating culture in Example 12.
[Figure 20B] Figure 20B is a figure showing results of MLR tests using neural retina on Day 50, Day 100, Day 160, and Day 200 after starting floating culture in Example 12.
[Figure 21] Figure 21 is a figure showing results of MLR tests using neural retina on Day 50, Day 100, Day 160, and Day 200 after starting floating culture in Example 12.
[Figure 22] Figure 22 is a figure showing results of immunogenic tests in Example 13.
[Figure 23] Figure 23 is a figure showing results of immunogenic tests in Example 13.
[Figure 24] Figure 24 is a figure showing a result of microscopy of monkey neural retina differentiated from human iPS cells in Example 14.
[Figure 25] Figure 25 is a figure showing results of immunogenicity evaluation of a neural retina differentiated from monkey iPS cells in Example 15.
[Figure 26] Figure 26 is confocal photomicrographs showing results of immunostaining of sections in which a neural retina differentiated from monkey iPS cells was grafted, in Example 16.
[Figure 27] Figure 27 is confocal photomicrographs showing results of immunostaining of sections in which a neural retina differentiated from monkey iPS cells was grafted, in Example 16.
[Figure 28] Figure 28 is a figure showing results of the evaluation of the ability of neural retina differentiated from monkey iPS cells to suppress activated immune cells in Example 17.
[Figure 29] Figure 29 is photomicrographs showing results of immunostaining of neural retina hESC-NR (A-C) differentiated from human ES on Day 80 of differentiation and neural retina hiPSC-NR (D-F) differentiated from human iPS cells. A and D show results of confirmation of expression of CRX, Chx10, and Pax6; B and E show results of confirmation of expression of CRX, Rxr-γ, and Brn3; and C and F show results of confirmation of expression of Islet-1, Prox1, and Brn3.
[Figure 30] Figure 30 is a figure showing results of flow cytometry analysis of HLA class I, β2-Microglobulorin, and HLA class II in hES-NR, hiPSC-NR, and hiPSC-RPE cells cultured for 2 days in the presence or the absence of an IFN-y.
[Figure 31] Figure 31 is a figure showing results of flow cytometry analysis of HLA class I and HLA class II in the Crx::Venus-positive fraction of hESC-NR and hESC-NR cultured for 2 days in the presence or the absence of IFN-y.
[Figure 32] Figure 32 is a figure showing results of flow cytometry analysis of β2-Microglobulorin, HLA-E, CD40, CD80, CD86, PD-L1, PD-L2 in hESC-NR and hiPSC-NR cultured for 2 days in the presence or the absence of IFN-y.
[Figure 33] Figure 33 is a figure showing results of flow cytometry analysis of CD47 in hESC-NR and hiPSC-NR cultured for 2 days in the presence or the absence of an IFN-y.
[Figure 34] Figure 34 is photomicrographs showing results of immunostaining of Crx::Venus and HLA class I in hESC-NR with no INF-γ stimulation.
[Figure 35] Figure 35 is photomicrographs showing results of immunostaining of Crx::Venus and HLA class I in hESC-NR with the INF-γ stimulation.
[Figure 36] Figure 36 is a figure showing results of immunostaining of Crx::Venus and HLA class I in hESC-NR with the INF-γ stimulation and with no INF-γ stimulation at the same time.
[Figure 37] Figure 37 is graphs showing HLA class I expression levels in a neural retina (NR) or single cells (hESCs) in the presence or the absence of IFN-y.
[Figure 38] Figure 38 is a graph showing results of the examination of immunosuppressive capacity of hESC-NR using the proliferation of peripheral blood mononuclear cells (PBMCs) activated with a CD3/28 antibody as an indicator.
[Figure 39] Figure 39 is photomicrographs showing results of the observation of immunosuppressive capacity of hESC-NR using the proliferation and aggregation of peripheral blood mononuclear cells (PBMCs) activated with a CD3/28 antibody as an indicator.
[Figure 40] Figure 40 is a figure showing results of the examination of dose-dependency of the activation-suppressing effect of hES-NR (upper panels)/hiPSC-NR (lower panels) on CD4-positive T cells and CD8-positive T cells.
[Figure 41] Figure 41 is a figure showing results on the immunosuppression pattern of hESC-NR when separately cocultured using Transwell (R).
[Figure 42] Figure 42 is a graph showing the extent of expression of TGF-β1, TGF-β2, and TGF-β3 in hiPSC-NR, iPS cells, and iPS-RPE cells.
[Figure 43] Figure 43 is a graph showing the dose-dependency of TGF-β2 expression by hiPSC-NR.
[Figure 44] Figure 44 is a graph showing the relation between the amount of TGF-β2 expression and days of differentiation.
[Figure 45] Figure 45 is photomicrographs showing the HLA expression pattern after the graft in Example 19.
[Figure 46] Figure 46 is photomicrographs showing the HLA expression pattern after the graft in Example 19.
[Figure 47] Figure 47 is graphs showing results of the examination of immunogenicity, to peripheral blood mononuclear cells (PBMCs), of hESC-NR treated with INF-γ leading to an increased expression of HLA-Class I.

### Description of Embodiments

### [Therapeutic drug]

The therapeutic drug herein is a therapeutic drug for a disease accompanied by a disorder in retinal cells or a retinal tissue, wherein the drug includes a retinal tissue being of an allogeneic origin and having a three-dimensional structure. The retinal tissue may be a retinal tissue differentiated from allogeneic stem cells and having a three-dimensional structure. An intended patient to be given the therapeutic drug may be a patient affected by a disease accompanied by a disorder of retinal cells or a retinal tissue, to be given systemically for a long period of time (for example, for 1 month or more after the administration of the therapeutic drug), before, during, and/or after the administration of the therapeutic drug, no immunosuppressive agent aimed at preventing graft rejection. The intended patient may be a patient to be given systemically, before, during, and/or after the administration of the therapeutic drug, no immunosuppressive agent aimed at preventing graft rejection, other than a steroidal anti-inflammatory drug and a cyclosporine.

Examples of the disease accompanied by a disorder in retinal cells or a retinal tissue include eye diseases such as macular degeneration, age-related macular degeneration(AMD), retinitis pigmentosa(RP), glaucoma, a corneal disease, retinal detachment, central serous chorioretinopathy, cone dystrophy, cone-rod dystrophy, and macular hole. The diseases accompanied by a disorder in retinal cells or a retinal tissue also include diseases accompanied by the damage of a retinal tissue. Examples of the condition of retinal tissue damage include the condition of degenerative photoreceptor cell death.

### (Allogeneic stem cells)

The term "allogeneic" herein means a human other than the patient (recipient) to be treated, that is, another person (donor). The "allogeneic stem cells" refers to stem cells derived from a human other than the patient (recipient), that is, stem cells derived from another person (donor).

The term "stem cells" refers to cells that have both multipotency (capability to differentiate into multiple types of cells) and self-renewal capacity and are capable of proliferating. Examples of the stem cells include pluripotent stem cells such as embryonic stem cells (ES cells) and induced pluripotent stem cells (iPS cells) artificially generated from cells derived from bone marrow, blood, or skin (epidermal, dermal, or subcutaneous tissue) by introduction of reprogramming genes, or the like; and somatic stem cells, which reside in the bone marrow, fat, hair follicles, brain, nerve, liver, pancreas, kidney, muscle, and other tissues and differentiate into a plurality of specific types of cells. It is preferred that allogeneic stem cells induced to differentiate in the present embodiment are pluripotent stem cells.

The "pluripotent stem cells" are not particularly limited, as long as they are stem cells having pluripotency that allows differentiation into a plurality of cells present in the living body, preferably into all cells other than extraembryonic tissues such as the placenta and also having proliferative capacity. The pluripotent stem cells can be induced from a fertilized egg, a clonal embryo, germline stem cells, tissue stem cells, somatic cells, and the like. Examples of the pluripotent stem cells include embryonic stem cells (ES cells), EG cells (Embryonic germ cells), and induced pluripotent stem cells (iPS cells). Muse cells (multi-lineage differentiating stress enduring cells) obtained from mesenchymal stem cells (MSCs) and spermatogenic stem cells (GS cell) generated from germ cells (for example, the testis) are also included in pluripotent stem cells. The embryonic stem cells were established in 1981 for the first time and have also been applied to the production of knockout mice since 1989. Human embryonic stem cells were established in 1998 and are increasingly used also in regenerative medicine. The embryonic stem cells can be produced by culturing the internal cell mass with feeder cells or in a medium containing LIF (leukemia inhibitory factor). Methods for producing embryonic stem cells are described in, for example, WO96/22362, WO02/101057, US5843780, US6200806, US6280718, and the like. Embryonic stem cells are available from certain organizations and can also be purchased as commercial products. For example, KhES-1, KhES-2, and KhES-3, which are human embryonic stem cells, are available from Institute for Frontier Medical Sciences, Kyoto University. The cell lines EB5 and D3, which are murine embryonic stem cells, are respectively available from Institute of Physical and Chemical Research and ATCC.

The nuclear transplantation embryonic stem cells (ntES cells), which are one of the embryonic stem cells, can be established from clonal embryos produced by transplanting a somatic nucleus to an ovum from which the nucleus is removed.

The EG cells can be produced by culturing primordial germ cells in a medium containing mSCF, LIF, and bFGF (Cell, 70: 841-847, 1992).

The "induced pluripotent stem cells" are cells in which pluripotency is induced by reprogramming somatic cells, e.g., by a known method. Specific examples thereof include cells in which pluripotency is induced by reprogramming differentiated somatic cells such as fibroblasts or peripheral blood mononuclear cells by expressing a combination of a plurality of genes selected from reprogramming genes including Oct3/4, Sox2, Klf4, Myc (c-Myc, N-Myc, L-Myc), Glis1, Nanog, Sall4, Lin28, and Esrrb. Examples of a preferred combination of reprogramming factors include (1) Oct3/4, Sox2, Klf4, and Myc (c-Myc or L-Myc), or (2) Oct3/4, Sox2, Klf4, Lin28, and L-Myc (Stem Cells, 2013; 31: 458-466).

Induced pluripotent stem cells were established with murine cells in 2006 by Yamanaka et al. (Cell, 2006, 126 (4), pp.663-676). Induced pluripotent stem cells were established also with human fibroblasts in 2007 and have pluripotency and self-renewal capacity like embryonic stem cells (Cell, 2007, 131 (5), pp.861-872; Science, 2007, 318 (5858), pp.1917-1920; Nat. Biotechnol., 2008, 26 (1), pp.101-106).

Induced pluripotent stem cells can be produced also by a method of obtaining induced pluripotent stem cells from somatic cells induced by addition of a compound or the like (Science, 2013, 341, pp.651-654), besides methods of production by reprogramming directly by gene expression.

Induced pluripotent stem cells can be obtained as established induced pluripotent stem cells. For example, human induced pluripotent cell lines such as 201B7 cells, 201B7-Ff cells, 253G1 cells, 253G4 cells, 1201C1 cells, 1205D1 cells, 1210B2 cells, 1231A3 cells, and the like established at Kyoto University are available from Kyoto University. As established induced pluripotent stem cells, for example, Ff-I01 cells, Ff-I01s04 cells, QHJ-I01 and Ff-I14 cells established at Kyoto University are available from Kyoto University. Moreover, for example, the cell line TLHD2 established at Institute of Physical and Chemical Research is available from Institute of Physical and Chemical Research.

Examples of the somatic cells to be used in the production of induced pluripotent stem cells include, but not particularly limited, fibroblasts derived from tissue, hematopoietic cells (for example, peripheral blood mononuclear cells (PBMCs), umbilical cord blood mononuclear cells, T cells, and the like), hepatic cells, pancreatic cells, intestinal epithelial cells, and smooth muscle cells.

When reprogramming is performed by expression of several genes in the production of induced pluripotent stem cells, the means for expressing the genes is not particularly limited. Examples of such means include infection methods using a virus vector (for example, a retroviral vector, a lentiviral vector, a Sendai virus vector, an adenoviral vector, or an adeno-associated virus vector); gene transfer methods (for example, the calcium phosphate method, the lipofection method, the RetroNectin method, or electroporation) using a plasmid vector (for example, a plasmid vector or an episomal vector); gene transfer methods using an RNA vector (for example, the calcium phosphate method, the lipofection method, or electroporation); and protein-direct injection methods (for example, methods using a needle, lipofection method or electroporation).

Induced pluripotent stem cells can be produced in the presence of feeder cells or in the absence of feeder cells (feeder-free). In the production of induced pluripotent stem cells in the absence of feeder cells, the induced pluripotent stem cells can be produced in the presence of a factor for maintaining undifferentiated state by a known method. The medium to be used in the production of induced pluripotent stem cells in the absence of feeder cells is not particularly limited, and examples thereof include maintenance media for known embryonic stem cells and/or induced pluripotent stem cells or media for establishing induced pluripotent stem cells in feeder-free conditions. Examples of the media for establishing induced pluripotent stem cells in feeder-free conditions include feeder-free medium such as the Essential 8 medium (E8 medium), the Essential 6 medium, the TeSR medium, the mTeSR medium, the mTeSR-E8 medium, the Stabilized Essential 8 medium, and the StemFit medium. In the production of induced pluripotent stem cells, the induced pluripotent stem cells can be produced, for example, by genetically introducing the four factors: Oct3/4, Sox2, Klf4, and Myc into somatic cells in feeder-free conditions using a Sendai virus vector.

The pluripotent stem cells may be human pluripotent stem cells and are preferably human induced pluripotent stem cells (iPS cells) or human embryonic stem cells (ES cells).

The HLA genotype of the allogeneic stem cells may be the same as or different from that of the recipient. In the major histocompatibility complex antigen (MHC) region present in the short arm of the sixth chromosome, many HLA loci (HLA-A, B, C, DR, and the like) are located and there is a plurality (dozens) of HLA types (alleles) for each locus. HLA is a membrane-bound glycoprotein and involved in the recognition of self and non-self by presenting antigens to immune cells such as T cells. The recognition of self and non-self mainly involves HLA class 1 and HLA class 2. HLA class 1 (MHC class 1) is mainly recognized by killer T cells and HLA class 2 (MHC class 2) is mainly recognized by helper T cells. Antigens that HLA class 1 (MHC class 1) presents are peptide fragments generated by the degradation of proteins synthesized in cells into small fragments by proteasomes. On the other hand, antigens that MHC class 2 presents are peptide fragments generated by fragmenting exogenous proteins taken up by endocytosis into small fragments by lysosomes.

### (Retinal tissue)

The "retinal tissue" means a tissue in which one or more types of retinal cells that constitute either of the retinal layers in the retina in vivo are located according to a certain arrangement and the "neural retina" means a retinal tissue containing the inner neural retinal layer and no retinal pigment epithelial layer, among the retinal layers described below.

The term "retinal cells" means cells constituting a retinal layer(s) in the retina in vivo or progenitor cells thereof. Examples of the retinal cells include, but are not limited to, cells such as photoreceptor cells (rod photoreceptor cells, cone photoreceptor cells), horizontal cells, amacrine cells, interneurons, retinal ganglion cells (ganglion cells), bipolar cells (rod bipolar cells, cone bipolar cells), Muller glial cells, retinal pigment epithelial (RPE) cells, ciliary marginal zone cells, progenitor cells thereof (examples: photoreceptor progenitor cells, progenitor cells of bipolar cells), and retinal progenitor cells. Specific examples of cells that constitute the neural retinal layer among the retinal cells include photoreceptor cells (rod photoreceptor cells, cone photoreceptor cells), horizontal cells, amacrine cells, interneurons, retinal ganglion cells (ganglion cells), bipolar cells (rod bipolar cells, cone bipolar cells), Muller glial cells, and progenitor cells thereof (examples: photoreceptor progenitor cells, bipolar progenitor cells).

The aforementioned retinal cells can be detected or identified respectively using markers expressed in the cells as indicators.

Examples of the markers expressed in the cells include Rx (also referred to as Rax) and PAX6 expressed in retinal progenitor cells, Rx, PAX6, and Chx10 expressed in neural retina progenitor cells, and Crx and Blimp 1 expressed in photoreceptor progenitor cells.

Examples of other markers include Chx10 strongly expressed in bipolar cells, PKCα, Goα, VSX1 expressed in bipolar cells and L7, TuJI and Brn3 expressed in retinal ganglion cells, Calretinin and HPC-1 expressed in amacrine cells, Calbindin expressed in horizontal cells, Recoverin expressed in photoreceptor cells and photoreceptor progenitor cells, Rhodopsin expressed in rod cells, Nrl expressed in rod photoreceptor cells and rod photoreceptor cell progenitor cells, S-opsin and LM-opsin expressed in cone photoreceptor cells, RXR-γ expressed in cone cells, cone photoreceptor cell progenitor cells, and ganglion cells, TRβ2, OTX2, and OC2 expressed in cone photoreceptor cells that emerge early in the differentiation, among cone photoreceptor cells, or progenitor cells thereof, Pax6 expressed commonly in horizontal cells, amacrine cells, and ganglion cells, RPE65 and Mitf expressed in retinal pigment epithelial cells, CRALBP expressed in Muller cells, and GS expressed in Muller glial cells.

The term "retinal layer(s)" means a layer(s) constituting the retina and specific examples thereof include the retinal pigment epithelial layer, the photoreceptor cell layer, the outer limiting membrane, the outer nuclear layer, the outer plexiform layer, the inner nuclear layer, the inner plexiform layer, the ganglion cell layer, the nerve fiber layer, and the inner limiting membrane.

The "neural retinal layer" means a layer(s) constituting the neural retina and specific examples thereof include the photoreceptor cell layer, the outer limiting membrane, the outer nuclear layer, the outer plexiform layer, the inner nuclear layer, the inner plexiform layer, the ganglion cell layer, the nerve fiber layer, and the inner limiting membrane. The "photoreceptor cell layer" means a retinal layer that is one of the retinal layers (neural retinal layers), formed at the outermost of the neural retina, and contains a lot of photoreceptor cells (rod photoreceptor cells, cone photoreceptor cells), photoreceptor progenitor cells, and retinal progenitor cells. Which retinal layer each kind of cells constitutes can be determined by a known method, for example, the presence or absence of expression or the expression level of a cell marker.

Since the retinal tissue in the therapeutic drug in the present embodiment has a three-dimensional structure, it does not require systemic administration of an immunosuppressive agent aimed at preventing graft rejection, other than a steroidal anti-inflammatory drug and a calcineurin inhibitor, before, during, and/or after the administration of the therapeutic drug, despite being of an allogeneic origin (for example, that differentiated from human iPS cells, human ES cells, or allogeneic stem cells).

The retinal tissue in the therapeutic drug of the present embodiment has a three-dimensional structure. The expression "has a three-dimensional structure" means that a certain number or more of retinal cells form aggregates, the retinal cells constitute a retinal layer(s) in the aggregates, the retinal cells are layered in the retinal layer(s), and it has an epithelial structure in which the polarity of an apical surface and a basal membrane (basal surface) is maintained at least in a range of tissue. In other words, the expression "a retinal tissue has a three-dimensional structure" means that the retinal tissue is a cell aggregate(s) (also referred to as an organoid(s)) containing retinal cells. Zo-1, β-catenin, Atypical PKC, and the like are expressed in the apical surface. Collagen, Laminin, and the like are expressed in the basal membrane.

In one aspect, the retinal tissue is a sphere-shaped cell aggregate(s). The "sphere-shaped cell aggregate" means a cell aggregate having a three-dimensional shape close to a spherical shape. The three-dimensional shape close to a spherical shape is a shape having a three-dimensional structure which is a spherical shape having a projection on a two-dimensional plane, for example, in a circle or oval shape and a shape formed by fusing a plurality of spherical shapes (for example, a shape having a projection on a two-dimensional plane in a shape formed by fusing 2-4 circle or oval shapes with overlap (example: a clover shape)). In one aspect, the sphere-shaped cell aggregate has a vesicular structure and is characterized in that it is observed with a dark central part and a bright circumference part under blight-field microscope.

The retinal tissue herein may comprise substantially no retinal pigment epithelial (RPE) cells. The term "retinal pigment epithelial cells" means epithelial cells that reside outside of the neural retina in the retina in vivo. Whether a cell is a retinal pigment epithelial cell can easily be determined by a person skilled in the art based on, for example, the expression of a cell marker(s) (RPE65, Mitf, CRALBP, MERTK, BEST1, and/or the like), the presence of melanin granules (blackish brown), tight junctions between cells, and/or a characteristic polygonal/cobblestone-like cell shape, and/or the like. Whether a cell has the function of retinal pigment epithelial cells can easily be determined based on the capability of secreting cytokines such as VEGF and PEDF. In one aspect, the retinal pigment epithelial cells are RPE65-positive cells, Mitf-positive cells, or RPE65-positive and Mitf-positive cells. In other words, the retinal tissue may include substantially no RPE65-positive cells, Mitf-positive cells, or RPE65-positive and Mitf-positive cells.

As used herein, the expression "comprise substantially no retinal pigment epithelial cells" means that it is not stained with the aforementioned marker of retinal pigment epithelial cells (example: RPE65 and/or Mitf) or cells stained with the marker is 5% or less (preferably, 4% or less, 3% or less, 2% or less, or 1% or less) of all cells.

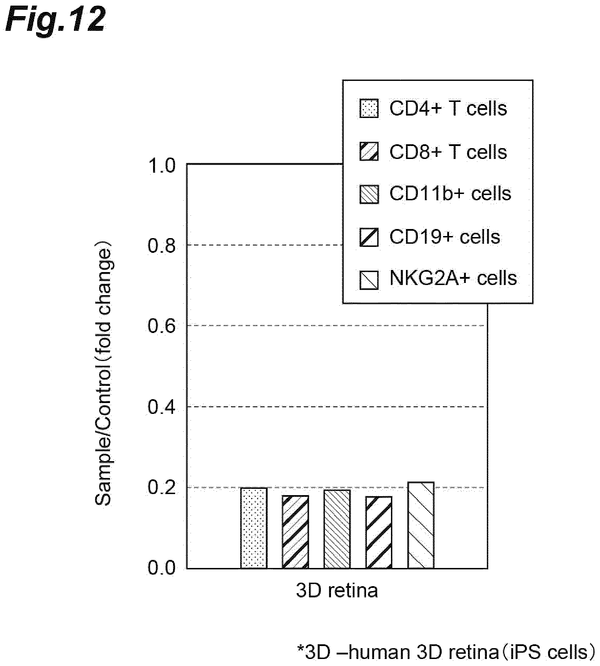

As another aspect of the aforementioned "sphere-shaped cell aggregate", a partial region(s) of the aggregate may include retinal pigment epithelial cells and/or a ciliary marginal zone-like structure. As one aspect, a part of a continuous boundary surface (constituted of the neural retina) formed to the external environment of the cell aggregate is constituted of retinal pigment epithelial cells and a ciliary marginal zone-like structure body is present in a border region of the neural retina and retinal pigment epithelial cells. In other words, the retinal pigment epithelium and the neural retina adjoin the ciliary marginal zone-like structure along the circumference in the same cell aggregate. Examples include cell aggregates disclosed in WO2013/183774 (for example, Figure 12A in WO2013/183774). In the case of a retinal tissue including retinal pigment epithelial cells and a ciliary marginal zone-like structure, a cell aggregate (a retinal tissue) can be used as a therapeutic drug in which a region not including retinal pigment epithelial cells and the ciliary marginal zone-like structure has been cut out by a method described below.

The ciliary marginal zone-like structure is a structure that resembles the ciliary marginal zone. Examples of the "ciliary marginal zone (CMZ)" include a region of a tissue that is in the border region between the neural retina and the retinal pigment epithelium in the retina in vivo, and the region containing tissue stem cells (retinal stem cells) in the retina. The ciliary marginal zone is also called the ciliary margin or the retinal margin, and the ciliary marginal zone, the ciliary margin, and the retinal margin are equivalent tissues. It is known that the ciliary marginal zone plays important roles in the supply of retinal progenitor cells/differentiated cells to retinal tissue, the maintenance of retinal tissue structure, and the like. Examples of the marker gene of the ciliary marginal zone include the Rdh10 gene (positive), the Otx1 gene (positive), and the Zic1 (positive).

The diameter in the major axis direction of the retinal tissue may be 0.1 mm or more or 0.2 mm or more (preferably 0.3 mm or more, 0.5 mm or more, 0.8 mm or more, 1.0 mm or more, 1.2 mm or more, 1.4 mm or more, 1.6 mm or more, 1.8 mm or more, 2.0 mm or more). There is no upper limit of the diameter in the major axis direction of retinal tissue in particular, but the diameter in the major axis direction may for example be 5.0 mm or less or 10 mm or less. The diameter in the major axis direction of the retinal tissue may be 0.1 mm or more and 10 mm or less, 0.2 mm or more and 10 mm or less, 0.3 mm or more and 10 mm or less, 0.5 mm or more and 10 mm or less, 0.8 mm or more and 10 mm or less, 1.0 mm or more and 10 mm or less, 1.2 mm or more and 5.0 mm or less, 1.4 mm or more and 5.0 mm or less, 1.6 mm or more and 5.0 mm or less, 1.8 mm or more and 5.0 mm or less, or 2.0 mm or more and 5.0 mm or less. In one aspect of the present invention, the diameter in the major axis direction of the retinal tissue may be about 1.2 fold, 1.5 fold, or 2 fold of the diameter in the minor axis direction. In another aspect, the diameter in the major axis direction and the diameter in the minor axis direction of the retinal tissue may be approximately equivalent.

The diameter in the minor axis direction of the retinal tissue may be 0.05 mm or more (preferably 0.1 mm or more, 0.2 mm or more, 0.4 mm or more, 0.6 mm or more, 0.8 mm or more, or 1.0 mm or more). There is no upper limit of the diameter in the minor axis direction of retinal tissue in particular, but the diameter in the minor axis direction may for example be 2.5 mm or less or 5 mm or less. The diameter in the minor axis direction of the retinal tissue may be 0.05 mm or more and 5 mm or less, 0.1 mm or more and 5 mm or less, 0.2 mm or more and 5 mm or less, 0.4 mm or more and 5 mm or less, 0.6 mm or more and 2.5 mm or less, 0.8 mm or more and 2.5 mm or less, or 1.0 mm or more and 2.5 mm or less.

Here, the diameter in the major axis direction or the minor axis direction of the retinal tissue means, for example, in the case of measuring the diameters based on an image photographed using a stereoscopic microscope, the length of the longest straight line (the diameter in the major axis direction) or the length of the shortest straight line (the diameter in the minor axis direction) among straight lines linking any two points on the circumference (outline, surface) of the apical surface of the retinal tissue. In an aggregate containing the retinal tissue, a plurality of retinal tissues may exist with overlap (example: a clover shape). In this case, the diameter in the major axis direction or diameter in the minor axis direction of retinal tissue means the diameter in the major axis direction or the minor axis direction in each retinal tissue in the aggregate and at least one of the retinal tissues may have a diameter in the major axis direction or the minor axis direction within the aforementioned range. In the case of a clover shape retinal tissue, specifically, the length measurement is made on the longest straight line or the shortest straight line among straight lines linked any two points on the circumference segmented by the points where two circles or ovals cross in the geometric point of view (more specifically, the points where, when continuous positions on the circumference of the aggregate containing the retinal tissue are arbitrarily determined, the continuity of the curve obtained by plotting the slope of the tangent line at the position as the ordinate versus the position as the abscissa is lost), assuming that circumference (outline, surface, apical surface) parts of the retinal tissue are circles or ovals. Whether an aggregate contains a plurality of retinal tissues can be determined easily by a person skilled in the art based, for example, on the observation under microscope.

The thickness of the epithelial structure of the retinal tissue may be 0.05 mm or more (preferably 0.1 mm or more, 0.12 mm or more, 0.15 mm or more, 0.2 mm or more, 0.25 mm or more, or 0.3 mm or more). There is no upper limit of the thickness of the epithelial structure of the retinal tissue in particular, but the thickness may be, for example, 1 mm or less (for example, 0.9 mm or less, 0.8 mm or less, 0.7 mm or less, 0.6 mm or less, or 0.5 mm or less). The thickness of the epithelial structure of the retinal tissue may be, for example, 0.05 mm or more and 1 mm or less, 0.1 mm or more and 0.9 mm or less, 0.12 mm or more and 0.8 mm or less, 0.15 mm or more and 0.7 mm or less, 0.2 mm or more and 0.6 mm or less, 0.25 mm or more and 0.5 mm or less, or 0.3 mm or more and 0.5 mm or less. Here, the thickness of the epithelial structure of the retinal tissue means the thickness from the apical surface of the retinal tissue to the basal membrane.

The total number of cells contained in one piece of the retinal tissue depends on the size of the retinal tissue. The total number of cells contained in one piece of the retinal tissue may be 1 × 10⁴ cells or more, 1 × 10⁵ cells or more, 5 × 10⁵ cells or more, or 1 × 10⁶ cells or more. For example, in the retinal tissue with a diameter in the major axis direction of 0.5 mm, the total number of cells in the retinal tissue may be 1 × 10⁴ cells or more, preferably 1 × 10⁵ cells or more, 5 × 10⁵ cells or more, or 1 × 10⁶ cells or more. Moreover, the outermost cells (photoreceptor progenitor cells, photoreceptor cells, etc.) in the apical surface of the photoreceptor cell layer are consecutive 10 cells or more, 15 cells or more, 20 cells or more in line per 0.1 mm.

The diameter in the major axis direction of the retinal tissue in one embodiment may be 0.5 mm or more, the thickness of the epithelial structure may be 0.2 mm or more, and the number of cells may be 1 × 10⁵ or more. The retinal tissue in one embodiment contains cells at a density of 1 × 10⁵ cells/mm³ or more, preferably 5 × 10⁵ cells/mm³ or more, or 1 × 10⁶ cells/mm³ or more.

The retinal tissue may be a retinal tissue having a continuous epithelial structure. The retinal tissue having the continuous epithelial structure may be a retinal tissue in which, for example, photoreceptor cells or progenitor cells thereof are located consecutively in at least 50% or more (preferably 60% or more, 70% or more, 80% or more, 85% or more, or 90% or more), in area ratio, of the surface of the retinal tissue, that is, the outermost layer in the apical surface. Examples of the retinal tissue having the continuous epithelial structure may include retinal tissues in which the area of the apical surface present on the surface of the retinal tissue is at least 50% or more (preferably 60% or more, 70% or more, 80% or more, 85% or more, or 90% or more) of the surface area of the retinal tissue.

As used herein, the "continuous epithelial structure" in the retinal tissue refers to a structure in which the retinal tissue has an apical surface characteristic of the epithelial tissue and the apical surface is formed substantially parallel to at least the photoreceptor cell layer (outer nuclear layer) or the neuroblastic layer among the layers that form the neural retinal layer and continually on the surface of the retinal tissue. In other words, the continuous epithelial structure has the directionality of the apical surface and the basal membrane maintained and has no structure with a segmented apical surface such as that seen in rosette-like structures. For example, in the case of a cell aggregate containing a retinal tissue produced from pluripotent stem cells, an apical surface is formed on the surface of the aggregate and 10 cells or more, preferably 30 cells or more, more preferably 100 cells or more, further preferably 400 cells or more of photoreceptor cells or photoreceptor progenitor cells are regularly and consecutively arranged in tangential direction to the surface. The number of photoreceptor cells or photoreceptor progenitor cells arranged consecutively is correlated with the size of the neural retina tissue contained in the cell aggregate. As used herein, the tangential direction to the epithelial tissue refers to, when respective cells forming, for example, an apical surface in an epithelial tissue are arranged in a certain direction, the direction of the arrangement of the cells that is parallel or lateral to the epithelial tissue (or epithelial sheet).

In one aspect, an apical surface is formed on the surface of the neural retina tissue and photoreceptor cells or photoreceptor progenitor cells are regularly and consecutively arranged along the apical surface.

In the case of retinal tissues in a stage in which the occurrence rate of photoreceptor cells or photoreceptor progenitor cells is low (example: retinal tissues in the early developmental stage), it is known by those skilled in the art that layers containing proliferating neural retina progenitor cells are called "neuroblastic layers". Moreover, on the surface of the retinal tissue in such a stage, there may be, other than photoreceptor cells or photoreceptor progenitor cells, neural retina progenitor cells, or cells that divide and proliferate from neural retina progenitor cells and/or cells in the stage in which they differentiate from neural retina progenitor cells to cells that constitute the neural retina, that have polarity and are capable of forming an apical surface. The retinal tissue in which photoreceptor cells or photoreceptor progenitor cells are regularly and consecutively arranged is obtained along an apical surface formed on the surface of the neural retina tissue, for example, by keeping culturing a retinal tissue in such a state under conditions for maintaining the "continuous epithelial structure".

In one aspect, the area of the apical surface present on the surface of the retinal tissue may be, on the average, 30% or more, preferably 50% or more, more preferably 60% or more, more preferably 70% or more, more preferably 80% or more, more preferably 90% or more, and further preferably 95% or more of the surface area of the retinal tissue. The proportion of the area of the apical surface present on the surface of the retinal tissue can be measured by staining a marker of the apical surface, as described below.

As used herein, the "rosette-like structure" of the retinal tissue refers to a structure in which cells are arranged radially or spirally to surround the lumen in the center. A retinal tissue that has formed a rosette-like structure is in a state where an apical surface and photoreceptor cells or photoreceptor progenitor cells are located condition along the center (lumen) thereof and the apical surface is segmented into respective rosette-like structures.

As used herein, the "apical surface" refers to a surface (outer face) formed on the other side of the basal membrane side where there is a 50-100 nm layer (basal membrane) rich in mucopolysaccharide (PAS staining positive) and containing a lot of laminin and type IV collagen that epithelial cells produce in an epithelial tissue. In one aspect, in a retinal tissue that has reached a developmental stage in which photoreceptor cells or photoreceptor progenitor cells are found, the "apical surface" refers to a face in contact with a photoreceptor cell layer (outer nuclear layer) in which an outer limiting membrane is formed and photoreceptor cells and photoreceptor progenitor cells are present. Moreover, such an apical surface can be identified by immunostaining which is well-known to those skilled in the art or the like using an antibody to an apical surface marker (examples: atypical-PKC (hereinafter, abbreviated as aPKC), E-cadherin, N-cadherin, Zo-1, beta-catenin, Ezrin). Even when no photoreceptor cells or photoreceptor progenitor cells have emerged in an early developmental stage or when photoreceptor cells or photoreceptor progenitor cells have not emerged to the extent that they cover up the surface of the retinal tissue, an epithelial tissue has polarity and the aforementioned apical surface marker is expressed on the apical surface.

Whether a retinal tissue has a continuous epithelial structure can be determined by the continuity (that is, a form that is not segmented) of the apical surface that the retinal tissue has. The continuity of an apical surface can be determined, for example, by immunostaining an apical surface marker (examples: aPKC, E-cadherin, N-cadherin, Zo-1, beta-catenin, Ezrin), a marker (example: Crx or recoverin) for photoreceptor cells or photoreceptor progenitor cells located in the apical surface side and analyzing the acquired images or the like for the positional relation between the apical surface and the photoreceptor cell layer and between retinal layers. The retinal layers other than the apical surface and the photoreceptor cell layer (outer nuclear layer) can be identified, for example, by DAPI staining, PI staining, Hoechst staining to stain nuclei or immunostaining, e.g., for a marker protein (Rx, Chx10, Ki67, Crx, etc.) that locate in the nucleus.

Whether a rosette-like structure has been generated or not can be determined by creating cryosections after fixing cell aggregates with 4% paraformaldehyde or the like and observing dysplasia (for example, a segmented apical surface or invasion of an apical surface into a cell aggregate) of the rosette-like structure by immunostaining or the like usually performed using an antibody to an apical surface marker aPKC, E-cadherin, N-cadherin, or DAPI or the like to specifically stain the nucleus.

In one aspect, the proportion of retinal progenitor cells, neural retina progenitor cells, and photoreceptor progenitor cells in the retinal tissue, that is, the proportion of cells expressing PAX6, Chx10, and/or Crx may be 50% or more, 60% or more, 70% or more, 80% or more, 85% or more, 90% or more, or 95% or more of the total number of cells. 50% or more of the total number of cells contained in a piece of the retinal tissue may be cells expressing at least one of PAX6, Chx10, and Crx.

In one aspect, the proportion of cells expressing PAX6, Chx10, and/or Crx present in the outermost layer (the photoreceptor cell layer or neuroblastic layer) in the apical surface side in the retinal tissue herein may be 80% or more of the total number of cells present in the outermost layer in the apical surface side and is preferably 85% or more, 90% or more, 95% or more, 98% or more, or 99% or more. In another aspect, the proportion of photoreceptor progenitor cells (Crx positive and rhodopsin, S-Opsin, and M/L-Opsin-negative cells) and photoreceptor cells (Recoverin-positive cells or cells positive for any of rhodopsin, S-Opsin, and M/L-Opsin) present in the outermost layer (the photoreceptor cell layer or neuroblastic layer) in the apical surface side in the retinal tissue herein may be 80% or more of the total number of cells present in the outermost layer in the apical surface side and is preferably 85% or more, 90% or more, 95% or more, 98% or more, or 99% or more.

In one aspect, the retinal tissue may include photoreceptor cells. The "photoreceptor cells" are present in the photoreceptor cell layer in the retina and has a role in absorbing light stimuli and converting them into electrical signals. There are two types of photoreceptor cells: the cone that functions in light places and the rod that functions in dark places. The photoreceptor cells differentiate and mature from photoreceptor progenitor cells. Whether a cell is a photoreceptor cell or a photoreceptor progenitor cell can be determined easily by a person skilled in the art, for example, based on the expression of a cell marker described later (such as Crx and Blimp 1 expressed in photoreceptor progenitor cells, Recoverin expressed in photoreceptor cells, rhodopsin, S-Opsin, and M/L-Opsin expressed in matured photoreceptor cells, and the like), the formation of the outer segment structure, or the like.

In one aspect, the retinal tissue herein may include photoreceptor progenitor cells (Crx positive and rhodopsin, S-Opsin, and M/L-Opsin-negative cells) and photoreceptor cells (Recoverin-positive cells or cells positive for any of rhodopsin, S-Opsin, and M/L-Opsin).

In one aspect, the proportion of cells that express Recoverin in the retinal tissue herein may be 5% or more of the total number of cells and may be preferably 10% or more, 20% or more, 40% or more, or 50% or more. In one aspect, the proportion of cells that express Recoverin in the retinal tissue herein may be 80% or more of the total number of cells present in the photoreceptor cell layer and is preferably 85% or more, 90% or more, 95% or more, 98% or more, or 99% or more.

The retinal tissue may be characterized by expressing TGF (transforming growth factor) β. TGFβ is one of cytokines that control the proliferation and differentiation of cells and maintain the homeostasis of the living body. For TGFβ, the existence of 5 subtypes (β1 to β5) is known and 3 subtypes (β1-3) are known in mammals. By the expression of these, immunosuppression effect is expected. Of these, the retinal tissue may preferably be one that expresses TGFβ2. TGFβ2 is expressed in immune-privileged sites such as the testis. By the expression of TGFβ2, immunosuppression effect is expected.

It is more preferred that TGFβ is present on the surface of the retinal tissue, that is, in cells present on the apical surface and/or in the outermost layer in the apical surface side. The expression "TGFβ is present on the surface of the retinal tissue" refers to a state in which TGFβ is expressed and secreted in cells present in the outermost layer in the apical surface side and/or TGFβ is present on the apical surface, that is to say, TGFβ secreted by the aforementioned cells is bound to matrices (such as N-cadherin) present on the apical surface. More specifically, it is preferred that, in the retinal tissue in which the directionality of the apical surface and the basal membrane is maintained by having the continuous epithelial structure, TGFβ is expressed and secreted in outermost cells (preferably cells located 1 or 2 or 3 cells or so inside from the outside) present in the apical surface side and TGFβ is present on the apical surface. Preferably, the abundance ratio of TGFβ in the apical surface, may be, in area ratio, 50% or more of the area of the whole apical surface and is further preferably 60% or more, 70% or more, 80% or more, 85% or more, 90% or more, or 95% or more. Moreover, TGF β is expressed preferably in 50% or more, more preferably 60% or more, 70% or more, 80% or more, 85% or more, 90% or more, or 95% or more of the outermost cells present in the apical surface side.

A person skilled in the art can measure the amount of TGFβ expressed using a well-known technology. Specifically, the amount of TGFβ expressed in a medium after culturing may be measured by ELISA. As one aspect, the amount of TGFβ expressed may be 10 fold or more of that in the pluripotent stem cells used as the raw material and is preferably 50 fold or more, 100 fold or more, 200 fold or more, or 500 fold or more. As another aspect, the amount of TGFβ expressed in one retinal tissue, when one retinal tissue is cultured in 1 mL of a medium for 2 days, may be 1 pg/mL or more and is preferably 2 pg/mL or more, 5 pg/mL or more, or 10 pg/mL or more, or the like. Moreover, a person skilled in the art can measure the expression site of TGFβ using a well-known technology. Specifically, it can be achieved by immunostaining of the retinal tissue using an anti-TGFβ antibody. The presence or absence and the proportion of TGFβ in the apical surface can be determined by co-staining with the aforementioned apical surface marker, as needed.

The retinal tissue that is an active ingredient in the therapeutic drug herein includes also cell aggregates obtained by making retinal tissue differentiated from stem cells semi-dissociated or partially cutting out the retinal tissue. The cell aggregates obtained by making the retinal tissue semi-dissociated or partially cutting out the retinal tissue may be, for example, 1/4, 1/8, 1/16, 1/32, 1/64, or 1/128 of the retinal tissue differentiated from stem cells or the like in size (in terms of the number of cells). The method of dissociation may be use of an enzyme or physical excision using scissors, a knife, or the like.

Accordingly, as one aspect, the retinal tissue having a three-dimensional structure that is an active ingredient of the therapeutic drug herein has the following characteristics.
(1) Retinal cells constitute a retinal layer(s) in cell aggregates and the retinal cells are layered in the retinal layer(s);
(2) the diameter in the major axis direction of the retinal tissue is 0.2 mm or more (preferably 0.5 mm or more), the thickness of the epithelial structure of the retinal tissue is 0.2 mm or more, and the total number of cells contained in the retinal tissue is 1 × 10⁵ cells or more (or the retinal tissue includes cells at a density of 1 × 10⁵ cells/mm³ or more);
(3) 50% or more (preferably 60% or more, 70% or more, 80% or more, or 90% or more) of the total number of cells contained in a piece of the retinal tissue are cells expressing at least one of PAX6, Chx10, and Crx and/or 80% or more (preferably 85% or more, 90% or more, or 95% or more) of the total number of cells present in the photoreceptor cell layer or neuroblastic layer are cells expressing at least one of PAX6, Chx10, and Crx;
(4) the retinal tissue has a continuous epithelial structure at an area ratio of at least 50% or more (preferably 60% or more, 70% or more, 80% or more, 85% or more or 90% or more) to the surface of the retinal tissue; and
(5) the retinal tissue expresses TGFβ (preferably TGFβ2) and TGFβ is preferably present on the surface of the retinal tissue.

The aforementioned retinal tissue may further have one or more of the following characteristics.
(A) The retinal tissue is a sphere-shaped cell aggregate and preferably has the vesicular structure.
(B) The retinal tissue includes substantially no retinal pigment epithelial cells.
(C) Outermost cells on the apical surface of the retinal tissue are arranged consecutively at a rate of 10 cells or more (preferably 15 cells or more or 20 cells or more) per 0.1 mm.
(D) The retinal tissue may include photoreceptor cells and they are preferably 80% or more of the total number of cells present in the photoreceptor cell layer or 5% or more of the total number of cells in the retinal tissue.
(E) The retinal tissue is derived from a pluripotent stem cell.

In one aspect, the retinal tissue is a tissue that was differentiated from an allogeneic stem cell (for example, an allogeneic pluripotent stem cell). The method for inducing differentiation is not particularly limited, and examples thereof include methods disclosed in WO2011/055855, WO2013/077425, WO2015/025967, WO2016/063985, WO2016/063986, WO2017/183732, PLoS One. 2010 Jan 20; 5 (1): e8763., Stem Cells. 2011 Aug; 29 (8): 1206-18., Proc Natl Acad Sci USA. 2014 Jun 10; 111 (23): 8518-23, and Nat Commun. 2014 Jun 10; 5: 4047. As another aspect, a retinal tissue from an allogeneic living body can be used. Methods for preparing a retinal tissue from a living body is well-known for those skilled in the art. Specifically, a retinal tissue can be excised under anesthesia. From the excised retinal tissue, RPE cells can be removed surgically.

Examples of one specific aspect of the method for producing neural retina by induction of differentiation include a method including the following steps (A), (B), and (C):
(A) a step of culturing a pluripotent stem cell, in the absence of feeder cells, in a medium including: 1) a TGFβ family signaling pathway inhibitor and/or a substance acting on a Sonic Hedgehog signaling pathway, and 2) a factor for maintaining undifferentiated state;
(B) a step of forming a cell aggregate by floating culture in a serum-free medium;
(C) a step of further conducting floating culture of the cell aggregate obtained in the step (B) in a medium including a substance acting on a BMP (bone morphogenetic protein) signaling pathway.

This method is also disclosed, for example, in WO2016/063985 or WO2017/183732 and, more particularly, it is possible to refer to WO2016/063985 or WO2017/183732.

The TGFβ family signaling pathway inhibitor refers to a substance that inhibits a TGFβ family signaling pathway, that is, a signaling pathway transduced with the Smad family and specific examples thereof include TGFβ signaling pathway inhibitors (examples: SB431542:
4-[4-(1,3-benzodioxol-5-yl)-5-(2-pyridinyl)-1H-imidazol-2-yl]-benzami de, LY-364947: 4-[3-(2-pyridinyl)-lH-pyrazol-4-yl]-quinoline, SB-505124:
2-[4-(1,3-benzodioxol-5-yl)-2-(1,1-dimethylethyl)-1H-imidazol-5-yl]-6-methyl-pyridine, A-83-01:
3-(6-Methyl-2-pyridinyl)-N-phenyl-4-(4-quinolinyl)-1H-pyrazole-1-car bothioamide, and the like), Nodal/Activin signaling pathway inhibitor (examples: SB431542, A-83-01, and the like) and BMP signaling pathway inhibitors (examples: LDN193189:
4-[6-[4-(1-Piperazinyl)phenyl]pyrazolo[1,5-a]pyrimidin-3-yl]quinolinc dihydrochloride, Dorsomorphin:
6-[4-[2-(1-Piperidinyl)ethoxy]phenyl]-3-(4-pyridinyl)-pyrazolo[1,5-a]p yrimidine, and the like). These substances are marketed and are available.

The substance acting on a Sonic Hedgehog (which may, hereinafter, be referred to as "Shh") signaling pathway is a substance that can enhance signal transduction transduced with Shh. Examples of the Shh signaling pathway agent include SHH, PMA (Purmorphamine:
9-Cyclohexyl-N-[4-(4-morpholinyl)phenyl]-2-(1-naphthalenyloxy)-9H-purin-6-amine), and SAG (Smoothened Agonist:
3-Chloro-N-[trans-4-(methylamino)cyclohexyl]-N-[3[-(4-pyridinyl)phe nyl]methyl]benzo[b]thiophene-2-carboxamide).

The concentrations of the TGFβ family signaling pathway inhibitor and the substance acting on a Sonic Hedgehog signaling pathway may be concentrations that can induce differentiation into retinal cells. For example, SB431542 is usually used at a concentration of 0.1-200 µM, preferably 2-50 µM. A-83-01 is usually used at a concentration of 0.05-50 µM, preferably 0.5-5 µM. LDN193189 is usually used at a concentration of 1-2000 nM, preferably 10-300 nM. SAG is usually used at a concentration of 1-2000 nM, preferably 10-700 nM. PMA is usually used at a concentration of 0.002-20 µM, preferably 0.02-2 µM.

In culturing the pluripotent stem cell under feeder-free conditions in the step (A), the aforementioned feeder-free medium containing the factor for maintaining undifferentiated state may be used as a medium.

In culturing the pluripotent stem cell under feeder-free conditions in the step (A), an appropriate matrix may be used as scaffolding for providing scaffolding for the pluripotent stem cell in the place of feeder cells. Examples of the matrix that can be used as scaffolding include Laminin (Nat Biotechnol 28,611-615, (2010)), a Laminin fragment (Nat Commun 3, 1236, (2012)), a basal membrane preparation (Nat Biotechnol 19, 971-974, (2001), example: Matrigel), gelatin, collagen, heparan sulfate proteoglycan, entactin, and vitronectin.

The time of culturing the pluripotent stem cell in the step (A) may be in a range in which the improving effect on quality of cell aggregates formed in step (B) can be achieved and is not particularly limited, and usually 0.5-144 hours. In one aspect, the time of culturing the pluripotent stem cell in the step (A) is preferably 2-96 hours, more preferably 6-48 hours, further preferably 12-48 hours, more further preferably 18-28 hours (example, 24 hours).

Preparations and the formation of the cell aggregate of the serum-free medium can be performed in a well-known method for those skilled in the art. Examples of the method of preparing the serum-free medium and forming a cell aggregate include the SFEB method: Serum-free Floating culture of Embryoid Bodies-like aggregates (Watanabe et al. Nature Neuroscience, volume 8, pages 288-296 (2005)); and the SFEBq method (Eiraku et al., Cell Stem Cell, Volume 3, ISSUE 5, P519-532, November 06, 2008), which is an improved method of the SFEB method. The SFEBq method specifically means a method in which about 12000 cells per dish of pluripotent stem cells are transferred into non-cell-adhesive culture dishes with a well diameter of a little shorter than 1 cm or so (example: a well of 96 well plates: about 7 mm) and aggregates are quickly generated within 2-3 hours.

In one aspect, the medium used in the step (B) may include a substance acting on a Sonic Hedgehog signaling pathway. As the substance acting on a Sonic Hedgehog signaling pathway, those described above may be used at a concentration described above. The substance acting on a Sonic Hedgehog signaling pathway is preferably contained in the medium from the starting time of the floating culture. A ROCK inhibitor (for example, Y-27632) may be added to the medium. The culture time may be, for example, 12 hours to 6 days.

The substance acting on a BMP signaling pathway is a substance that can enhance signal transduction mediated by BMP. Examples of the substance acting on a BMP signaling pathway include BMP proteins such as BMP2, BMP4, or BMP7, GDF proteins such as GDF7, anti-BMP receptor antibodies, or partial BMP peptides. The BMP2 protein, the BMP4 protein, and the BMP7 protein are available from, for example, R&D Systems, Inc. and the GDF7 protein is available from, for example, Fuji film Wako Pure Chemical Industries, Ltd.

Examples of the medium to be used include a serum-free medium or a serum medium (preferably a serum-free medium) having a substance acting on a BMP signaling pathway added therein. The serum-free medium and the serum medium can be prepared as described above.

The concentration of the substance acting on a BMP signaling pathway may be a concentration that can induce the differentiation into retinal cells. For example, in the case of the human BMP4 protein, it is added to a medium at a concentration of about 0.01 nM to about 1 µM, preferably about 0.1 nM to about 100 nM, more preferably about 1 nM to about 10 nM, further preferably about 1.5 nM (55 ng/mL).

The substance acting on a BMP signaling pathway may be added to a medium in about 24 hours after starting the floating culture in step (A) and it may be added to a medium within several days (for example, within 15 days) after starting the floating culture. The substance acting on a BMP signaling pathway may be added to a medium preferably from day-1 to day-15, more preferably from day-1 to day-9, most preferably on day-3 after starting the floating culture.

As a specific aspect, for example, on day-1 to day-9 after starting the floating culture in the step (B), preferably day-1 to day-3 after starting the floating culture in the step (B), a part or all of the medium is exchanged with a medium containing BMP4 and the final concentration of BMP4 is adjusted to about 1-10 nM and culturing can be conducted in the presence of BMP4, for example, for 1-12 days, preferably for 2-9 days, further preferably for 2-5 days. Here, in order to maintain the concentration of BMP4 at a constant concentration, a part or all of the medium can be exchanged with a medium containing BMP4 once or 2 times or so. Or the concentration of BMP4 can be reduced step by step.

The culture conditions such as culture temperature and the CO₂ concentration in the step (A) to the step (C) can be set as appropriate. For example, the culture temperature is about 30°C to about 40°C, preferably about 37°C. Moreover, the CO₂ concentration is, for example, about 1% to about 10%, preferably about 5%.

By changing the culture period in the step (C) described above, it is possible to produce retinal tissues containing retinal cells at various differentiation stages. More specifically, retinal tissues containing immature retinal cells (examples: retinal progenitor cells, photoreceptor progenitor cells) and mature retinal cells (example: photoreceptor cells) at various proportions can be produced. By extending the culture period in the step (C), the proportion of mature retinal cells can be increased. The "immature retinal cells" means progenitor cells determined to differentiate into mature retinal cells.

In the step (B) and/or the step (C) described above, a method disclosed in WO2017/183732 may also be used. Accordingly, in the step (B) and/or the step (C), neural retina can be formed by conducting floating culture of cells obtained by the step (A) in a medium further including a Wnt signaling pathway inhibitor.

The Wnt signaling pathway inhibitor to use in the step (B) and/or the step (C) is not particularly limited, as long as it can suppress the signal transduction mediated by Wnt, and may be any of a protein, a nucleic acid, a low molecular weight compound, and the like. The signal mediated by Wnt is transduced via a Wnt receptor, which exists as a heterodimer of Frizzled (Fz) and LRP5/6 (low-density lipoprotein receptor-related protein 5/6). Examples of the Wnt signaling pathway inhibitor include, but are not limited to, substances that directly act on Wnt or the Wnt receptor (anti-Wnt neutralizing antibodies, anti-Wnt receptor neutralizing antibodies, and the like), substances that suppress expression of a gene encoding Wnt or the Wnt receptor (for example, antisense oligonucleotides, siRNAs, and the like), substances that inhibit binding of Wnt to the Wnt receptor (soluble Wnt receptors, dominant negative Wnt receptors, and the like, and Wnt antagonists, Dkkl, Cerberus protein, and the like), and substances that inhibit physiological activity caused by the signal transduction via the Wnt receptor [low molecular weight compounds such as CKI-7 (N-(2-aminoethyl)-5-chloroisoquinoline-8-sulfonamide), D4476 (4-[4-(2,3-dihydro-1,4-benzodioxin-6-yl)-5-(2-pyridinyl)-1H-imidazol-2 -yl]benzamide), IWR-1-endo (IWR1e) (4-[(3aR,4S,7R,7aS)-1,3,3a,4,7,7a-hexahydro-1,3-dioxo-4,7-methano-2 H-isoindol-2-yl]-N-8-quinolinyl-benzamide), and IWP-2 (N-(6-methyl-2-benzothiazolyl)-2-[(3,4,6,7-tetrahydro-4-oxo-3-phenyl thieno[3,2-d]pyrimidin-2-yl)thio] acetamide), and the like]. The Wnt signaling pathway inhibitor may include one or two or more of these. CKI-7, D4476, IWR-1-endo (IWR1e), IWP-2, and the like are known Wnt signaling pathway inhibitors and commercially available products thereof may be obtained as appropriate. As the Wnt signaling pathway inhibitor, IWRle is preferably used.

The concentration of the Wnt signaling pathway inhibitor may be a concentration that can induce the formation of good cell aggregates in the step (B). For example, IWR-1-endo is added to the medium at a concentration of about 0.1 µM to about 100 µM, preferably about 0.3 µM to about 30 µM, more preferably about 1 µM to about 10 µM, and further preferably about 3 µM. When a Wnt signaling pathway inhibitor other than IWR-1-endo is used, it is desirable that the Wnt signaling pathway inhibitor is used at a concentration at which the Wnt signaling pathway inhibitor exhibits the Wnt signaling pathway inhibitory activity equivalent to that of the aforementioned concentration of IWR-1-endo.

In the step (B), earlier is preferable in the timing of adding the Wnt signaling pathway inhibitor to the medium. The Wnt signaling pathway inhibitor is added to the medium usually within 6 days, preferably within 3 days, more preferably within 1 day, more preferably within 12 hours, after starting the floating culture in the step (B), and further preferably at the time of starting the floating culture in the step (B). Specifically, for example, addition of a basal medium which the Wnt signaling pathway inhibitor has been added to or partial or total medium change with the basal medium may be conducted. The period of time during which the Wnt signaling pathway inhibitor, in the step (B), is allowed to act on the cells obtained in the step (A) is not particularly limited, and it is preferable to allow the Wnt signaling pathway inhibitor to act after the addition of the Wnt signaling pathway inhibitor to the medium at the time of starting the floating culture in the step (B) until the time of end of the step (B) (just before the addition of the substance acting on a BMP signaling pathway). Further preferably, the cells are exposed to the Wnt signaling pathway inhibitor also after the end of the step (B) (that is, during the step (C)) continuously, as described below. As one aspect, the Wnt signaling pathway inhibitor may still be allowed to act after the end of the step (B) (that is, during the step (C)) continuously, as described below, until retinal tissue is formed.

As the Wnt signaling pathway inhibitor in the step (C), any of the above-mentioned Wnt signaling pathway inhibitors may be used, but that of the same kind as the Wnt signaling pathway inhibitor used in the step (B) is preferably used in the step (C).

The concentration of the Wnt signaling pathway inhibitor in the step (C) may be a concentration at which retinal progenitor cells and retinal tissue can be induced. For example, IWR-1-endo is added to the medium at a concentration of about 0.1 µM to about 100 µM, preferably about 0.3 µM to about 30 µM, more preferably about 1 µM to about 10 µM, and further preferably about 3 µM. When a Wnt signaling pathway inhibitor other than IWR-1-endo is used, it is desirable that the Wnt signaling pathway inhibitor is used at a concentration at which the Wnt signaling pathway inhibitor exhibits the Wnt signaling pathway inhibitory activity equivalent to that of the aforementioned concentration of IWR-1-endo. If the concentration of the Wnt signaling pathway inhibitor in the medium in the step (B) is 100, the concentration of the Wnt signaling pathway inhibitor in the medium in the step (C) is preferably 50-150, more preferably 80-120, further preferably 90-110 and it is more preferable that the concentration is equivalent to the concentration of the Wnt signaling pathway inhibitor in the medium in the second step.

The timing of the addition of the Wnt signaling pathway inhibitor to the medium is not particularly limited, as long as the formation of aggregates containing retinal cells or retinal tissue can be achieved, but the earlier is the more preferable. Preferably, the Wnt signaling pathway inhibitor is added to the medium at the time of starting the step (C). More preferably, after the Wnt signaling pathway inhibitor is added in the step (B), the Wnt signaling pathway inhibitor is contained in the medium continuously in the step (C) (that is, from the start of the step (B)). Further preferably, after the Wnt signaling pathway inhibitor is added at the time of starting the floating culture in the step (B), the Wnt signaling pathway inhibitor is contained in the medium also in the step (C) continuously. For example, the BMP signal transduction agent (for example, BMP4) may be added to the culture obtained in the step (B) (the suspension of aggregates in the medium containing the Wnt signaling pathway inhibitor).

The period of time during which the Wnt signaling pathway inhibitor is allowed to act is not particularly limited, and when the Wnt signaling pathway inhibitor is added at the time of starting the floating culture in the step (B), it is preferably 2 days to 30 days, more preferably 6 days to 20 days, 8 days to 18 days, 10 days to 18 days, or 10 days to 17 days (for example, 10 days), starting count at the time of starting the floating culture in the step (B). In another aspect, when the Wnt signaling pathway inhibitor is added at the time of starting the floating culture in the step (B), the period of time during which the Wnt signaling pathway inhibitor is allowed to act is preferably 3 days to 15 days (for example, 5 days, 6 days, or 7 days) and more preferably 6 days to 10 days (for example, 6 days), starting count at the time of starting the floating culture in the step (B).

By further subjecting the cell aggregates of retinal cells obtained in the method described above to the following steps, it is also possible to produce neural retina including a ciliary marginal zone-like structure. By producing the neural retina including a ciliary marginal zone-like structure, it is possible to produce neural retina including photoreceptor progenitor cells in rich and including a continuous epithelial structure at high frequency:
Step D: a step of culturing for a period of 3 days to 6 days or so in a serum-free medium or a serum medium including a Wnt signaling pathway activating substance and/or an FGF signaling pathway inhibitor;
Step E: a step of culturing cell aggregates obtained by the step D for 30 days to 150 days (preferably, 30 days to 120 days or 30 days to 100 days) in a serum-free medium or a serum medium not including a substance acting on a Wnt signaling pathway and an FGF signaling pathway inhibitor.

As one aspect, a ciliary marginal zone-like structure can be produced by the aforementioned steps (D) and (E) from a cell aggregate including neural retina obtained in the steps (A) to (C), the cell aggregate including neural retina day-6 to day-30, day-10 to day-20 (day-10, day-11, day-12, day-13, day-14, day-15, day-16, day-17, day-18, day-19, or day-20) after the starting the floating culture in the step (B).

The substance acting on a Wnt signaling pathway is not particularly limited, as long as it can enhance the signal transduction mediated by Wnt. Specific examples of the substance acting on a Wnt signaling pathway include GSK3β inhibitors (for example, 6-Bromoindirubin-3'-oxime (BIO), CHIR99021, Kenpaullone). Examples of the concentration of the substance acting on a Wnt signaling pathway in the serum-free medium or serum medium in the step D, for example, for CHIR99021, include a range of about 0.1 µM to about 100 µM, preferably about 1 µM to about 30 µM.

The FGF signaling pathway inhibitor is not particularly limited, as long as it can inhibit the signal transduction mediated by FGF. Examples of the FGF signaling pathway inhibitor include SU-5402, AZD4547, and BGJ398. As for the concentration of the FGF signaling pathway inhibitor in the serum-free medium or serum medium in the step D, for example, in the case of SU-5402, the FGF signaling pathway inhibitor is added at a concentration of about 0.1 µM to about 100 µM, preferably about 1 µM to about 30 µM, and more preferably about 5 µM.

The retinal tissue (neural retina) can be produced by the methods described above, but methods are not limited to these.

The "graft rejection" means the protective reaction of an individual to reject the grafted tissue or organ by the immune response of the patient (recipient) who underwent graft.

The graft rejection is, depending on the time of development, classified into, for example, the acute phase and the chronic phase. The acute phase of rejection occurs on the day of graft or within several days (for example, 1 day, 2 days, or 3 days after graft or the like) to about 2 months after graft and the chronic phase of rejection develops on or after about 2 months after graft. The acute rejection may further be classified into acute early rejection (within about 10 days after graft) and acute late rejection (within about 2 months from about 11 days after graft).

Examples of specific symptoms include infiltration of lymphocytes and activation of the immune cells. Moreover, the degree of graft rejection can be determined by examination of the fundus, fundus fluorescence photography, and/or mixed lymphocyte assay as an indicator.

Since it is highly possible in acute rejection (particularly acute early rejection) that complicated and strong immune response also affected by the inflammatory reaction by surgical invasion is induced, an immunosuppressive agent such as tacrolimus is usually administered systemically in kidney graft. If there is no observation of rejection, the dose of the immunosuppressive agent is subsequently reduced, but usually use of the immunosuppressive agent is continued.

An immunosuppressive agent is an agent that is used for suppressing or inhibiting the activity of the immune system. The immunosuppressive agent is used for the prevention and suppression of rejection to a grafted organ or tissue and inflammatory control of autoimmune diseases and inflammatory diseases such as allergy.

As for immunosuppressive agents, some classifications are possible based on similarity of the mechanism of action or structure, or the like. Examples of the classification based on the mechanism of action include steroidal anti-inflammatory drugs (SAIDs), calcineurin inhibitors, mTOR inhibitors, cytotoxic agents, antimetabolites, cytotoxic antibiotics, alkylating agents, and microtubule synthesis inhibitors. The substance used as an immunosuppressive agent is not limited and may be any of a low molecular weight compound, a protein (including an antibody), a nucleic acid, and the like. These immunosuppressive agents include agents that are used as immunosuppressive agents for suppressing graft rejection because of having immunosuppressive activity, although they are used as therapeutic agents of other diseases. Examples of immunosuppressive agents administered to organ graft patients for suppressing graft rejection include calcineurin inhibitors, mTOR inhibitors, antimetabolites, and steroidal anti-inflammatory drugs.

The steroidal anti-inflammatory drug is an anti-inflammatory drug containing a glucocorticoid or a derivative thereof as an active ingredient. The steroidal anti-inflammatory drug suppresses the expression of the inflammatory genes NF-κB and AP-1 by binding to a glucocorticoid receptor (GR/NR3C1), which is a nuclear receptor, and inhibits proliferation signals for lymphocytes (in addition to cells involved in the acquired immune system, particularly cells involved in the innate immune system: neutrophils, macrophages). Examples thereof include, but are not limited to, prednisolone, methylprednisolone, triamcinolone, dexamethasone, betamethasone, and fluorometholone.

Herein, as for the steroidal anti-inflammatory drugs, a classification based on the strength of action may be performed. Specifically, they are classified into strong steroidal anti-inflammatory drugs and mild steroidal anti-inflammatory drugs. Examples of the strong steroidal anti-inflammatory drugs include dexamethasone, betamethasone, prednisolone, and methylprednisolone. Examples of the mild steroidal anti-inflammatory drugs include fluorometholone, betamethasone dipropionate (betamethasone valerate), triamcinolone, cortisone and hydrocortisone.

Examples of steroidal anti-inflammatory drugs used in the ophthalmologic field include betamethasone valerate or fluorometholone, or the like used as an instillation, and triamcinolone acetonide used as an intravitreal injection and/or a Tenon injection. Moreover, as an oral formulation, cortisone or hydrocortisone, having a short action time, prednisolone or methylprednisolone sodium succinate, having a moderate action time, or dexamethasone or betamethasone, having a long action time. Usually, a solution of methylprednisolone for infusions is used for the steroid pulse therapy.

It is known that steroidal anti-inflammatory drugs are also effective for the prevention of graft rejection and, for example, prednisolone is used. Moreover, it has been reported that a pulse therapy using an oral formulation of a steroidal anti-inflammatory drug or a steroidal anti-inflammatory drug is used for the purpose of prevention of immunorejection due to graft of RPE cells or the like, but it has not been reported that a steroidal anti-inflammatory drug is used in an instillation or an intravitreal injection or a Tenon injection alone.

A calcineurin inhibitor is an agent having the calcineurin inhibitory action, and particularly an agent that controls the activation of T cells as the main action. Specifically, after binding to an intracellular receptor called immunophilin, the calcineurin inhibitor suppresses the activity of the phosphatase calcineurin PP2B, suppresses the production of interleukin-2 (IL-2) and interferon γ, and exhibits the suppression of T cell proliferation. Examples of the calcineurin inhibitor include cyclosporine and tacrolimus.

In the ophthalmologic field, while cyclosporine has, as an instillation, an application, e.g., in noninfective uveitis, tacrolimus has no application in the ophthalmologic field.

Moreover, it is known that an agent having the calcineurin inhibitory action is effective in the prevention of graft rejection; it has been reported that oral formulations for calcineurin inhibition are used for the purpose of prevention of immunorejection due to graft of RPE cells or the like. On the other hand, use of an instillation of a calcineurin inhibitor has not been reported.

The mTOR inhibitor is an agent having the mTOR (mammalian target of rapamycin) inhibitory action. Specifically, after binding to immunophilin, the mTOR inhibitor inhibits the mTOR activity and exhibits effects such as the arrest of cell cycle of immunocompetent cells and the suppression of protein translation. It is known to inhibit the activation of T cells and B cells by lowering the production of interleukin-2 (IL-2). Examples of the mTOR inhibitor include sirolimus (rapamycin), and temsirolimus and everolimus, which are derivatives of sirolimus.

The cytotoxicity agent is an agent having the cell division inhibitory action, which is used as an anticancer agent. The cytotoxicity agent is also used as an immunosuppressive agent by using a small quantity in comparison with cancer treatments. The cytotoxicity agents can further be classified into antimetabolites that interfere the nucleic acid synthesis, cytotoxic antibiotics, alkylating agents, microtubule synthesis inhibitor and the like.

Examples of the antimetabolites include mercaptopurine and azathioprine, which are a purine analog and a precursor thereof, methotrexate, which is a folic acid analog, mycophenolic acid, which is a guanosine production inhibitor, and leflunomide, which is a pyrimidine synthesis inhibitor. Azathioprine, and 6-Mercaptopurine which is an active metabolite, become 6-MP ribonucleic acid and mycophenolic acid and mizoribine suppress the activity of the inosine-1-phosphate dehydrogenase IMPDH, which is involved in the main route of proliferation of lymphocytes to exhibit the proliferation suppression of lymphocytes by inhibiting de novo nucleic acid synthesis. Moreover, it has been reported that oral formulations of mycophenolic acid are used for the purpose of prevention of immunorejection due to the graft of RPE cells or the like.

The cytotoxic antibiotics are antibiotics having cytotoxic activity. Examples of the cytotoxic antibiotics include dactinomycin, anthracycline, mitomycin C, bleomycin, and mithramycin.

The alkylating agent is a type of cytotoxic anticancer agents. Examples of the alkylating agent include cyclophosphamide.

The microtubule synthesis inhibitor is an agent that inhibits microtubule synthesis and inhibits cell migration and the like. Examples of the microtubule synthesis inhibitor include colchicine and vinblastine.

Examples of the proteins and antibodies include cytokines (IL-2, TNFα, and the like) and receptors thereof, and proteins and antibodies that act on surface markers (CD3 and CD25 and the like) of immune cells such as T cells. The antibodies can be classified into polyclonal antibodies and monoclonal antibodies. Examples of the anti-CD25 antibodies include basiliximab and examples of the anti-CD20 antibodies include rituximab. These suppress the function of the target immune cells.

The immunosuppressive agents can be classified into oral formulations, injections, and topical agents based on the dosage form. The oral formulations are formulations of a pharmaceutical preparation (agent) to be administered orally. The oral formulations include, but are not limited to, various dosage forms such as tablets, capsules, and granules. An injection is an agent to be directly administered using a needle, for example, into intradermal or subcutaneous tissue, intravascularly or intraocularly (sub-Tenon capsule, intravitreally) that is liquid or dissolved before use into liquid to be used. The topical agent is a generic name of all agents to be directly used on the human body other than the oral formulations and the injections. The topical agents include, but are not limited to, instillations; ophthalmic ointments; inhalers such as liquids, aerosol, and dry powder; transdermal agents such as ointments, creams, poultices, and tape agents.

As the dosage forms of immunosuppressive agents used for graft in the ophthalmic field, oral formulations, injections (intravenous injections, sub-Tenon capsule injections, intravitreal injections, and the like) and particularly instillations as topical agents are used.

The expression "give an immunosuppressive agent systemically" means administering in a way such that the immunosuppressive agent is distributed over the whole body, that is, in a way such that the active ingredient of the immunosuppressive agent is present in the bloodflow, and specifically, administering the immunosuppressive agent by oral administration or intravenous administration, or the like.

The expression "give an immunosuppressive agent locally" means administering in a way such that the immunosuppressive agent is distributed in a local site such as in an eye or skin and substantially no active ingredient of the immunosuppressive agent is present in the bloodflow. In the present embodiment, the expression "give an immunosuppressive agent locally" specifically means administering the immunosuppressive agent by an injection (a sub-Tenon capsule injection, an intravitreal injection, or the like) into an eye or ophthalmic administration, or the like. In terms of the reduction of patient's burdens such as side effects, the local administration of an immunosuppressive agent is preferred and administration of an instillation is particularly preferred. However, in current transplantation therapies, it is usual that an immunosuppressive agent is administered systemically and it has not been reported that an immunosuppressive agent can be administered locally for the purpose of prevention of graft rejection.

The sub-Tenon capsule injection is the mode of administration in which the conjunctiva is cut open small and an agent is injected into the Tenon capsule behind the eyeball. It is a treatment that is much more powerful than an instillation and effect on the posterior eye segment can be expected and it is considered that the effect lasts for a long period of time by one injection.

The intravitreal injection is a way of administering an agent directly in an eye, and reduces the risk of systemic side effects, and exerts the therapeutic effect more strongly on an intraocular lesion.

The administration of an immunosuppressive agent aimed at preventing graft rejection means administering preventively before graft rejection is found. In other words, the administration of an immunosuppressive agent for the purpose of the prevention and/or treatment of other diseases and the administration of an immunosuppressive agent for the purpose of controlling graft rejection that already occurred are not included.

Usually, the administration of an immunosuppressive agent aimed at preventing graft rejection is started from just before or just after the graft and is continued permanently. During the period of continuation of the immunosuppressive agent after graft, the kind, the dosage form, or the usage or the dose of the immunosuppressive agent may be changed. Specifically, a high dose of the immunosuppressive agent is frequently administered early in the graft, but the dose and the frequency are increased or decreased as appropriate depending on subsequent symptoms. It is considered that the dose is gradually reduced after a stable state is obtained and maintained at a minimum effective dose. However, systemic administration of an immunosuppressive agent aimed at preventing rejection due to graft including graft in the ophthalmologic field is continued.

The therapeutic drug of the present embodiment is characterized by inducing no immune responses or suppressing immune responses leading to rejection associated with graft. In other words, it is characterized in that the reduction of patient's burdens of the administration of an immunosuppressive agent aimed at preventing rejection is possible by using the therapeutic drug of the present embodiment in graft. Therefore, the therapeutic drug of the present embodiment is characterized in that no administration of an immunosuppressive agent aimed at preventing graft rejection or local administration or shorter-term administration of an immunosuppressive agent with less patient's burdens such as few side effects before and after the administration of the therapeutic drug becomes possible.

It is preferable that the period of use of the immunosuppressive agent is shorter. Considering that invasion by surgery leads to immune induction, it is acceptable to administer an immunosuppressive agent before, during, and/or for 1 week (for 2 weeks, 1 month, or 2 months) or so (or a period of time in which the acute phase of rejection may occur) after the administration of the therapeutic drug of the present embodiment. In this case, the local administration of the immunosuppressive agent is preferred and use of a steroidal anti-inflammatory drug is preferred, as described later.

In order to suppress systemic action, the local administration of the immunosuppressive agent is more preferred and, when using the therapeutic drug according to the present invention, the local administration to an eye is preferred.

Considering the strength of immunosuppression, the presence or the absence of side effects or the degree of concern about side effects, the frequency of occurrence and/or the severity thereof, and the like, examples of the "immunosuppressive agent with a few side effects" include steroidal anti-inflammatory drugs. Accordingly, systemic or local administration of a steroidal anti-inflammatory drug is acceptable when graft of the therapeutic drug of the present embodiment is performed. Use of a steroidal anti-inflammatory drug for a certain period of time is acceptable also in that it is possible to suppress immune induction that invasion by surgery induces.

On the other hand, considering that the therapeutic drug of the present embodiment can strongly suppress the activation of T cells and activated T cells, it is possible to suppress use of calcineurin inhibitors and mTOR inhibitors, which primarily target T cells. It is clinically significant that calcineurin inhibitors and mTOR inhibitors, which are used most often in current transplantation therapies and whose side effects are of great concern, are not used, not administered for a long period of time, not administered systemically, or reduced in the amount of use.

In one aspect of the therapeutic drug of the present embodiment, the intended patient is given systemically, before, during, and/or after the administration of the aforementioned therapeutic drug, no immunosuppressive agent aimed at preventing graft rejection, other than a steroidal anti-inflammatory agent and a calcineurin inhibitor. The intended patient is preferably given systemically no immunosuppressive agent other than a steroidal anti-inflammatory drug.

In one aspect of the therapeutic drug of the present embodiment, preferably no immunosuppressive agent is administered systemically, further preferably no immunosuppressive agent is administered systemically and no immunosuppressive agent other than a steroidal anti-inflammatory drug and a calcineurin inhibitor is administered even locally (in other words, only a steroidal anti-inflammatory drug or a calcineurin inhibitor is administered locally as an immunosuppressive agent), and further preferably no immunosuppressive agent is administered at all.

The expression "not given during the administration of the therapeutic drug" means no administration simultaneously with the administration of the therapeutic drug of the present embodiment or no administration at a timing that is just before or just after the administration of the therapeutic drug and considered to be simultaneous administration, for example, within 24 hours or 12 hours before and after the administration of the therapeutic drug. The expression "not given before the administration of the therapeutic drug" means no administration before the administration of the therapeutic drug of the present embodiment, before timings considered to be coadministration, after at least 1 week before the administration. The expression "not given after the administration of the therapeutic drug" means no administration after the administration of the therapeutic drug of the present invention, after timings considered to be coadministration, within at least 6 months or 1 year.

As for the therapeutic drug of the present embodiment, it is acceptable to administer systemically or locally (preferably locally) a steroidal anti-inflammatory drug and/or a calcineurin inhibitor before, during, or after the administration of the aforementioned therapeutic drug. Moreover, as for the therapeutic drug of the present embodiment, an immunosuppressive agent other than a steroidal anti-inflammatory drug and a calcineurin inhibitor may be administered locally before, during, or after the administration of the aforementioned therapeutic drug. The administration of the immunosuppressive agent can be stopped within 1 year, preferably within 6 months, more preferably within 3 months, more preferably within 2 months, more preferably within 1 month, more preferably within 2 weeks, and more preferably within 1 week, after the administration of the aforementioned therapeutic drug.

The therapeutic drug of the present embodiment may include an effective amount of a retinal tissue. The effective amount of the retinal tissue varies depending on the purpose of administration, the mode of administration, the situation (sex, age, body weight, pathological condition, and the like) of the subject of administration, but may be, for example, 1.0 × 10⁴ cells or more (for example, 1 × 10⁵ cells, 1 × 10⁶ cells, or 1 × 10⁷ cells), for example, in the number of cells.

The therapeutic drug of the present embodiment may include a pharmaceutically acceptable carrier. As the pharmaceutically acceptable carrier, a physiological aqueous solvent (physiological saline, buffer solution, serum-free medium, and the like) may be used. A preservative, a stabilizer, a reducing agent, an isotonizing agent, a pH adjuster, or the like usually used for pharmaceuticals containing tissue or cells to be grafted in transplantation medicine may be mixed as needed.

The therapeutic drug of the present embodiment may be produced as a cell suspension by suspending the retinal tissue with an appropriate physiological aqueous solvent. The therapeutic drug of the present embodiment may be cryopreserved after adding a freeze preservative, if necessary, thawed before use, washed with a buffer solution, and used in transplantation medicine.

The retinal tissue contained in the therapeutic drug of the present embodiment may be, for example, sliced into an appropriate size using tweezers, a knife, scissors, and the like and a part of an aggregate is excised and used for graft. The shape after the excision is not particularly limited, and examples thereof include a cell sheet.

The therapeutic drug of the present embodiment is administered to a patient by graft. The administration to a patient is carried out, for example, by a method of grafting subretinally using a needle or a method of incising a part of an eyeball and grafting from the incision site to a damaged site or a lesion. For administration of the therapeutic drug of the present embodiment, no immunosuppressive agent aimed at preventing graft rejection, other than a steroidal anti-inflammatory agent and a calcineurin inhibitor is administered systemically, before, during, and/or after the administration of the therapeutic drug. For administration of the therapeutic drug of the present embodiment, a steroidal anti-inflammatory agent and a calcineurin inhibitor aimed at preventing graft rejection may be administered systemically, before, during, and/or after the administration of the therapeutic drug and an immunosuppressive agent aimed at preventing graft rejection may be administered locally.

### (Patient)

The intended patient of the therapeutic drug of the present embodiment is a patient affected by a disease accompanied by a disorder of retinal cells or a retinal tissue and the therapeutic drug is a therapeutic drug to graft into the intended patient. The intended patient to be given the therapeutic drug is given systemically, before, during, and/or after the administration of the aforementioned therapeutic drug, no immunosuppressive agent aimed at preventing graft rejection, other than a steroidal anti-inflammatory agent and a calcineurin inhibitor. However, the intended patient may be given systemically, before, during, and/or after the administration of the aforementioned therapeutic drug, a steroidal anti-inflammatory agent and a calcineurin inhibitor aimed at preventing graft rejection and may also be given locally an immunosuppressive agent aimed at preventing graft rejection.

The intended patient is given systemically, preferably, at least 2 months (or at least 1 month) after the administration of the therapeutic drug, none of a steroidal anti-inflammatory agent and/or a calcineurin inhibitor, aimed at preventing graft rejection. In other words, the intended patient may be given systemically or locally, before the administration, during the administration, and/or within 2 months (or 1 month) after the administration of the therapeutic drug, a steroidal anti-inflammatory agent and/or a calcineurin inhibitor, aimed at preventing graft rejection, and may be given locally, but not systemically, at least 2 months (or at least 1 month) after the administration of the therapeutic drug, a steroidal anti-inflammatory agent and/or a calcineurin inhibitor.

The intended patient is given systemically, before, during, and/or after the administration of the therapeutic drug, preferably no immunosuppressive agent aimed at preventing graft rejection. In other words, the intended patient may be given locally, but not systemically, before, during, and/or after the administration of the therapeutic drug, an immunosuppressive agent including a steroidal anti-inflammatory agent and a calcineurin inhibitor aimed at preventing graft rejection.

The intended patient is given locally, before, during, and/or after the administration of the therapeutic drug, preferably no immunosuppressive agent aimed at preventing graft rejection, other than a steroidal anti-inflammatory agent and a calcineurin inhibitor (preferably other than a steroidal anti-inflammatory agent). In other words, the intended patient is given systemically or locally, before, during, and/or after the administration of the therapeutic drug, no immunosuppressive agent aimed at preventing graft rejection, other than a steroidal anti-inflammatory agent and a calcineurin inhibitor (preferably, other than a steroidal anti-inflammatory agent), but may be given systemically or locally (preferably locally) a steroidal anti-inflammatory drug and/or a calcineurin inhibitor. Here, examples of a preferable local administration include Tenon injection or vitreous injection

The intended patient may be given locally, before, during, and/or after the administration of the therapeutic drug, one or more immunosuppressive agents selected from the group consisting of triamcinolone, fluocinolone, and calcineurin inhibitors (cyclosporine, tacrolimus, and the like), aimed at preventing graft rejection. Here, examples of a preferable local administration include Tenon injection or vitreous injection.

The intended patient is given locally, preferably, at least 2 months (or at least 1 month) after the administration of the therapeutic drug, none of a steroidal anti-inflammatory agent and/or a calcineurin inhibitor, aimed at preventing graft rejection.

The intended patient is given, before, during, and/or after the administration of the therapeutic drug, preferably no immunosuppressive agent aimed at preventing graft rejection.

Examples of the disease based on the disorder of a retinal tissue or retinal cells include, but are not limited to, macular degeneration, age-related macular degeneration, retinitis pigmentosa, cataract, glaucoma, corneal diseases, retinopathies, and macular hole, which are eye diseases.

As one aspect of the present invention, a therapeutic drug for a patient having a mismatched HLA type with a retinal tissue (therapeutic drug) for graft and being affected by a disease based on a disorder of a retinal tissue or retinal cells is provided. The patient having a mismatched HLA type with a retinal tissue for graft is a patient for which, for example, 1, 2, 3, or all of the HLAs selected from the group consisting of HLA-A, B, C, and DR are mismatched. A person skilled in the art can determine the degree of matching of the HLA type by well-known technologies such as a serologic test and an examination of DNA type.

As one aspect of the present invention, a therapeutic drug for a patient affected by a disorder of the blood-retinal barrier is provided. It is considered that immune responses become easy to occur and graft rejection becomes easy to occur if the blood-retinal barrier is affected by a disorder. The therapeutic drug of the present embodiment induces no immune responses and is rather capable of suppressing immune responses. Therefore, therapeutic drug of the present embodiment can be used even for a patient affected by a disorder of the blood-retinal barrier with reduced use of an immunosuppressive agent. The disorders of the blood-retinal barrier include disorders due to surgery. It can be determined whether the blood-retinal barrier is affected by a disorder by the examination of the fundus, fundus fluorescence photography, or an optical coherence tomograph (OCT) examination. Examples of the disease with a disorder of the blood-retinal barrier are age-related macular degeneration, diabetic retinopathy, Behcet disease, and sarcoidosis.

In the organ graft, there are patients who cannot be grafted or who are forced to have long-term hospitalization or go to hospital and to have various examinations for sufficient follow-up, even if graft is possible. Particularly QOL of these patients decreases greatly. Examples of a cause thereof include contraindications for immunosuppressive agents essential to be used in combination, contraindications for coadministration, and patients who require careful administration. Specific examples thereof include elderly persons, pregnant women, women who may be pregnant, nursing mothers, children (including low birth weight infants, newborn infants, and infants), patients with infection, carriers of viruses such as hepatitis B or hepatitis C virus, patients with malignant tumor or a history thereof, patients with renal dysfunction, patients with liver dysfunction, and patients with pancreatic dysfunction. The therapeutic drug of the present embodiment uses no immunosuppressive agent or can reduce the amount of an immunosuppressive agent used. Accordingly, in one embodiment, a therapeutic drug is provided to one or more patients selected from the group consisting of elderly persons, pregnant women, women who may be pregnant, nursing mothers, children (including low birth weight infants, newborn infants, and infants), patients with infection, carriers of viruses such as hepatitis B or hepatitis C virus, patients with malignant tumor or a history thereof, patients with renal dysfunction, patients with liver dysfunction, and patients with pancreatic dysfunction.

As one aspect of the retinal tissue of the present embodiment, provided is a retinal tissue that is of an allogeneic origin, has a three-dimensional structure, and is for use in treatment of a disease accompanied by a disorder of retinal cells or a retinal tissue in a patient affected by a disease accompanied by a disorder of retinal cells or a retinal tissue, to be given systemically no immunosuppressive agent aimed at preventing graft rejection, other than a steroidal anti-inflammatory agent and a calcineurin inhibitor.

Moreover, as another aspect of the retinal tissue according to the present invention, provided is a retinal tissue that is of an allogeneic origin, has a three-dimensional structure, and is for use in treatment of a disease accompanied by a disorder of retinal cells or a retinal tissue, wherein upon administration of the retinal tissue, no immunosuppressive agent aimed at preventing graft rejection, other than a steroidal anti-inflammatory agent and a calcineurin inhibitor is administered systemically, before, during, and/or after the administration.

### [Method of treatment]

As one aspect of the method of treatment of the present embodiment, provided is a method of treatment for a disease accompanied by a disorder in retinal cells or a retinal tissue, including administering (grafting) an effective amount of a retinal tissue that is of an allogeneic origin and has a three-dimensional structure to a patient affected by a disease accompanied by a disorder of retinal cells or a retinal tissue, wherein the intended patient is given systemically, before, during, and/or after the administration (graft) of the retinal tissue, no immunosuppressive agent aimed at preventing graft rejection, other than a steroidal anti-inflammatory agent and a calcineurin inhibitor. The retinal tissue is grafted to the patient as a formulation, specifically a formulation for graft.

As one aspect of the method of treatment of the present embodiment, provided is a method of treatment for a disease accompanied by a disorder in retinal cells or a retinal tissue, including administering an effective amount of a retinal tissue that is of an allogeneic origin and has a three-dimensional structure to a patient affected by a disease accompanied by a disorder of retinal cells or a retinal tissue, wherein for administration of the retinal tissue, no immunosuppressive agent aimed at preventing graft rejection, other than a steroidal anti-inflammatory agent and a calcineurin inhibitor is administered systemically, before, during, and/or after the administration. Examples of one aspect of the present invention include a method of administering the therapeutic drug according to the present invention to a patient in which the suppressive effect of the therapeutic drug on immune responses has been observed in pre-examination described above.

Accordingly, as one aspect, the method of treatment of the present embodiment includes a step of inducing differentiation into a retinal tissue having a three-dimensional structure from an allogeneic stem cell, and a step of administering (grafting) an effective amount of the retinal tissue to a patient affected by a disease accompanied by a disorder of retinal cells or a retinal tissue. The intended patient and the mode of administration may be as described above.

As for the retinal tissue, upon the treatment (graft), the retinal tissue may be stored in a vehicle necessary for maintaining viability of the retinal tissue. Examples of the "vehicle necessary for maintaining viability" include a medium and a physiological buffer solution, but the vehicle is not particularly limited as long as the cell population containing retinal cells such as retinal progenitor cells survives and a person skilled in the art can select such a vehicle as appropriate. Examples thereof include media prepared from media usually used for culture of animal cells as basal media. Examples of the basal media include media that can be used in culture of animal cells such as BME medium, BGJb medium, CMRL 1066 medium, Glasgow MEM (GMEM) medium, Improved MEM Zinc Option medium, Neurobasal medium, IMDM medium, Medium 199, Eagle MEM medium, αMEM medium, DMEM medium, F-12 medium, DMEM/F12 medium, IMDM/F12 medium, Ham's medium, RPMI 1640 medium, Fischer's medium, or a mixed medium thereof.

The graft is carried out, for example, by a method of grafting subretinally using a needle or a method of incising a part of an eyeball and grafting from the incision site to a damaged site or a lesion.

### [Kit]

As one embodiment, provided is a kit including: a retinal tissue differentiated from an allogeneic stem cell and having a three-dimensional structure and a written order, a manual, an attached document, or a product label indicating that, on or after the administration of a therapeutic drug to a patient affected by a disease accompanied by a disorder of retinal cells or a retinal tissue, (1) no immunosuppressive agent is administered, (2) no immunosuppressive agent is systemically administered (including the case where there is a statement limiting the period that no immunosuppressive agent is systemically administered for one month or more after the administration of the therapeutic drug), (3) immunosuppressive agents are only administered locally, (4) as an immunosuppressive agent(s), only a steroidal anti-inflammatory drug(s) or a calcineurin inhibitor(s) (preferably only a steroidal anti-inflammatory drug(s)) is administered systemically or locally, or (5) a statement that is substantially equal to stating any of (1) to (4) described above. Examples of the statement that is substantially equal to stating any of (1) to (4) include disclosing non-clinical or clinical data of such a degree that typical physicians can understand that administration according to (1)-(4) is possible. The aforementioned data may be of any animal species, is also not limited to in vivo data, and includes data indicating immune tolerance or immunosuppression effect in vitro. Furthermore, the kit may include an instrument(s) for graft (examples: needles and syringes for ocular administration), a vehicle(s) for graft, a vehicle(s) necessary for maintaining viability of retinal tissue, and the like.

It is preferred that the retinal tissue and the written order, manual, attached document, or product label are included in a package together. The written order, manual, attached document, or product label may be a print or electronic information posted on the Internet or the like. In the case of electronic information, it is preferred that a method of obtaining the information is included in the package.

### Examples

The present invention will be described in detail with reference to Examples below, but the present invention is not limited to these Examples.

All animals used in the following Examples were treated according to Association for Research in Vision and Ophthalmology statement for the Animals in Ophthalmology and Vision Research. The animal experiments were approved by the BDR Ethical Review Board, the Institute of Physical and Chemical Research, and conducted based on the guideline on animal experiments of the Biodynamics Research Center, the Institute of Physical and Chemical Research.

### <Example 1: Preparation of neural retina (NR) from human ES/iPS cells by SFEBq method>

The human ES cells genetically modified to have the Crx::Venus reporter gene (Strain KhES-1, (Non Patent Literature 3)) and human iPS cells (Strain TLHD2) established in the Institute of Physical and Chemical Research were cultured under a feeder-free condition following a method described in "Scientific Reports, 4, 3594 (2014)". The StemFit medium (trade name: AK03N, manufactured by Ajinomoto Co., Inc.) was used as a feeder-free medium and Laminin511-E8 (trade name, manufactured by Nippi, Incorporated) was used as scaffolding substituting for feeder cells.

The strains KhES-1 and TLHD2 expressed Oct3/4, Nanog, and SSEA-4, which are pluripotent markers in the feeder-free medium.

Specific maintaining culture operations for human ES and human iPS cells (human ES/iPS cells) were conducted as follows. First, subconfluent (about 60% of the culture area were covered with cells) human ES/iPS cells (the strains KhES-1 and TLHD2) were washed with PBS and then dispersed into single cells using TrypLE Select (trade name, manufactured by Life Technologies). Subsequently, the human ES/iPS cells dispersed into single cells were seeded in the presence of Y27632 (a ROCK inhibitor, 10 µM) in a plastic culture dish coated with Laminin511-E8 and cultured under a feeder-free condition in the StemFit medium. When a 6 well plate (manufactured by Iwaki Co., Ltd., for cell culture, 9.4 cm² in culture area) is used as the plastic culture dish, the seeding cell count of the human ES/iPS cells dispersed into single cells was 0.4-1.2 × 10⁴ cells per well. One day after the seeding, the medium was changed with the StemFit medium without Y27632. After that, the medium was changed with the StemFit medium without Y27632 once in 1-2 days. Subsequently, the human ES/iPS cells were cultured under feeder-free conditions until 1 day before reaching subconfluent. The human ES/iPS cells on 1 day before reaching subconfluent were cultured in the presence of SB431542 (a TGFβ signaling pathway inhibitor, 5 µM) and SAG (an Shh signaling pathway agent, 300 nM) (precondition treatment) under feeder-free conditions for 1 day.

The human ES/iPS cells were washed with PBS, then treated with a cell dispersion liquid using TrypLE Select, and dissociated into single cells by further pipetting manipulation and then the human ES/iPS cells dissociated into single cells were floated in 100 µL of a serum-free medium and cultured in the floating state at 1.2 × 10⁴ cells per well in a 96-well non-cell-adhesive culture plate (trade name: PrimeSurface 96-well V bottom plate, manufactured by Sumitomo Bakelite Co., Ltd.) at 37°C, 5% CO₂. For the serum-free medium (gfCDM + KSR) in this culture, a serum-free medium, which was a 1:1 mixture of the F-12 medium and the IMDM medium supplemented with 10% KSR, 450 µM 1-monothioglycerol, and 1× Chemically defined lipid concentrate, was used.

On starting the floating culture (Day 0 after starting the floating culture), Y27632 (a ROCK inhibitor, final concentration 20 µM) and SAG (an Shh signaling pathway agent, 300 nM or 30nM, 0nM) were added to the serum-free medium. On Day 3 after starting the floating culture, 50 µL of a medium containing exogenous human recombination BMP4 in a final concentration of 1.5 nM was added using a medium containing no Y27632 nor SAG and containing human recombinant BMP4 (trade name: Recombinant Human BMP-4, manufactured by R & D Systems, Inc.). On Day 6 or more after starting the floating culture, a half amount of the medium was changed once in 3 days with the medium containing none of Y27632 and SAG and human recombinant BMP4.

Aggregates on Day 15 to Day 18 after the starting the floating culture were transferred into a 90 mm low adhesive culture dish (manufactured by Sumitomo Bakelite Co., Ltd.) and cultured in a serum-free medium (DMEM/F12 medium supplemented with 1% N₂ Supplement) containing substance acting on a Wnt signaling pathway (CHIR99021, 3 µM) and an FGF signaling pathway inhibitor (SU5402, 5 µM) for 3-4 days at 37°C, 5% CO₂. Subsequently, they were cultured in a DMEM/F12 medium without a substance acting on a Wnt signaling pathway and FGF signaling pathway inhibitor and containing serum (which may be, hereinafter, also referred to as the NucT0 medium) in a 90 mm low adhesive culture dish (manufactured by Sumitomo Bakelite Co., Ltd.). On Day 40 or more after starting the floating culture (induction of differentiation), they were cultured in a serum medium without substance acting on a Wnt signaling pathway and FGF signaling pathway inhibitor (a mixed medium of the NucT0 medium and the NucT2 medium, which may be, hereinafter, also referred to as the NucT1 medium). On Day 60 or more after starting the floating culture (induction of differentiation), they were cultured in a Neurobasal medium containing the thyroid hormone signaling pathway agent T3 (which may be, hereinafter, also referred to as the NucT2 medium) until Day 80 to Day 95 after starting the floating culture (induction of differentiation).

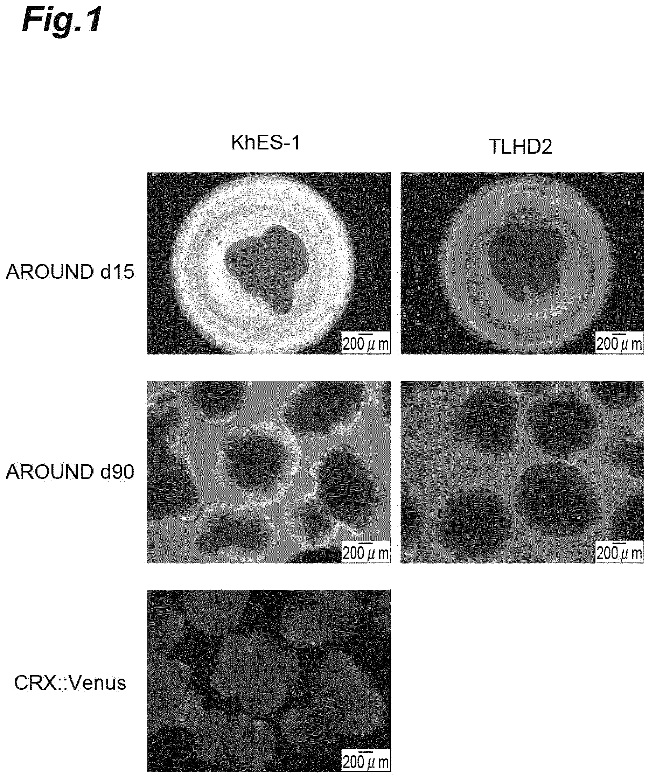

As a result of observation using a microscope, it was found that neural retina (NR) having a layered structure are formed on Day 80 to Day 95 after induction of differentiation in a human ES cell line (khES-1) and a human iPS cell line (TLHD2) (Figure 1).

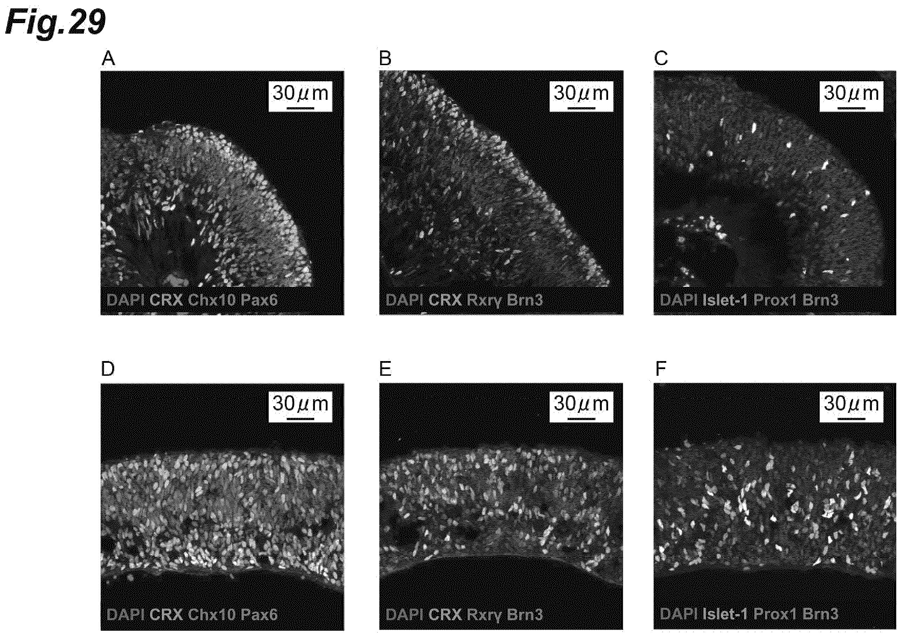

Figure 29 is photomicrographs showing the extent of expression of CRX, Chx10, Pax6, Rxr-γ, Brn3, Islet-1, Proxl in the neural retina hES-NR (A-C) and hiPSC-NR (D-F), each differentiated from human ES cells and human iPS cells on Day 80 after starting the floating culture produced in the above-described method. DAPI was used for the nucleus staining. The scale bar in Figure 29 represents 30 µm.

In the middle of the cell layers of hES-NR and hiPSC-NR, Chx10 and Pax6, which are markers for neural retina progenitor cells, were expressed (Figure 29A, D). In the basal membrane side of the neural retina, Pax6 and Brn3, which is a marker for retinal ganglion cells, were expressed (Figure 29A-B, D-E). On the stage Day 80 after starting the floating culture, Crx, which is a marker for photoreceptor precursors, and Rxr-γ, which is a marker for cone cells, cone photoreceptor progenitor cells, and ganglion cells, were expressed in layers in the apical surface side (Figure 29B, E). Islet-1 and Proxl were expressed in both of the apical surface side and the basal membrane side (Figure 29C, F). Islet-1 and Proxl are markers for inner retinal cells in the early differentiation period.

Most Crx was located with Rxr-γ, but not with Brn3, in layers in the apical surface side (Figure 29B, E).

hES-NR and hiPSC-NR expressed Islet-1, Proxl and Brn3 (Figure 29C, F). This result indicates the existence of retinal ganglion cells, amacrine cells, and horizontal cells.

### <Example 2: HLA expression analysis 1 of neural retina>

Recombination IFN-γ (100 ng/mL) (manufactured by R & D Systems, Inc.) was added into the culture liquid of the neural retina derived from human ES/iPS cells on Day 80 to Day 100 after starting the floating culture prepared in Example 1 and the neural retina were cultured for 2 days. Meanwhile, a group to which no recombinant IFN-γ was added was also prepared.

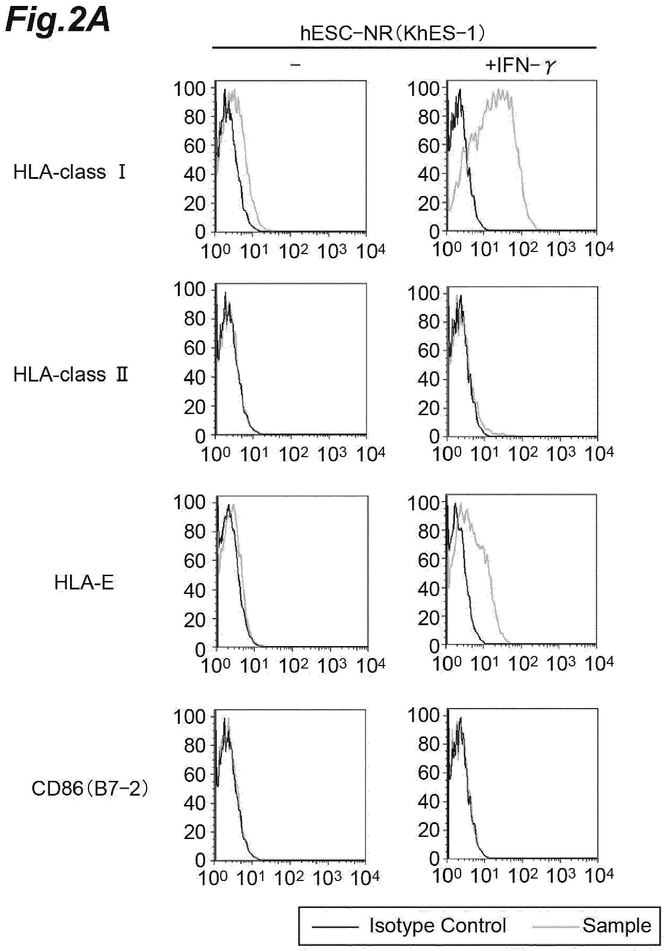

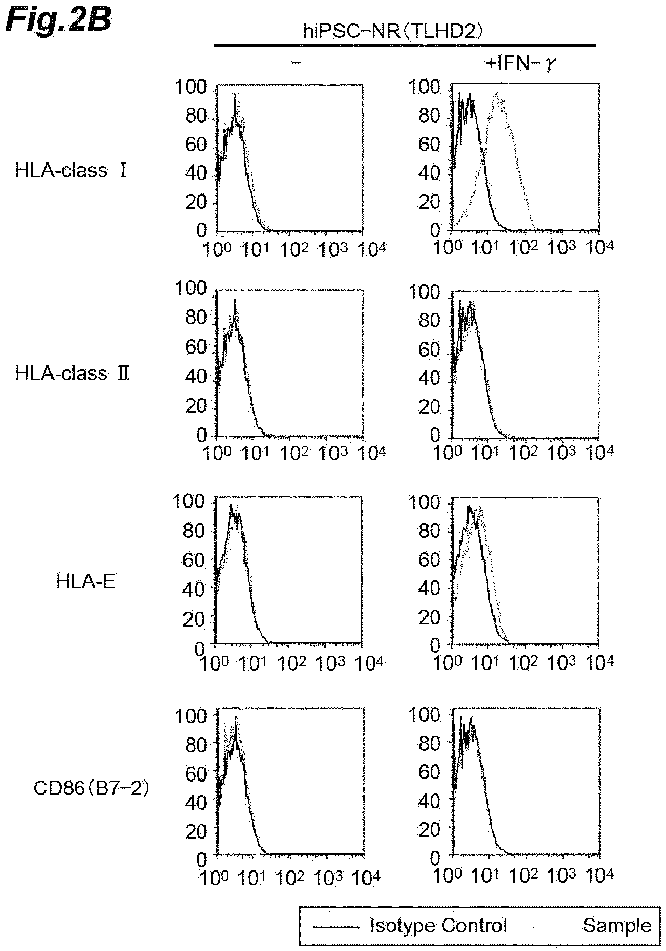

2 days later, the neural retina derived from human ES/iPS cells were washed with PBS and a neural cell dispersion liquid (manufactured by WAKO) was added. After incubation at 37°C, they were dispersed into single cells by pipetting. These cells were immunostained using an anti-HLA class I antibody (FITC anti-human HLA-A, -B, -C, Sigma-Aldrich, #F5662), an anti-HLA class II antibody (FITC anti-human HLA-DR, -DP, -DQ, DakoCytomation, #F0817 or BD PharMingen, #555558), an anti-HLA E antibody (Biolegend, #342604), an anti-CD86 (B7-2) antibody (eBioscience, #12-0862), and an isotype antibody (mouse IgG2a, κ isotype control, FITC, BD PharMingen, #555573 or mouse IgG2b, κ isotype control, FITC, eBioscience, #11-4732) as a control. These immunostained cells were measured using a flow cytometry (FACSCanto flow cytometer, manufactured by BD Biosciences) and analyzed using FlowJo software. As a result, under the conditions (-) in which no recombinant interferon γ was added, it was found that the neural retina derived from human ES/iPS cells have little expression of HLA-class I and no expression of HLA-class II, HLA-E, and CD86. On the other hand, under the conditions (+IFN-y) in which recombinant interferon γ was added, it was found that there is expression of HLA-class I, but it is low, there is little expression of HLA-E, and there is no expression of HLA-class II and CD86 (Figure 2A and Figure 2B).

From these results, it was found that neural retina differentiated from human ES/iPS cells have very low immunogenicity.

### <Example 3: HLA expression analysis 2 of neural retina>

To the culture liquid of neural retina derived from human ES cells on Day 27, Day 91, Day 149, Day 239 after starting the floating culture prepared in Example 1 was added recombinant IFN-γ (100 ng/mL) (manufactured by R & D Systems, Inc.) and the cultures were cultured for 2 days. Meanwhile, a group to which without recombinant IFN-γ was added was also prepared. The neural retina derived from human ES cells on Day 27, Day 91, Day 149, Day 239 after starting the floating culture typically have the following characteristics, respectively.
Day 27 after starting the floating culture: Chx10+ neural retina progenitor cells are present in predominance and Brn3+/Pax6+ ganglion cells are also present.
Day 91 after starting the floating culture: Rxr-y+/recoverin+ cone photoreceptor cells begin to appear.
Day 149 after starting the floating culture: The number of various Crx+ photoreceptor cells in the apical surface increases.
Day 239 after starting the floating culture: PKCα+ rod bipolar cells, GS+/GFAP- Muller glial cells begin to appear.

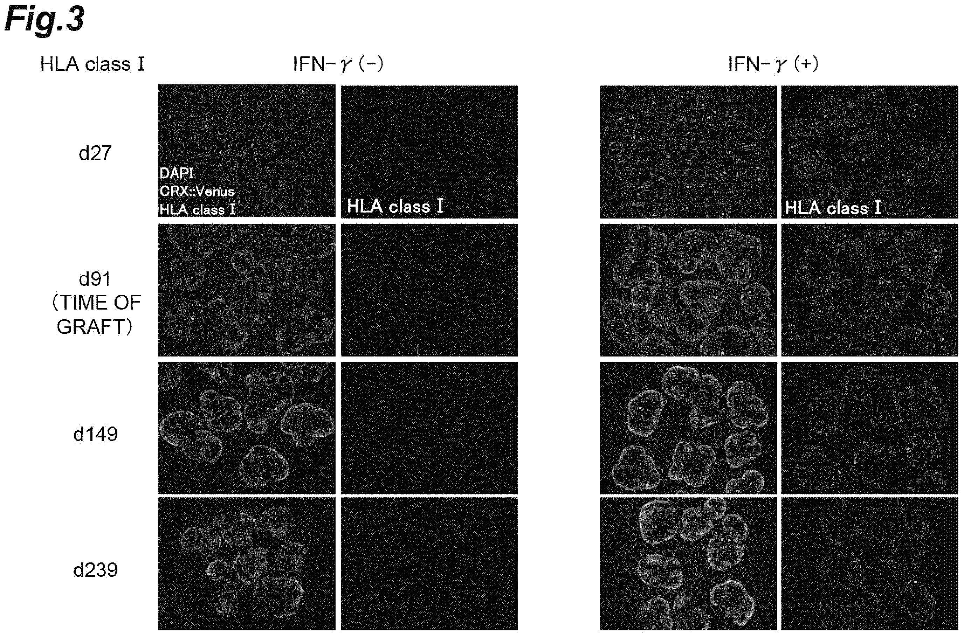

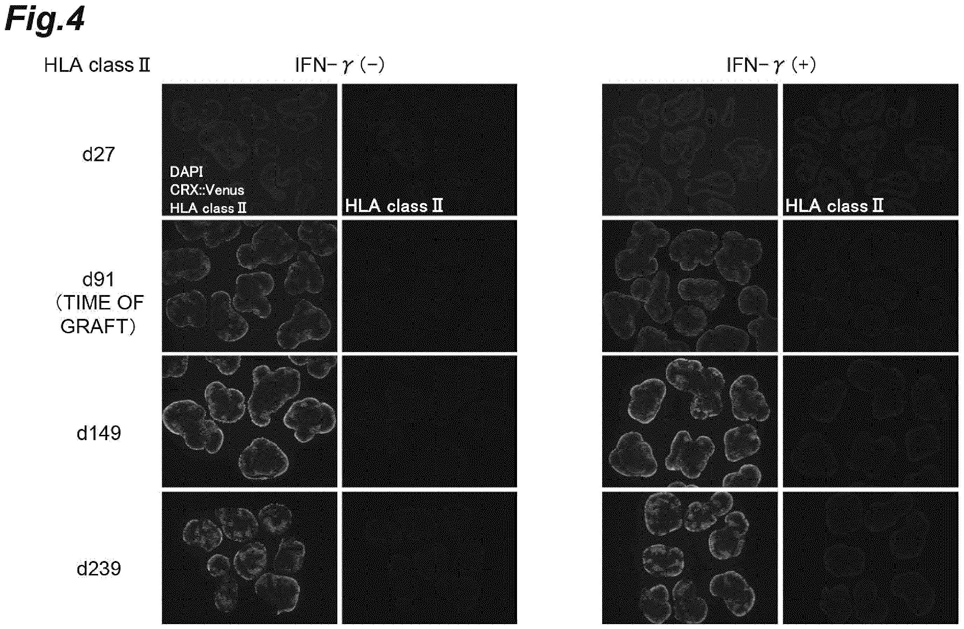

2 days later, the neural retina derived from human ES/iPS cells were washed with PBS and fixed at 4°C using 4% PFA for 15 minutes. After washing with PBS, they were soaked in a 30% sucrose solution. Subsequently, they were embedded in Cryomold using OCT Compound and then 12 µm sections were made with a cryostat. These sections were immunostained using an anti-HLA class I antibody (eBioscience, #14-9983), an HLA class II antibody (BD pharmingen, #555557). These immunostained cells were observed with a fluorescence microscope (Keyence Corporation). As a result, it was found that, for neural retina at any differentiation stage, the neural retina derived from human ES/iPS cells have little expression of HLA-class I and no expression of HLA-class II under the conditions in which no recombinant interferon γ is added. On the other hand, it was found that under the conditions in which recombinant interferon γ is added, there is expression of HLA-class I, but it is low, and there is no expression of HLA-class II (Figure 3 and Figure 4).

### <Example 4: HLA expression analysis 3 of neural retina>

The neural retina derived from human ES cells on Day 80 to Day 100 after starting the floating culture prepared in Example 1 were grafted subretinally to immunocompromised retinal degenerative rats.

After 5 months or more after graft, the eyes were removed, washed with PBS, and fixed with 4% PFA at room temperature for 60 minutes. After washing with PBS, they were soaked in a 30% sucrose solution. Subsequently, they were embedded in Cryomold using OCT Compound and then 12 µm sections were made with a cryostat. These sections were immunostained using anti-HLA class I antibody (eBioscience, #14-9983), HLA class II antibody (BD pharmingen, #555557), Stem121 antibody (TaKaRa), Stem123 (human specific GFAP; hGFAP) (TaKaRa). These immunostained sections were observed with a fluorescence microscope (Keyence Corporation).

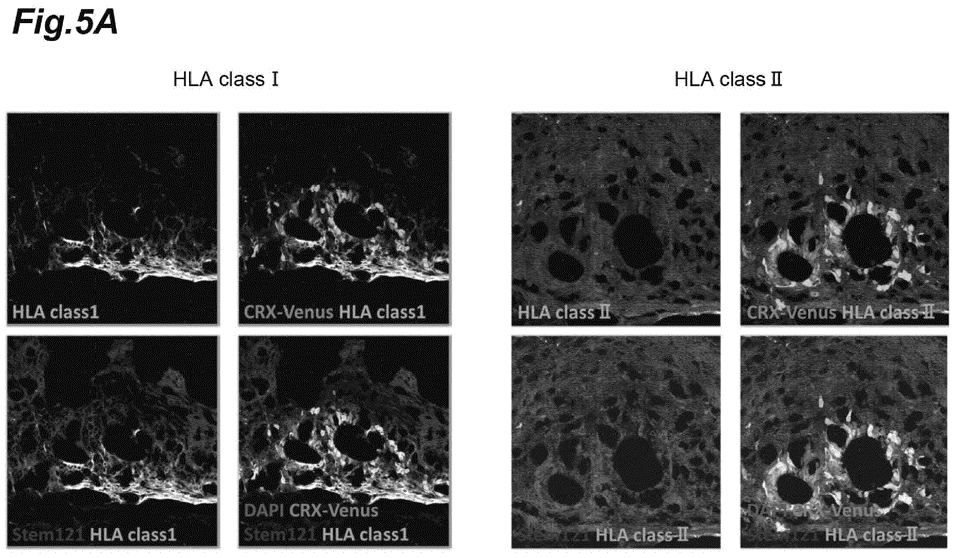

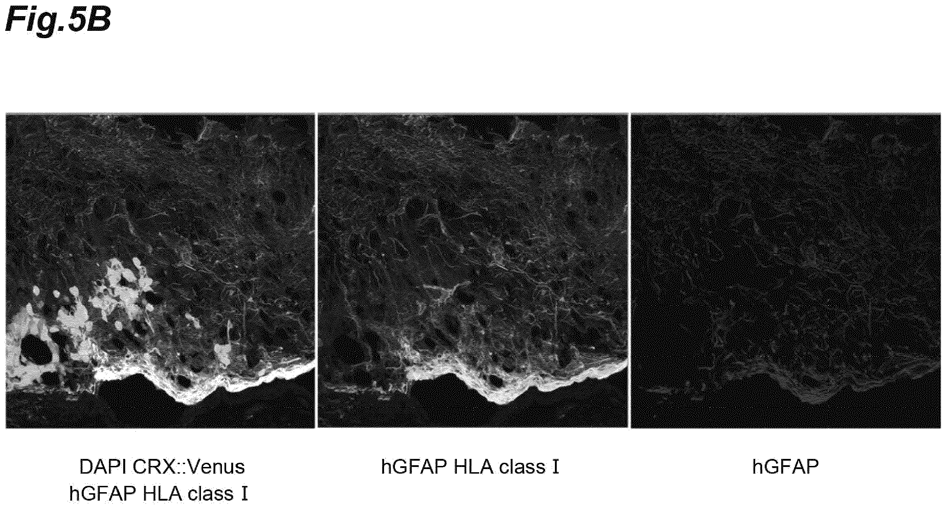

As a result, it was found that in the neural retina derived from human ES cells, some cells have the expression of HLA-class I, but the majority of photoreceptor cells do not, and there is no expression of HLA-class II (Figure 5A). Moreover, it was found that the cells expressing HLA class I after graft are likely to be Muller cells derived from the graft since the cells expressing HLA-class I are also stained with hGFAP (Figure 5B).

### <Example 5: Evaluation 1 by immunogenic test of neural retina to immune cells>

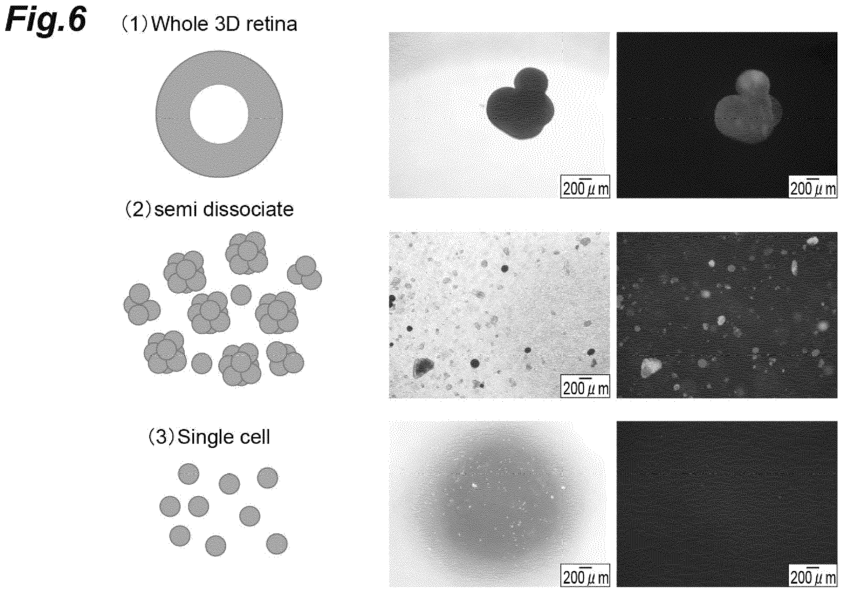

As samples for evaluating immunogenicity of neural retina to immune cells, (1) the neural retina derived from human ES/iPS cells on Day 80 to Day 100 after starting the floating culture prepared in Example 1 (samples of neural retina not dispersed into single cells (Whole retina)); (2) samples in the semi dissociated state (Semi dissociate) obtained by washing the neural retina of (1) with PBS, then adding Neuron dissociation solution (manufactured by Sumitomo Bakelite Co., Ltd.) to the neural retina, incubating at 37°C, and then gently pipetting; and (3) samples obtained by completely dissociating the neural retina of (1) into single cells were prepared. The blood was collected from healthy subjects from which informed consent was obtained and mixed culture of each of the aforementioned (1), (2), and (3) from the collected PBMCs (peripheral blood mononuclear cells) was conducted for 2 days using a low adhesive 24-well plate (manufactured by Sumitomo Bakelite Co., Ltd.). Microscopic results of (1), (2), and (3) for the neural retina derived from human ES are shown in Figure 6.

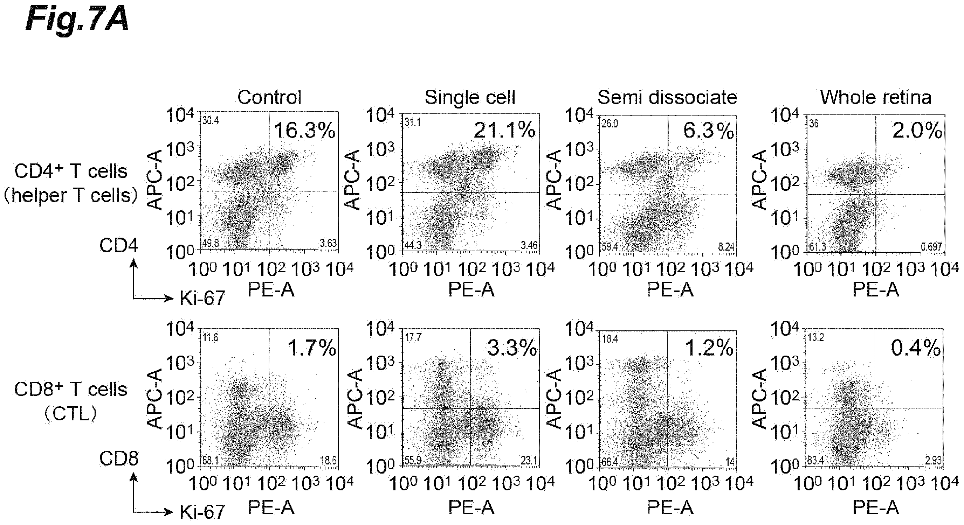

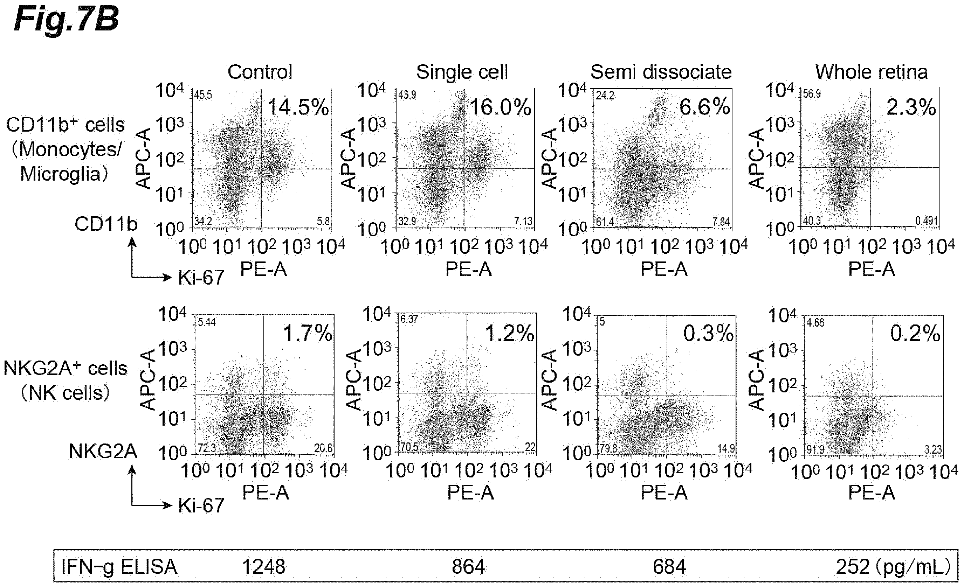

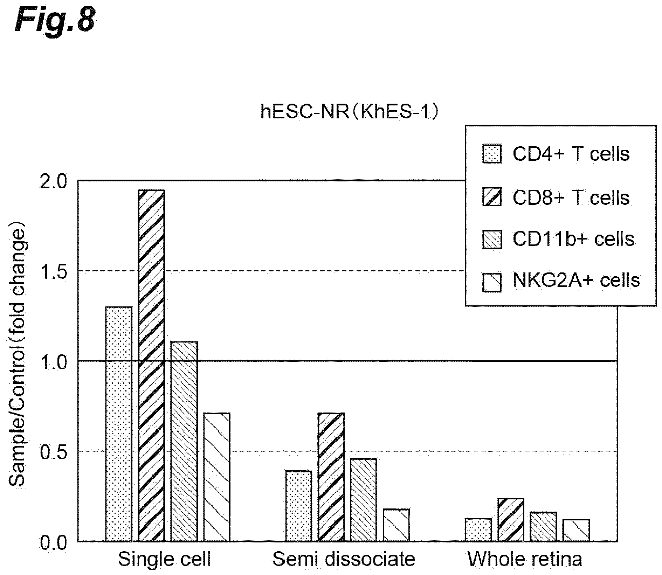

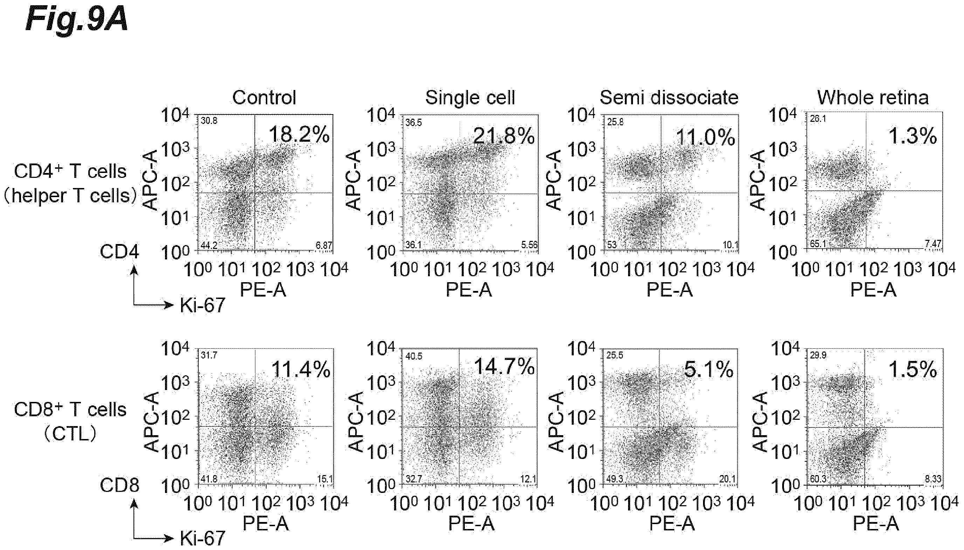

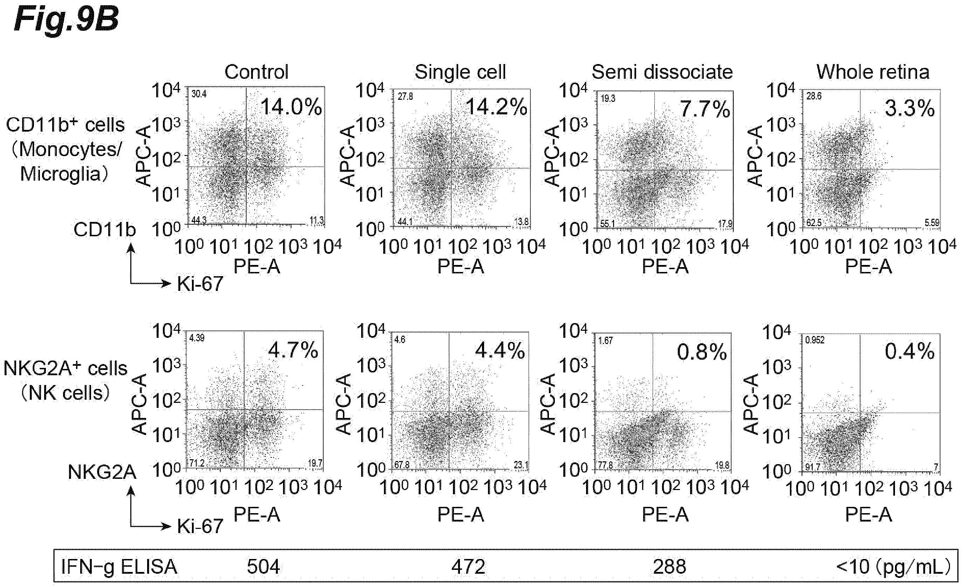

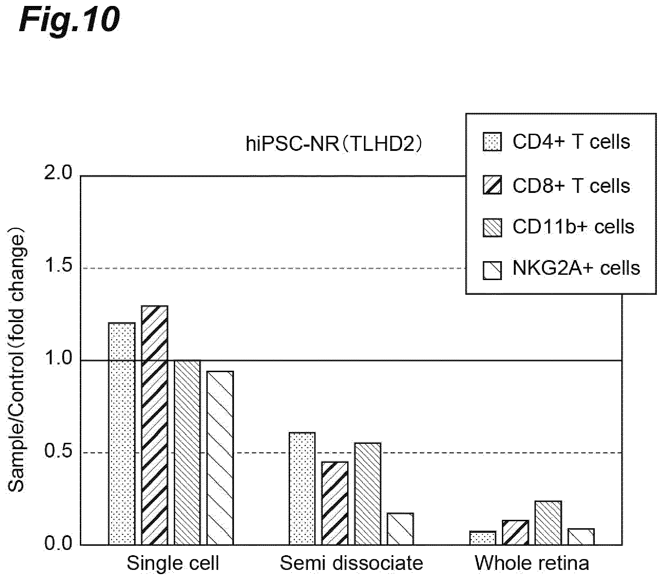

2 days later, PBMCs were collected, washed with PBS, and then immunostained using an anti-APC-CD4 antibody (manufactured by BioLegend, #317416), an anti-APC-CD8 antibody (manufactured by eBioscience, #17-0088-42), an anti-APC-CD11b antibody (manufactured by Miltenyi Biotec, #130-091-241), an anti-APC-NKG2A antibody (manufactured by Miltenyi Biotec, #130-098-809), and an anti-PE-Ki-67 antibody (BioLegend, #350504). Moreover, as control antibodies, mouse IgG1κ isotype control APC (Miltenyi Biotec, #130-113-196) and mouse IgG1) κ isotype control phycoerythrin (PE) (BioLegend, #400112) were used. These immunostained cells were measured using a flow cytometry (FACSCanto flow cytometer, manufactured by BD Biosciences) and analyzed using FlowJo software. Figure 7A, Figure 7B, and Figure 8 show results of analysis when the neural retina differentiated from human ES cells were used and Figure 9A, Figure 9B, and Figure 10 show results of analysis when the neural retina differentiated from human iPS cells were used.

As a result, under the conditions in which the cells were mixed with the sample of (3) in which the neural retina were dissociated into single cells, very weak rejection of CD4-positive T cells, CD8-positive T cells, CD11b-positive monocytes, and microglia was confirmed. On the other hand, it was found that the neural retina that were not dissociated into single cells of (1) and the sample in a state of Semi dissociated of (2) do not cause immunorejection in CD4-positive T cells, CD8-positive T cells, CD11b-positive monocytes, microglia, and NKG2A-positive NK cells, in contrast to (3).

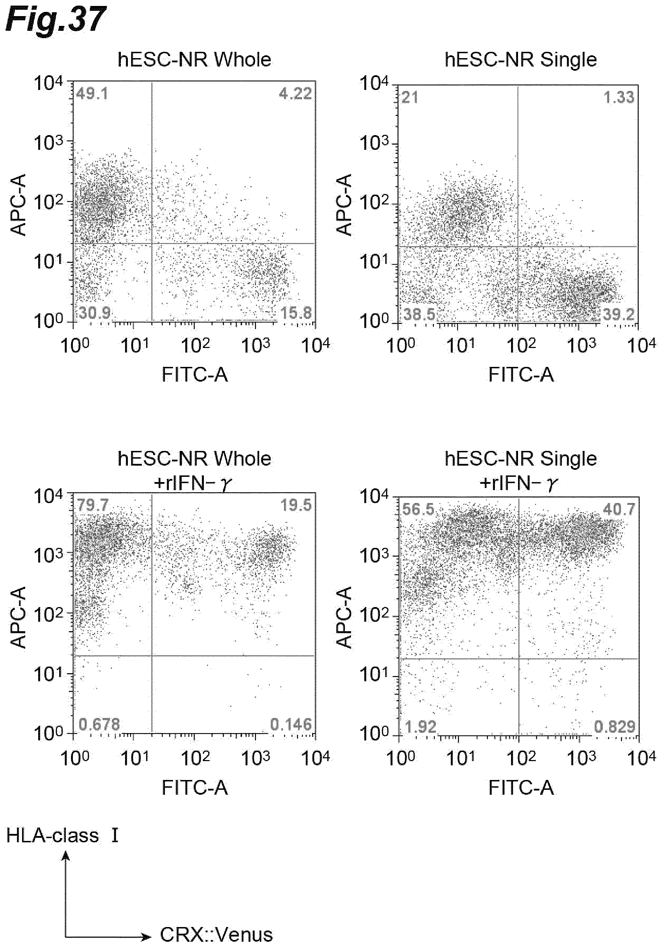

Moreover, as for expression of HLA class I in the neural retina derived from human ES cells, no big difference was found between the sample of (1) (Whole retina) and the sample of (3) (Figure 37). On the other hand, as shown in Figure 7B and Figure 9B, it was indicated, as a result of measuring IFN-γ by ELISA, that samples of (1) (Whole retina) from both the neural retina derived from human ES cells and the neural retina derived from human iPS cells strongly suppress the expression of INF-γ by PBMCs (comparison of the samples of (1) (Whole retina) with the samples of (2) (semi dissociate), the samples of (3) (single cell), and the control).

From these results, it was found that the neural retina derived from human ES/iPS cells and semi dissociate do not undergo immune rejection, but, on the contrary, suppress immune cells.

### <Example 6: Evaluation of ability to suppress activation of immune cells (activated state of immune cells)>

As samples for evaluating the ability to suppress immune cells activated, the neural retina (samples of neural retina that are not dispersed into single cells) derived from human ES/iPS cells on Day 80 to Day 100 after starting the floating culture prepared in Example 1 were provided by stimulating through addition of CD3, CD28 antibodies. The blood was collected from healthy subjects from which informed consent was obtained and mixed culture of the collected PBMCs (peripheral blood mononuclear cells) was conducted for 2 days using a 24-well plate (manufactured by Sumitomo Bakelite Co., Ltd.).

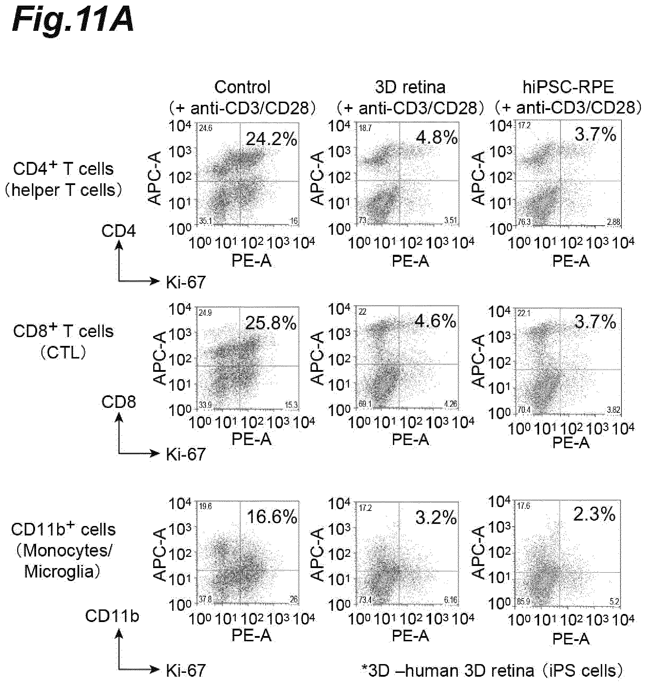

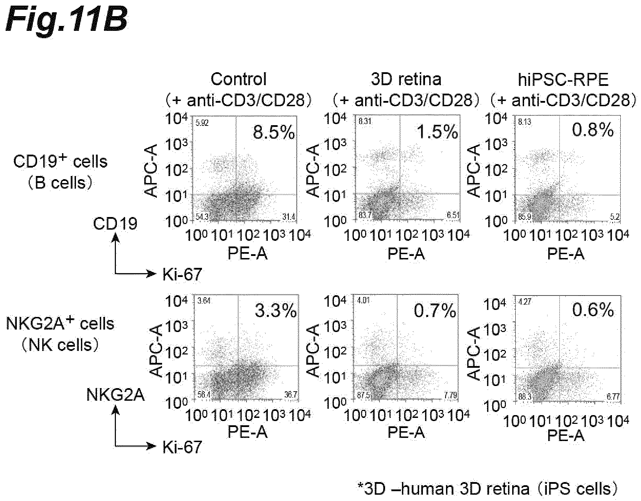

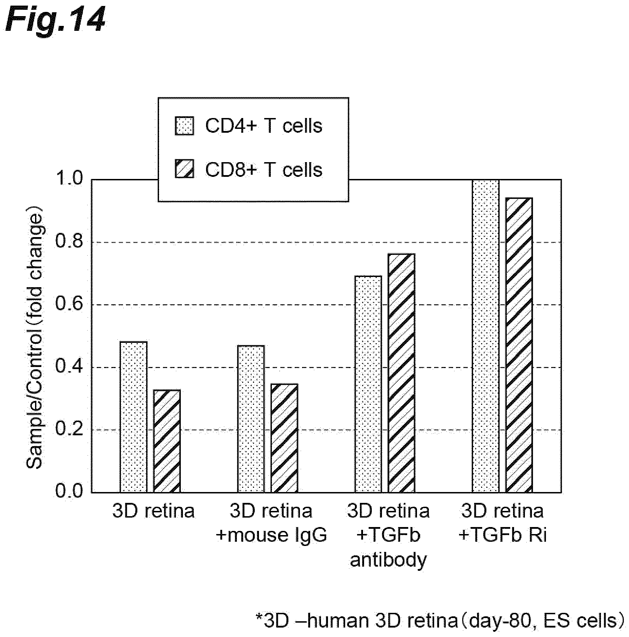

2 days later, PBMCs were collected, washed with PBS, and then immunostained using an anti-APC-CD4 antibody (manufactured by BioLegend, #317416), an anti-APC-CD8 antibody (manufactured by eBioscience, #17-0088), an anti-APC-CD11b antibody (manufactured by Miltenyi Biotec, #130-091-241), an anti-APC-CD19 antibody, an anti-NKG2A antibody, an anti-PE-Ki-67 antibody (BioLegend, #350504). Moreover, as isotype antibodies, mouse immunoglobulin 2a (IgG2a) κ isotype control fluorescein isothiocyanate (FITC) (BioLegend, #400208), mouse IgG1κ isotype control APC, and mouse IgG1 (BioLegend, #400122) κ isotype control phycoerythrin (PE) (BioLegend, #400112) were used. These immunostained cells were measures using a flow cytometry (FACSCanto flow cytometer, manufactured by BD Biosciences) and analyzed using FlowJo software. Figures 11-14 show results of analysis when the neural retina differentiated from human iPS cells were used.

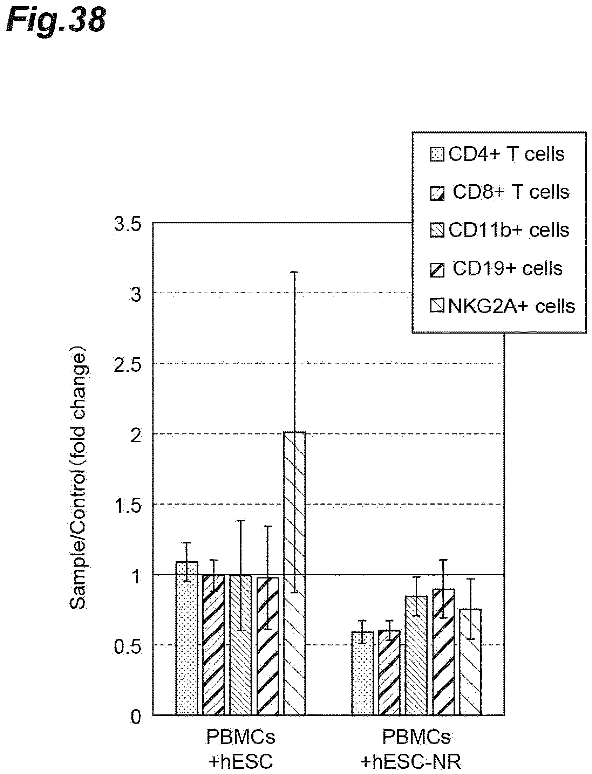

As a result, it was found that they suppress the activation in activated CD4-positive T cells, CD8-positive T cells, CD11b-positive monocytes, CD19-positive B cells, NKG2A-positive NK cells (Figure 11A, Figure 11B, Figure 12, Figure 38). This effect was effect at the same level as RPE cells derived from human iPS cells (Figure 11A, Figure 11B).

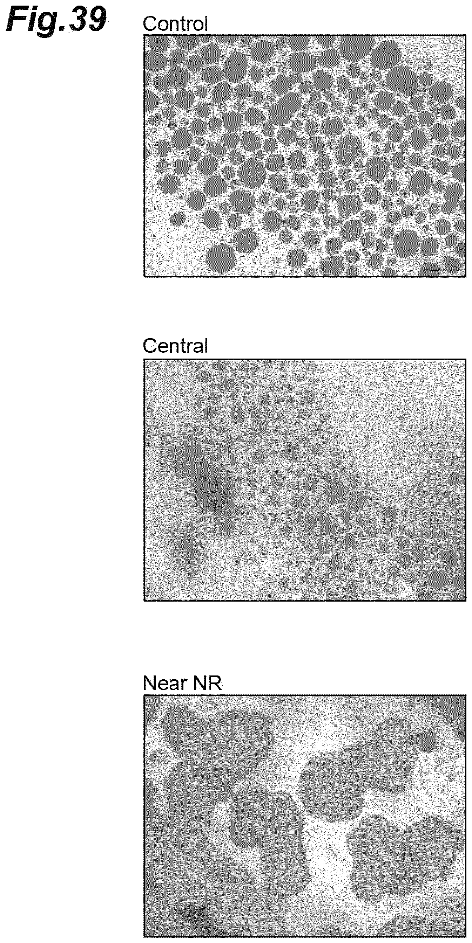

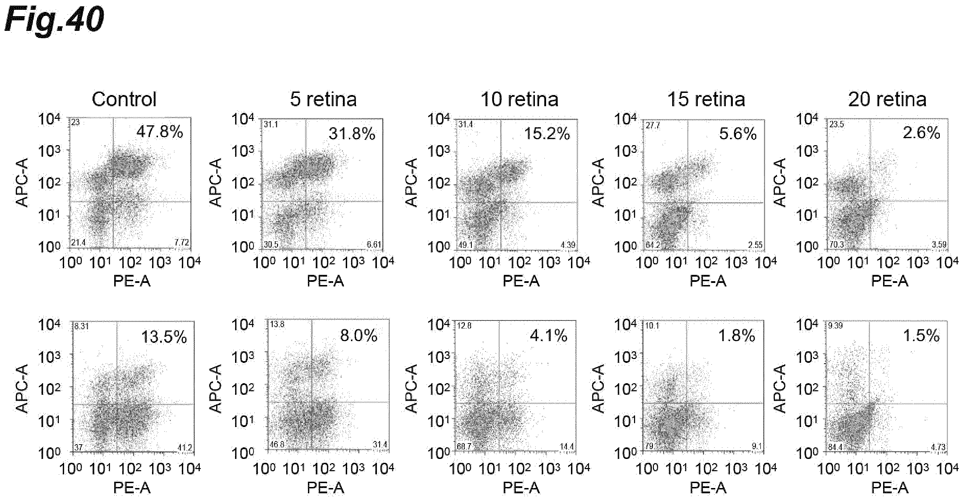

The control PBMCs (PBMCs not cocultured with neural retina derived from human ES cells) proliferated and formed large aggregates, but aggregates of PBMCs cocultured with neural retina derived from human ES cells were small, and the degree of the aggregation was smaller as getting closer to the neural retina derived from human ES cells (Figure 39). The suppressive effect of hESC-NR was apparent near neural retina (hES-NR) differentiated from human ES cells in co-culture dishes. Moreover, the suppressive effect of the neural retina derived from human ES/iPS cells on the immune cells in the activated state had dose-dependency (Figure 40).

From these results, it was found that the neural retina derived from human ES/iPS cells suppress the activated state of immune cells also for immune cells in the activated state. Thus, it can be said that the neural retina derived from human iPS cells have immunosuppressive capacity.

For the culture assay using Transwell (R), a Transwell chamber having a membrane (Corning Costar, Cambridge, MA) with a pore size of 0.3 mm, the aforementioned PBMCs (peripheral blood mononuclear cells), and RPMI 1640 (Sugita et al 2015) containing a recombinant IL2 as a medium were prepared.

In the presence of the medium, the PBMCs were added to a lower well in the Transwell plate and the neural retina derived from human ES cells were added in an upper well. On the upper well, the neural retina derived from human ES cells were cultured for 4-5 days. After the end of the 5-day culturing, the PBMCs were collected. The collected PBMCs were stained using a PE conjugated Ki67 antibody and APC conjugated CD4, CD8, CD11b, CD19, NKG2A antibodies and the proliferation was evaluated by flow cytometry.

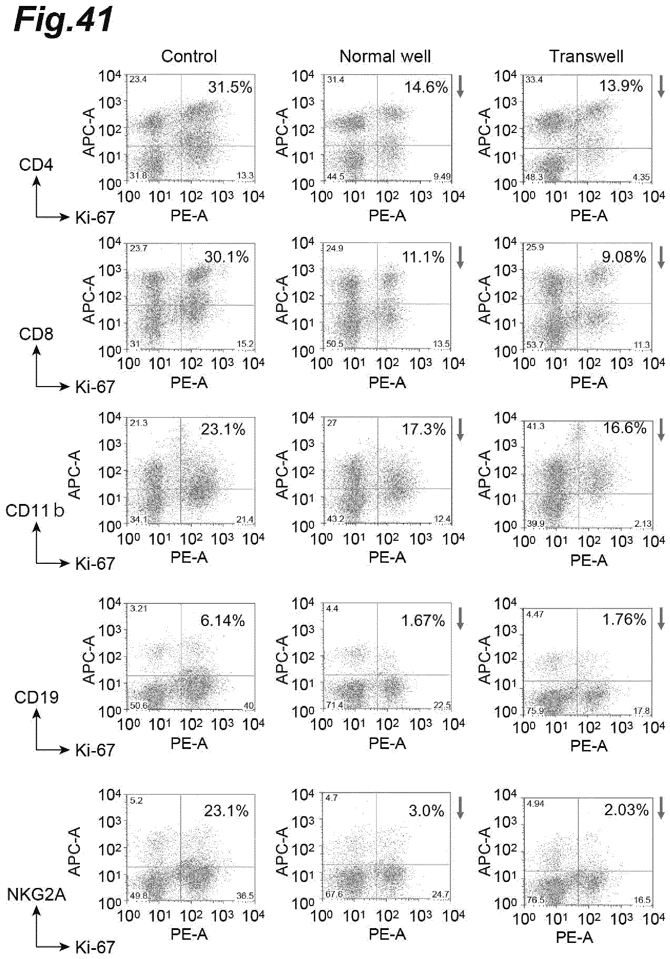

The immunosuppression pattern of the neural retina (hESC-NR) derived from human ES cells was similar also in separated coculture using Transwell (R). From this, it was indicated that a secreted humoral molecule(s) contributes to the immunosuppression by the neural retina derived from human ES/iPS cells (Figure 41).

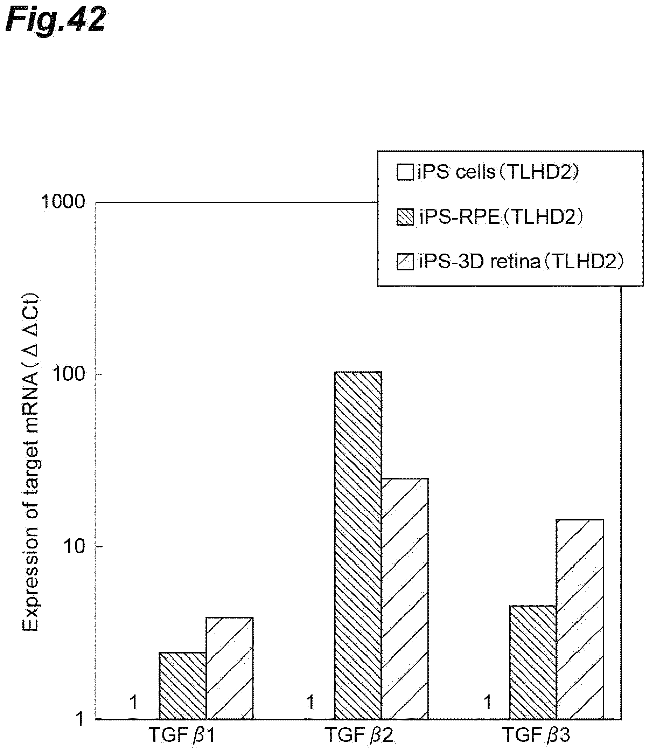

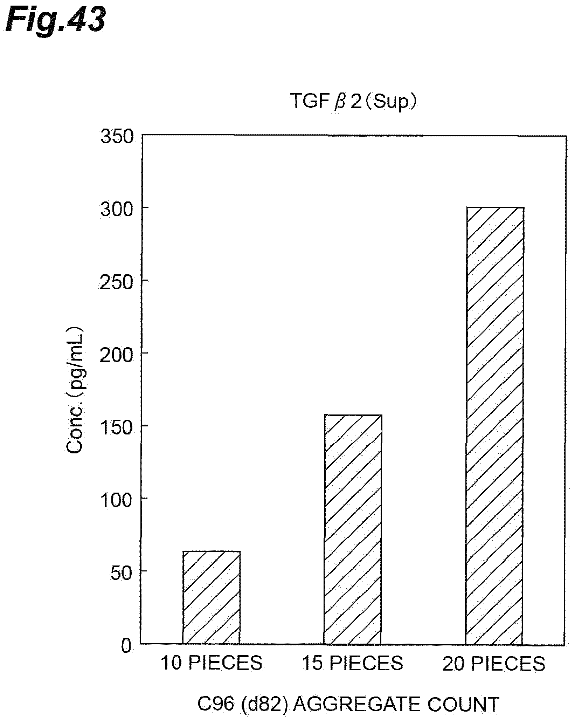

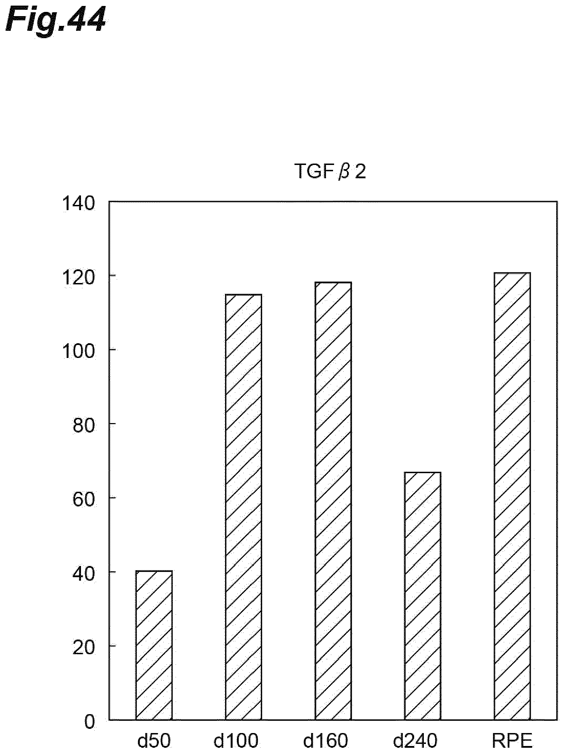

In comparison with hiPS cells (hiPSCs), the neural retina (hiPSC-NR) derived from human iPS cells prepared in Example 1 highly expressed TGF-β1, TGF-β2, TGF-β3 (Figure 42). TGF-β1, TGF-β2, TGF-β3 are also expressed by retinal pigment epithelial cells (hiPSC-RPE cells) derived from human iPS cells. It was shown by ELISA that TGF-β2 in a medium supernatant increases in an hiPSC-NR dose-dependent manner (Figure 43). In different differentiation stages of hESC-NR on differentiation day (DD) 50 to differentiation day (DD) 240, TGF-β2 was always secreted. The secretion of TGFβ2 exhibited the highest values on DDI00 and DD160. The secretion levels of TGFβ2 on DD100 and DD160 were similar to the level of TGFβ2 secreted by hiPSC-RPE cells (Figure 44).

Retinal pigment epithelial cells (hiPSC-RPE cells) derived from human iPS cells were prepared by the following method. hiPSCs were cultured in a GMEM medium containing 10 uM Y-27632 (Wako), 5 µM SB431542 (Sigma), and 3 uM CKI-7 (Sigma) to induce RPE cells. When RPE cell-like colonies appeared, the RPE cell-like colonies were scraped off and transferred to a dish coated with CELL start (Thermo) and the RPE cells were cultured in a DMEM/F12 (Thermo) medium containing B27 (Thermo) and bFGF (Wako) and SB431542 (SIGMA).

### <Example 7: Evaluation of ability to suppress activation of immune cells (TGFβ antibody, receptor inhibitor)>

The neural retina derived from human ES/iPS cells on Day 80 to Day 100 after starting the floating culture prepared in Example 1 were provided. The blood was collected from healthy subjects from which informed consent was obtained and mixed culture of the collected PBMCs and the neural retina was conducted for 2 days using a 24-well plate (manufactured by Sumitomo Bakelite Co., Ltd.) under the following four conditions: (1) the PBMCs only; (2) mixture of PBMCs and the neural retina; (3) addition of a mouse IgG antibody (isotypic control), in addition to the PBMCs and the neural retina, into the culture liquid; (4) addition of an anti-TGF β antibody (manufactured by R & D Systems, Inc.), in addition to the PBMCs and the neural retina, into the culture liquid; (5) addition of the TGFβ receptor inhibitor SB431542, in addition to the PBMCs and the neural retina, into the culture liquid.

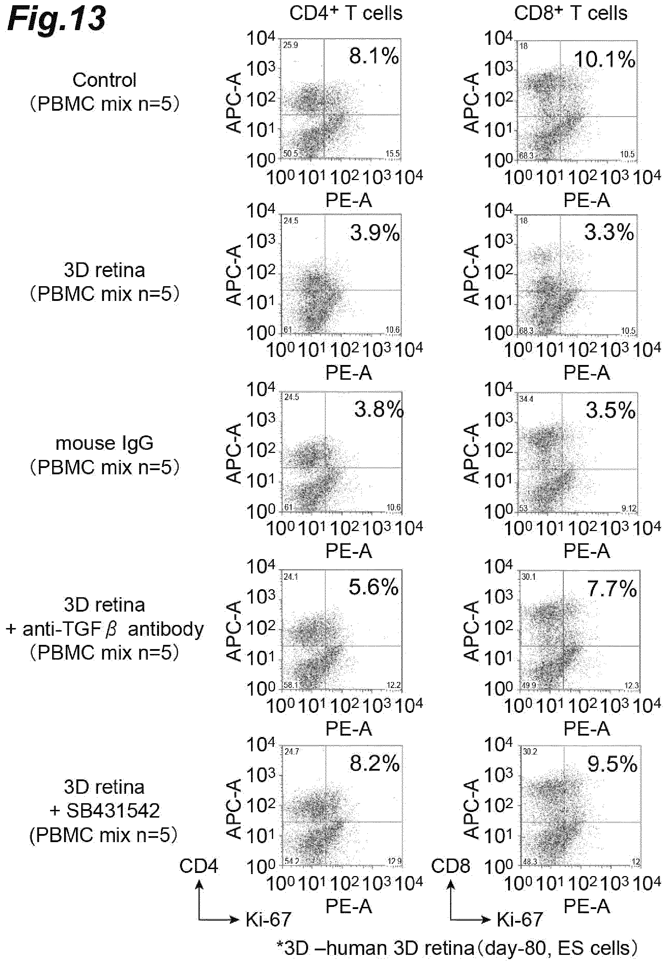

2 days later, PBMCs were collected, washed with PBS, and then immunostained using an anti-human CD4 antibody (product made by BioLegend, catalog #317416), an anti-CD8 antibody (manufactured by eBioscience, #17-0088). Moreover, as isotype antibodies, mouse IgG1 (BioLegend, #400122), κ isotype control phycoerythrin (PE) (BioLegend, #400112) were used. These immunostained cells were measures using a flow cytometry (FACSCanto flow cytometer, manufactured by BD Biosciences) and analyzed using FlowJo software. As a result, it was found in comparison with the control that the suppression of activation of CD4-positive T cells and CD8-positive T cells was canceled under the conditions of (4) and (5) in which a TGFβ antibody or a TGFβ receptor inhibitor was added into the culture liquid (Figures 13-14).

From these results, it was found that the neural retina derived from human ES/iPS cells suppress the activation of immune cells mainly via the TGFβ signal.

### <Example 8: TGFβ expression 1>

After washing the neural retina differentiated from human iPS cells (iPS-3D retina) on Day 80 to Day 100 after starting the floating culture prepared in Example 1 with PBS, a neural cell dispersion liquid (manufactured by Sumitomo Bakelite Co., Ltd.) was added and the neural retina were dispersed into single cells after incubation at 37°C. High Pure RNA Isolation Kit (manufactured by Roche) was used for these cells to collect RNA samples. Subsequently, cDNA was synthesized in LightCycler using Transcriptor First Strand cDNA Synthesis Kit (Roche: 04 897 030 001). qPCR of TGFβ1, TGFβ2, TGF β3 was conducted using Light Cycler 480 Probes Master (Roche: 04 707 494 001) kit. The measurement was conducted using Light Cycler.

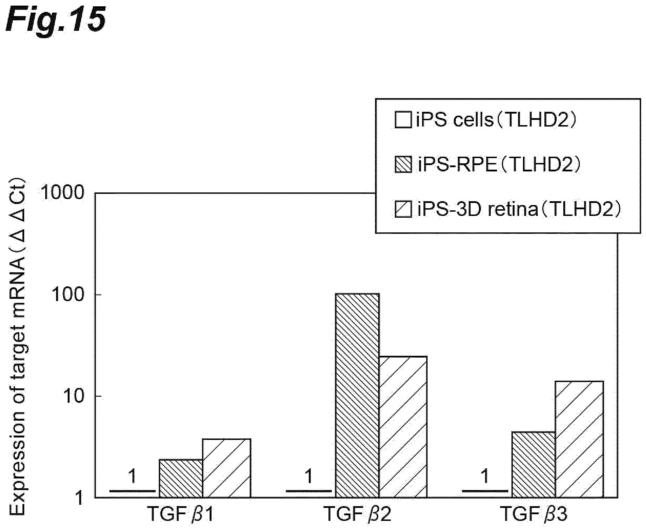

As a result, it was found that, in comparison with iPS cells, the TGFβ1 expression was increased 2-fold to 4-fold or so, the TGFβ2 expression was increased 100-fold or so, and the TGF β3 expression was increased 5-fold to 8-fold or so (Figure 15).

### <Example 9: TGFβ expression 2>

After washing the neural retina derived from human ES cells on Day 80 to Day 100 after starting the floating culture prepared in Example 1 with PBS, they were fixed at 4°C using 4% PFA for 15 minutes. After washing with PBS, they were soaked in a 30% sucrose solution. Subsequently, they were embedded in Cryomold using OCT Compound and then 12 µm sections were made with a cryostat. These sections were immunostained using an anti-N-cadherin antibody (BD), an anti-Ezrin antibody (R & D Systems, Inc.), an anti-TGFβ1 antibody (R & D Systems, Inc.), a TGFβ2 antibody (R & D Systems, Inc.), an anti-TGFβ3 antibody (R & D Systems, Inc.). These immunostained cells were observed with a confocal microscope (Leica SP8). As a result, it was found that all TGFβ1, TGFβ2, TGFβ3 were located in the apical surface markers N-cadherin and Ezrin-positive side.

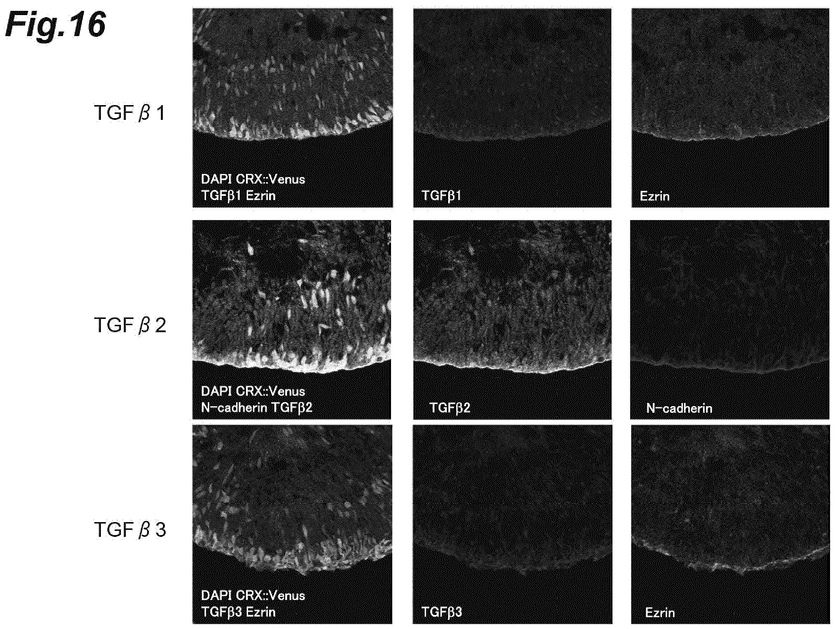

Therefore, it was suggested that TGFβ expressed by the neural retina was located in the apical surface side and secreted (Figure 16).

### <Example 10: Measurement of TGFβ2 secretion level by ELISA>

After washing the neural retina derived from human ES cells on Day 50, Day 100, Day 160, Day 240 after starting the floating culture prepared in Example 1 with PBS, 15 pieces of neural retina were transferred into a 24-well plate (Sumitomo Bakelite) and cultured in 1 ml of DMEM/F12 (ThermoFisher) for 1-2 days.

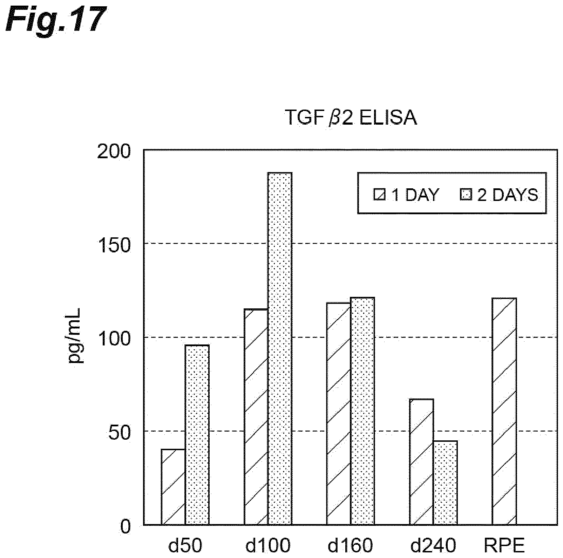

1, 2 days later, the culture supernatant was collected and measured for the amount of TGFβ2 secreted using a TGFβ2 ELISA kit (R & D Systems, Inc.). As a result, it was revealed that TGFβ2 is secreted in all differentiation stages and that a very large amount of TGFβ2 is secreted in 2 Day culture for Day 100 (Figure 17).

### <Example 11: Relationship between TGFβ2 expression and ELISA with or without enzymatic treatment>

The neural retina derived from human ES cells on Day 80 to Day 100 after starting the floating culture prepared in Example 1 were provided (1) with no treatment; (2) with washing with PBS, then treating with a neural cell dispersion liquid (Sumitomo Bakelite), and recovering without pipetting; (3) as samples in the semi dissociated state (Semi dissociate) obtained by washing the neural retina of (1) with PBS, then adding a neural cell dispersion liquid (manufactured by Sumitomo Bakelite Co., Ltd.) to the neural retina, incubating at 37°C, and then gently pipetting; (4) as samples obtained by completely dissociating the neural retina of (1) into single cells. Using a 24-well plate (manufactured by Sumitomo Bakelite Co., Ltd.), they were cultured in 1 ml of DMEM/F12 (ThermoFisher) for 2 days.

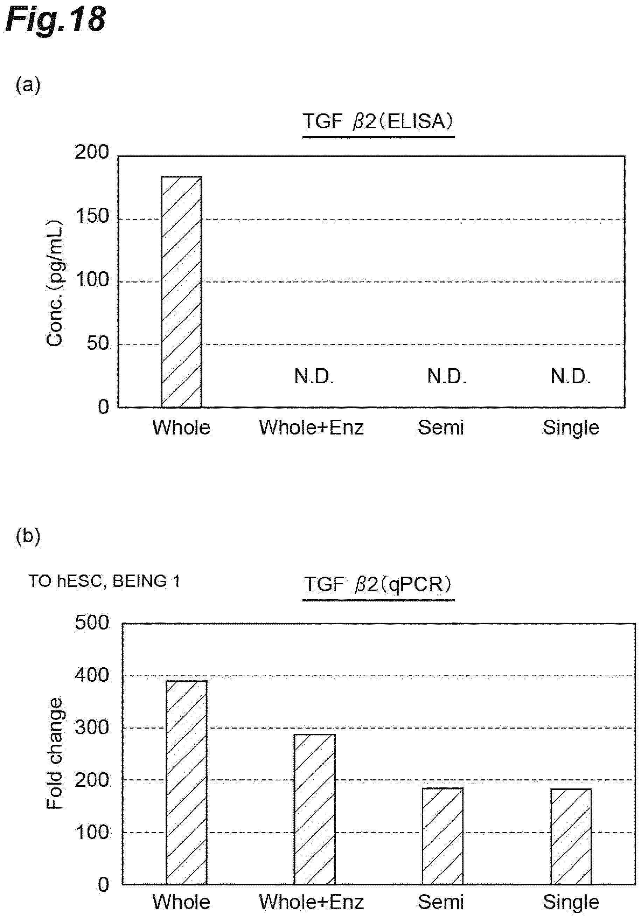

2 days later, the culture supernatant was collected and measured for the amount of TGFβ2 secreted using a TGFβ2 ELISA kit (R & D Systems, Inc.). The result of the measurement is shown in Figure 18 (a).

Moreover, High Pure RNA Isolation Kit (manufactured by Roche) was used for the cells of (1), (2), (3), and (4) to collect RNA samples. Subsequently, cDNA was synthesized in LightCycler using Transcriptor First Strand cDNA Synthesis Kit (Roche: 04 897 030 001). qPCR of TGFβ2 was conducted using Light Cycler 480 Probes Master (Roche: 04 707 494 001) kit. The measurement was conducted using Light Cycler. The result of the measurement is shown in Figure 18 (b).

Furthermore, after washing with PBS the neural retina derived from human ES cells just after the enzymatic treatments of (1) and (2), they were fixed at 4°C using 4% PFA for 15 minutes. After washing with PBS, they were soaked in a 30% sucrose solution. Subsequently, they were embedded in Cryomold using OCT Compound and then 12 µm sections were made with a cryostat. These sections were immunostained using an anti-N-cadherin antibody (BD), a TGFβ2 antibody (R & D Systems, Inc.). These immunostained cells were observed with a confocal microscope (Leica SP8).

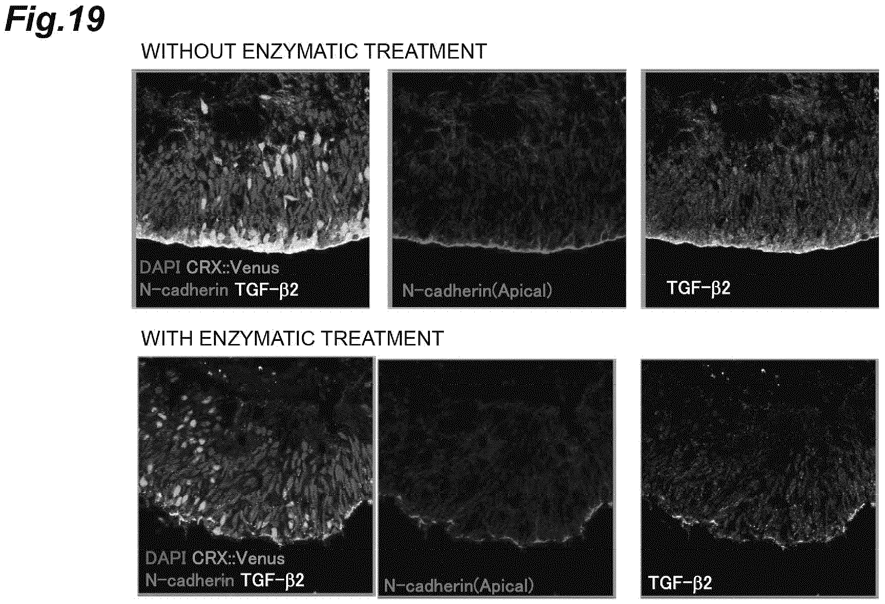

As a result, while secretion of TGFβ2 was detected by ELISA in the sample of (1) with no enzymatic treatment, it was under the detection limit in any of (2), (3), and (4). On the other hand, it was found, in the expression determined by qPCR, that while the amount expressed in (1) is the highest, it is expressed also in (2), (3), and (4). Moreover, it was found, as a result of observation with a confocal microscope, that while TGFβ2 and the apical surface marker N-cadherin are arranged and localized on the surface with no enzymatic treatment, the surface is disturbed in the sample of (2) and the localization of TGFβ2 is also sparse with the enzymatic treatment (Figure 19).

Therefore, it was suggested that the structure (for example, the apical surface is arranged in line) of the Apical surface, which is a membrane surface, is important for neural retina to secrete TGFβ2. Meanwhile, it was found that the amount secreted is reduced by disturbance of the structure such as matrices of the membrane surface by an enzymatic treatment or the like.

### <Example 12: Differentiation day and MLR>

The neural retina derived from human ES cells on Day 50, Day 80, Day 150, Day 200 after starting the floating culture prepared in Example 1 were provided. The blood was collected from healthy subjects from which informed consent was obtained and mixed culture of the PBMCs and the neural retina was conducted using a 24-well plate (manufactured by Sumitomo Bakelite Co., Ltd.) for the collected PBMCs and the neural retina.

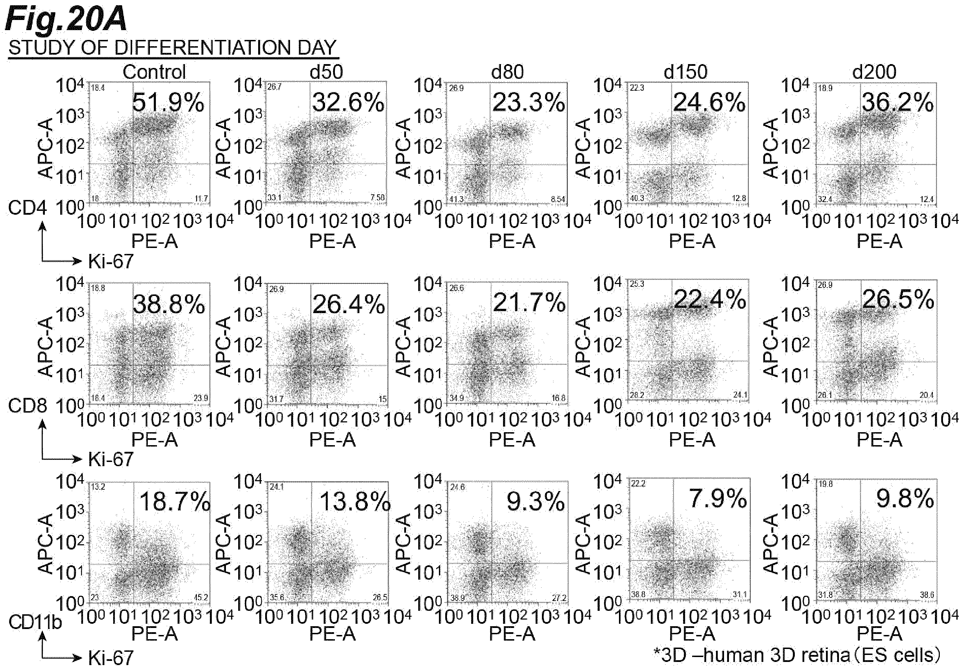

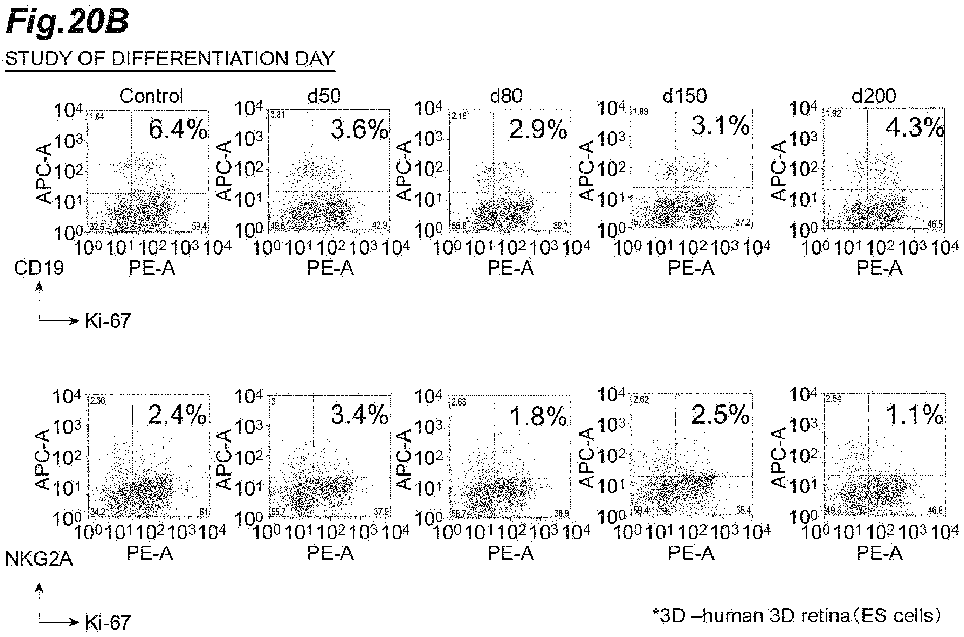

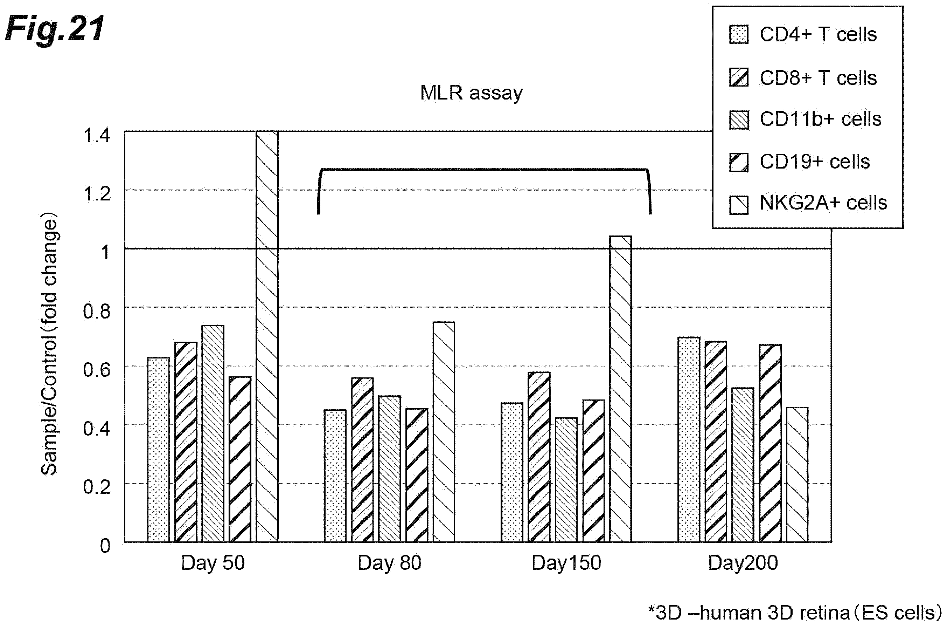

2 days later, PBMCs were collected, washed with PBS, and then immunostained using an anti-human CD4 antibody (manufactured by BioLegend, #317416), an anti-human CD8 antibody (manufactured by eBioscience, #17-0088), an anti-CD19 antibody, an anti-NKG2A antibody. As isotype antibodies, mouse IgG1 (BioLegend, #400122), κ isotype control phycoerythrin (PE) (BioLegend, #400112) were used. These immunostained cells were measured using a flow cytometry (FACSCanto flow cytometer, manufactured by BD Biosciences) and analyzed using FlowJo software. As a result, it was found that the activation of immune cells was suppressed in all differentiation stages (Figure 20A, Figure 20B, Figure 21).

### <Example 13>

In order to see the difference between an autologous origin and an allogeneic origin in the aforementioned MLR assay, the blood was collected from the donor of the iPS cells (TLHD2) and healthy subjects from which informed consent was obtained and mixed culture was conducted for 2 days using a low adhesive 24-well plate (manufactured by Sumitomo Bakelite Co., Ltd.) for the collected PBMCs and neural retina turned into single cells with a neural cell dispersion liquid (manufactured by Sumitomo Bakelite Co., Ltd.).

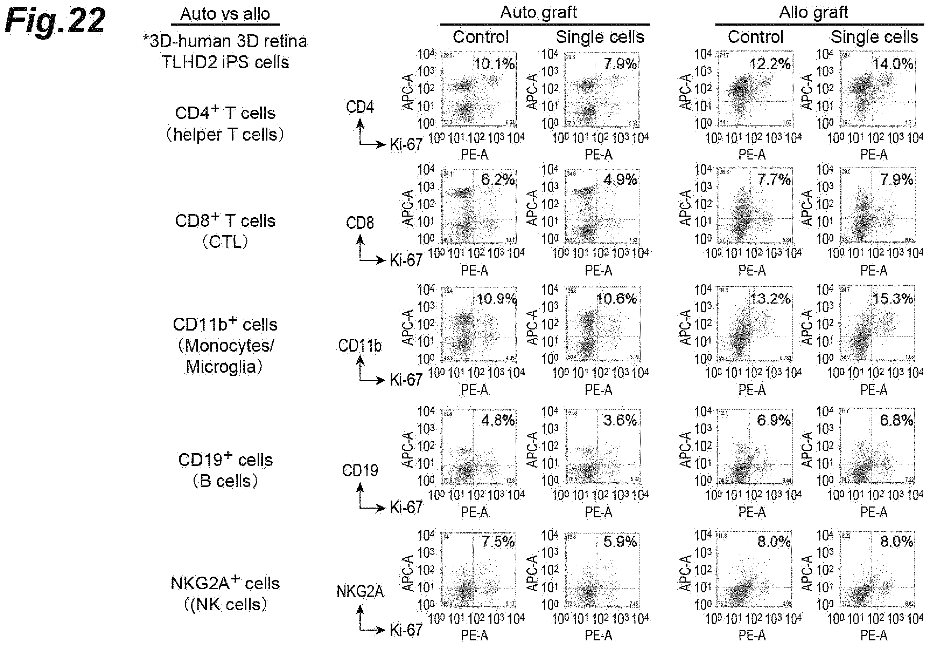

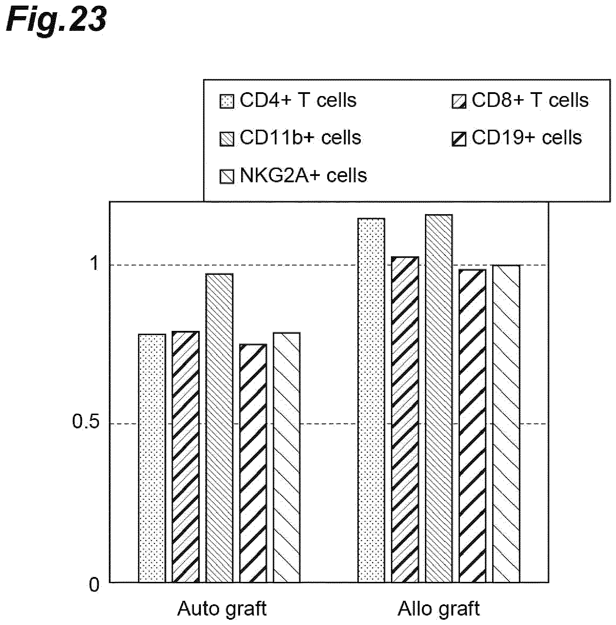

2 days later, PBMCs were collected, washed with PBS, and then immunostained using an anti-APC-human CD4 antibody (manufactured by BioLegend, #317416), an anti-human APC-CD8 antibody (manufactured by eBioscience, #17-0088), an anti-APC-human CD11b antibody (manufactured by Miltenyi Biotec, #130-091-241), an anti-CD 19 antibody, an anti-APC-NKG2A antibody (manufactured by Miltenyi Biotec, #130-098-809), an anti-human PE-Ki-67 antibody (BioLegend, #350504). Moreover, as isotypic antibodies, mouse immunoglobulin 2a (IgG2a), κ isotype control fluorescein isothiocyanate (FITC) (BioLegend, #400208), mouse IgG1, κ isotype control APC (BioLegend, #400122), and mouse IgG1 (BioLegend, #400122), κ isotype control phycoerythrin (PE) (BioLegend, #400112) were used. These immunostained cells were measured using a flow cytometry (FACSCanto flow cytometer, manufactured by BD Biosciences) and analyzed using FlowJo software. Figure 22 and Figure 23 show results of analysis when the neural retina differentiated from human iPS cells were used.

As a result, the immunostimulation was not confirmed under the conditions in which autologous PBMCs and neural retina were mixed. Moreover, for allogeneic Allo, while very weak rejection of CD4-positive T cells, CD8-positive T cells, CD11b-positive monocytes, and microglia was confirmed, no large difference was confirmed.

From these results, it was found that neural retina derived from human iPS cells are more unlikely to undergo immune rejection if they are of an autologous origin, but do not cause rejection greatly, even if they are of an allogeneic origin.

### <Example 14: Preparation of neural retina (NR) from monkey iPS cells by SFEBq method>

Monkey iPS cells (1121A1 strain) established at Kyoto University were cultured under on-feeder conditions using MEF (Millipore, PMEF-CFX-C). As a non-differentiating medium, a medium was obtained by mixing the Primate ES medium (Reprocell) and the mTeSR1 (STEMCELL TECHNOLOGIES) medium at 3:1 and adding 10 mg/ml bFGF (manufactured by Wako) and 10⁶ units/ml LIF (manufactured by Millipore) respectively as 1000-fold dilutions and used.

Specific maintaining culture operations of monkey iPS cells were conducted as follows. First, subconfluent (about 60% of the culture area were covered with cells) monkey iPS cells were physically detached using a 1 ml tip and the monkey iPS cells collected in the state of colonies were seeded in a plastic culture dish in which MEF had been seeded, and cultured in the presence of bFGF and LIF in a mixed medium of Primate ES and mTeSR1 under on-feeder conditions. As the aforementioned plastic culture dish, a 60 mm dish or a 100 mm dish (manufactured by Iwaki Co., Ltd., for cell culture) was used. The medium was changed every day after seeding and the cells were subsequently cultured until subconfluent.

After washing the monkey iPS cells with PBS, cells were subjected to a detachment treatment using CTK (Reprocell) and collected in a 15 ml of tube in the state of colonies. The tube was left to stand and, after confirming that the colonies have fallen to the bottom, the supernatant was removed with an aspirator. Subsequently, the colonies were washed with PBS, Tryple Select was then added thereto, and the mixture was incubated at 37°C for about 5 minutes. After dispersing the colonies into single cells by pipetting manipulation, the monkey iPS cells dispersed into single cells were floated in 100 µL of a serum-free medium at 1.2 × 10⁴ cells per well of a 96-well non-cell-adhesive culture plate (trade name: PrimeSurface 96-well V bottom plate, manufactured by Sumitomo Bakelite Co., Ltd.) and cultured in the floating state at 37°C, 5% CO₂. As the serum-free medium (gfCDM+KSR) in the culture, a serum-free medium obtained by adding 10% KSR, 450 µM 1-monothioglycerol, 1× Chemically defined lipid concentrate to a 1:1 mixture of the F-12 medium and the IMDM medium was used.

Y27632 (a ROCK inhibitor, final concentration 10-20 µM) and SAG (an Shh signaling pathway agent, 300 nM or 30nM) were added to the serum-free medium. On Day 3 after starting the floating culture, 50 µL of a medium containing exogenous human recombination BMP4 in a final concentration of 1.5 nM was added using a medium containing no Y27632 nor SAG and containing human recombinant BMP4 (trade name: Recombinant Human BMP-4, manufactured by R & D). On Day 6 or more after starting the floating culture, a half amount of the medium was changed once in 3 days with the medium containing none of Y27632 and SAG and human recombinant BMP4.

Aggregates on Day 15 after the starting the floating culture were transferred into a 90 mm low adhesive culture dish (manufactured by Sumitomo Bakelite Co., Ltd.) and cultured in a serum-free medium (a DMEM/F12 medium supplemented with 1% N₂ Supplement) containing a substance acting on a Wnt signaling pathway (CHIR99021, 3 µM) and an FGF signaling pathway inhibitor (SU5402, 5 µM) for 3-4 days at 37°C, 5% CO₂. Subsequently, they were cultured in a serum medium (NucT0 medium) containing no substance acting on a Wnt signaling pathway and no FGF signaling pathway inhibitor in a 90 mm low adhesive culture dish (manufactured by Sumitomo Bakelite Co., Ltd.). On Day 40 or more after starting the floating culture (induction of differentiation), they were cultured in a serum medium containing no substance acting on a Wnt signaling pathway and no FGF signaling pathway inhibitor (NucT1 medium). On Day 60 or more after starting the floating culture (induction of differentiation), they were cultured in the NucT2 medium for a long term.

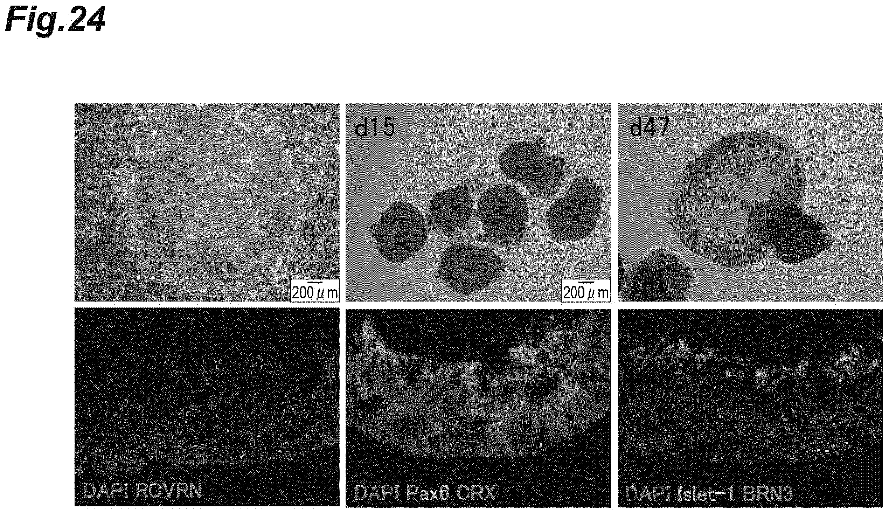

As a result of observation using a microscope, it was found that neural retina (NR) having a layered structure are formed in the monkey iPS cell line (Figure 24).

After washing the neural retina derived from monkey iPS cells with PBS, they were fixed at 4°C using 4% PFA for 15 minutes. After washing with PBS, they were soaked in a 30% sucrose solution. Subsequently, they were embedded in Cryomold using OCT Compound and then 12 µm sections were made with a cryostat. These sections were immunostained using an anti-Recoverin antibody (Millipore), a Pax6 antibody (BD pharmingen), a CRX antibody (Takara Bio Inc.), an Islet-1 (DSHB), BRN3 (Santa cruz). These immunostained cells were observed with a fluorescence microscope (Keyence Corporation). As a result, it was confirmed that the neural retina derived from monkey iPS cells had been differentiated into the retina, like those of human (Figure 24).

### <Example 15>

To a culture liquid of the neural retina derived from monkey iPS cells on Day 45 to Day 55 after starting the floating culture prepared in Example 14 was added recombinant IFN-γ (100 ng/mL) (manufactured by R & D Systems, Inc.) and the cultures were cultured for 2 days. Meanwhile, a group to which no recombinant IFN-γ was added was also prepared.

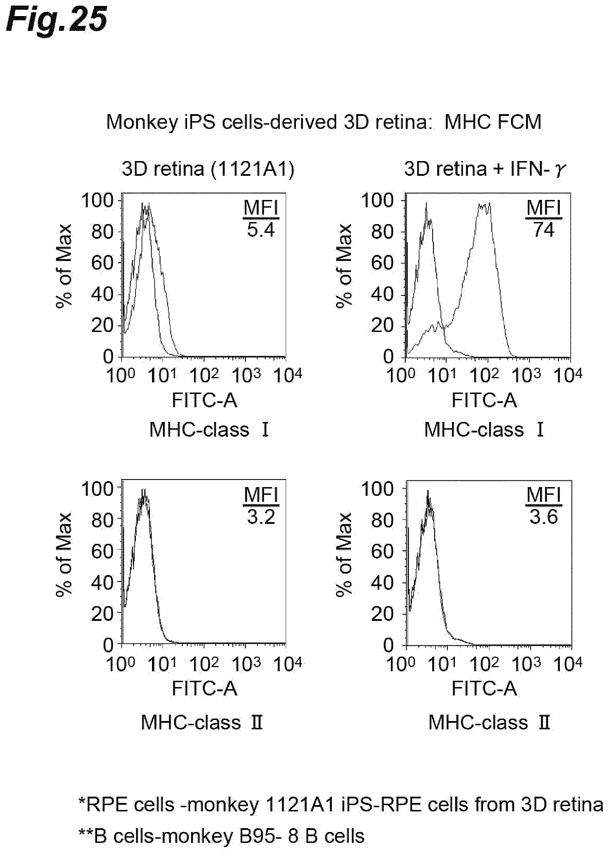

After washing the neural retina derived from monkey iPS cells with PBS 2 days later, a neural cell dispersion liquid (manufactured by Sumitomo Bakelite Co., Ltd.) was added and the neural retina were dispersed into single cells after incubation at 37°C. These cells were immunostained using an anti-HLA class I antibody (FITC anti-human HLA-A, -B, -C, Sigma-Aldrich, #F 5662), an anti-HLA class II antibody (FITC anti-human HLA-DR, -DP, -DQ, DakoCytomation, #F0817 or BD PharMingen, #555558), and an isotypic antibody (mouse IgG2a, κ isotype control, FITC, BD PharMingen, #555573 or mouse IgG2b, κ isotype control, FITC, eBioscience, #11-4732) as control. These immunostained cells were measures using a flow cytometry (FACSCanto flow cytometer, manufactured by BD Biosciences) and analyzed using FlowJo software. As a result, it was found that under the conditions in which no recombinant interferon γ is added, the neural retina differentiated from monkey iPS cells have little expression of MHC-class I (HLA-class I) and no expression of MHC-class II (HLA-class II). On the other hand, it was found that under the conditions in which recombinant interferon γ is added, there is expression of MHC-class I (HLA-class I), but it is low, and there is no expression of MHC-class II (HLA-class II) (Figure 25).

From these results, it was found that neural retina differentiated from monkey iPS cells have very low immunogenicity.

### <Example 16: MHC-matched, non-matched monkey-monkey graft>

The neural retina derived from monkey iPS cells on Day 45 to Day 55 after starting the floating culture prepared in Example 14 were grafted into cynomolgus monkeys having an MHC type matched with the monkey iPS cells and cynomolgus monkeys having a mismatched MHC type in which photoreceptor cell denaturation model was produced by laser. After graft, no immunosuppressive agent was administered.

6 months later, the eyes in which the neural retina derived from monkey iPS cells were grafted were washed with PBS, and then they were fixed at 4°C using 4% PFA for 60 minutes. After washing with PBS, they were soaked in a 30% sucrose solution. Subsequently, they were embedded in Cryomold using OCT Compound and then 12 µm sections were made with a cryostat. These sections were immunostained using an anti-Rhodopsin antibody (Sigma), an anti-Recoverin antibody (Millipore), an anti-PKCa antibody (R & D Systems, Inc.), a Cone arrestin antibody (R & D Systems, Inc.). These immunostained tissues were observed with a confocal microscope (Leica SP8). As a result, it was confirmed that in both the monkeys having a matched MHC and the monkeys having a mismatched MHC, rod photoreceptor cells and cone photoreceptor cells had survived and Rhodopsin-positive rod photoreceptor cells had matured.

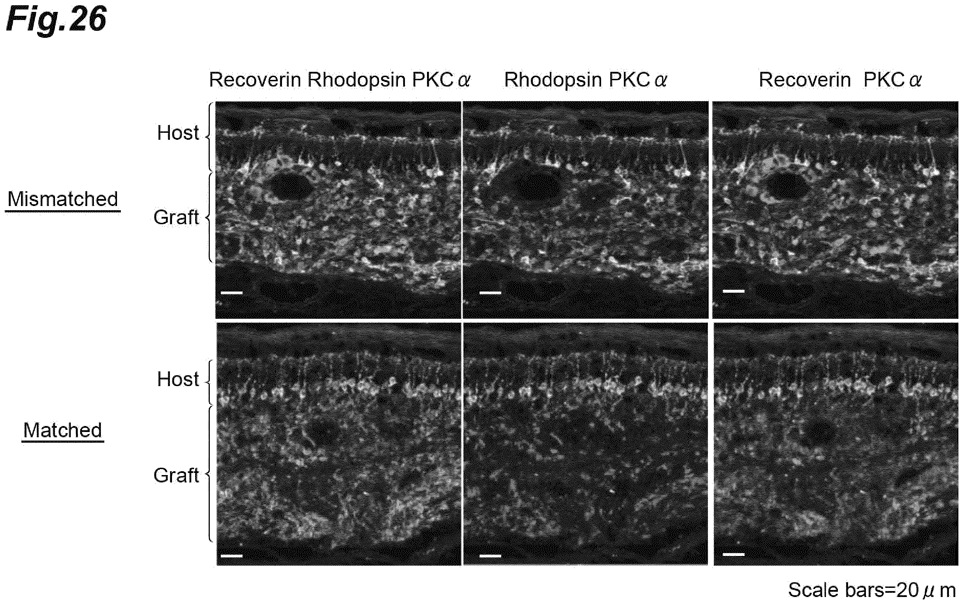

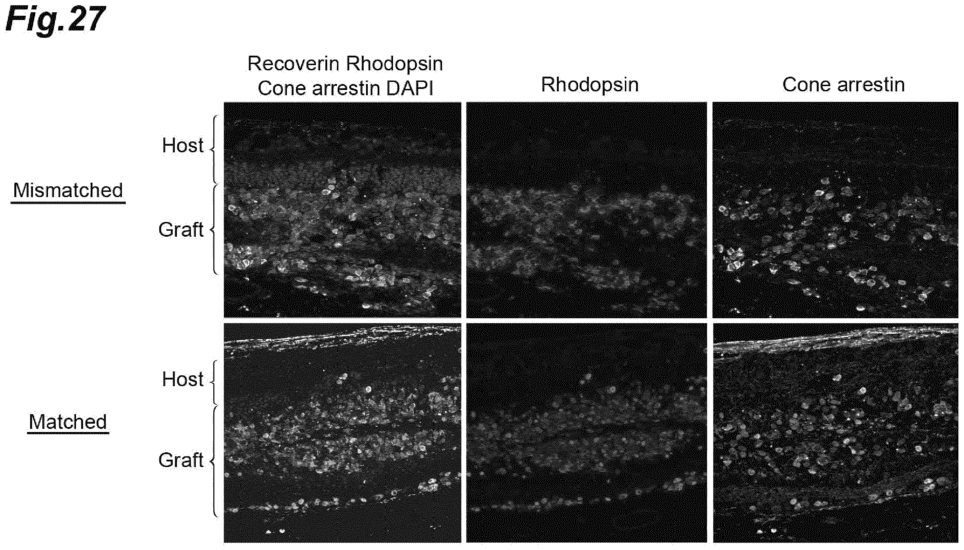

Accordingly, it was revealed that in both the monkeys having a matched MHC and the monkeys having a mismatched MHC, photoreceptor cells survive and partially mature without administration of an immunosuppressive agent (Figure 26, Figure 27).

### <Example 17: Evaluation of ability of neural retina in living monkey to suppress activated immune cells>

As samples for evaluating the ability to suppress monkey-derived immune cells activated, a neural retina (a sample of a neural retina not dispersed into single cells) derived from the living body of a monkey at 8 years and 6 months of age was provided by stimulating through addition of an anti-CD3 antibody and an anti-CD28 antibody. The blood was collected from an allogeneic monkey and mixed culture of the collected PBMCs (peripheral blood mononuclear cells) was conducted for 2 days using a 24-well plate (manufactured by Sumitomo Bakelite Co., Ltd.).

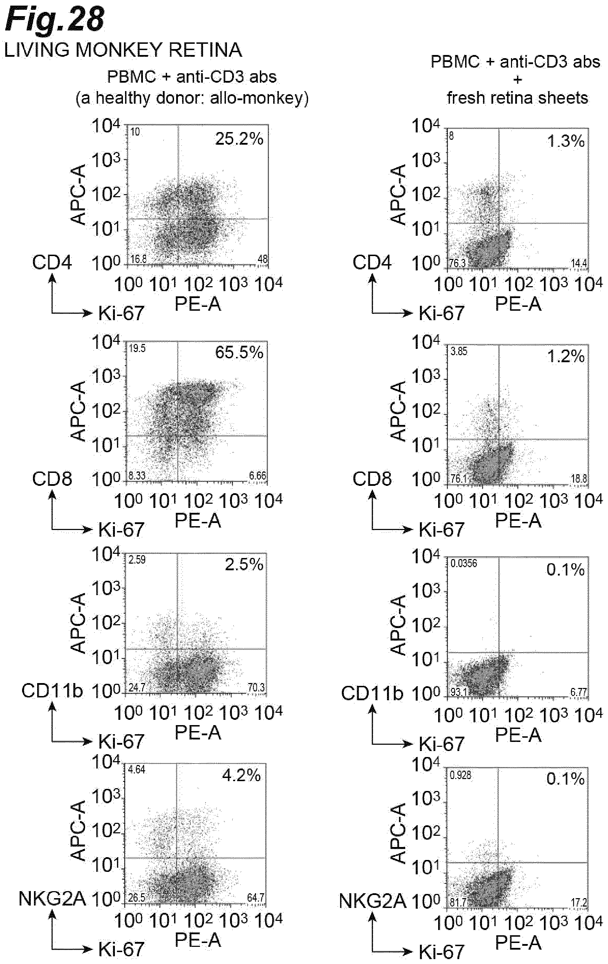

2 days later, PBMCs were collected, washed with PBS, and then immunostained using an anti-APC-CD4 antibody (manufactured by BioLegend, #317416), an anti-APC-CD8 antibody (manufactured by eBioscience, #17-0088), an anti-APC-CD11b antibody (manufactured by Miltenyi Biotec, #130-091-241), an anti-APC-CD19 antibody, an anti-NKG2A antibody, an anti-PE-Ki-67 antibody (BioLegend, #350504). Moreover, as isotype antibodies, mouse immunoglobulin 2a (IgG2a) κ isotype control fluorescein isothiocyanate (FITC) (BioLegend, #400208), mouse IgG1κ isotype control APC, and mouse IgG1 (BioLegend, #400122) κ isotype control phycoerythrin (PE) (BioLegend, #400112) were used. These immunostained cells were measured using a flow cytometry (FACSCanto flow cytometer, manufactured by BD Biosciences) and analyzed using FlowJo software. Figure 28 shows results of analysis when a neural retina derived from a living monkey was used.

As a result, it was found that monkey-derived neural retina suppress the activation state of activated CD4-positive T cells, CD8-positive T cells, CD11b-positive monocytes and microglia, and NKG2A-positive NK cells (Figure 28).

From these results, it was found that the neural retina derived from living monkeys suppress the activated state of immune cells also for immune cells in the activated state. Thus, it can be said that the neural retina derived from living monkeys have immunosuppressive capacity.

### <Example 18: About HLA class I, II expression in neural retina derived from human ES/iPSC by IFN-γ stimulation>

To the culture liquid of the neural retina derived from human ES/iPS cells on Day 80 to Day 100 after starting the floating culture prepared in Example 1 was added recombinant IFN-γ (100 ng/mL) (manufactured by R & D Systems, Inc.) and the neural retina were cultured for 2 days. Meanwhile, a group to which no recombinant IFN-γ was added was also prepared.

2 days later, the neural retina derived from human ES/iPS cells were washed with PBS and a neural cell dispersion liquid (manufactured by WAKO) was added. After incubation at 37°C, they were dispersed into single cells by pipetting. These cells were immunostained using an anti-HLA class I antibody (APC anti-human HLA-A, -B, -C, Bio Legend, #311410), an anti-HLA class II antibody (APC anti-human HLA-DR, -DP, -DQ, Bio Legend #361714), an anti-β2-microglobulin antibody (APC Bio Legend, #316312), and an isotypic antibody (mouse IgG2a, κ isotype control, APC; Bio Legend, #400220) as a control. These immunostained cells were measured using a flow cytometry (FACSCanto flow cytometer, manufactured by BD Biosciences) and analyzed using FlowJo software.

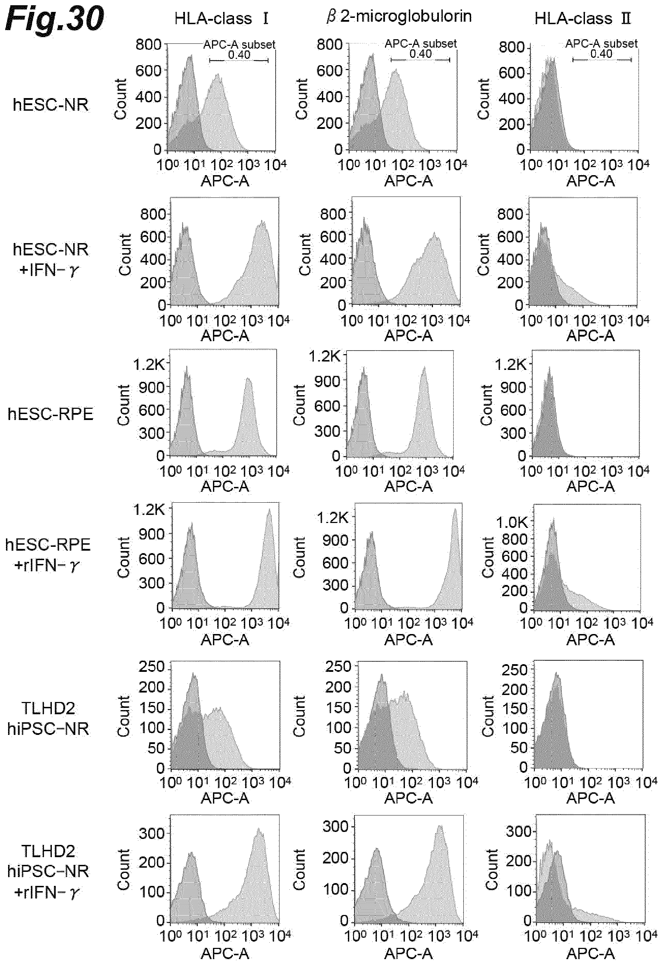

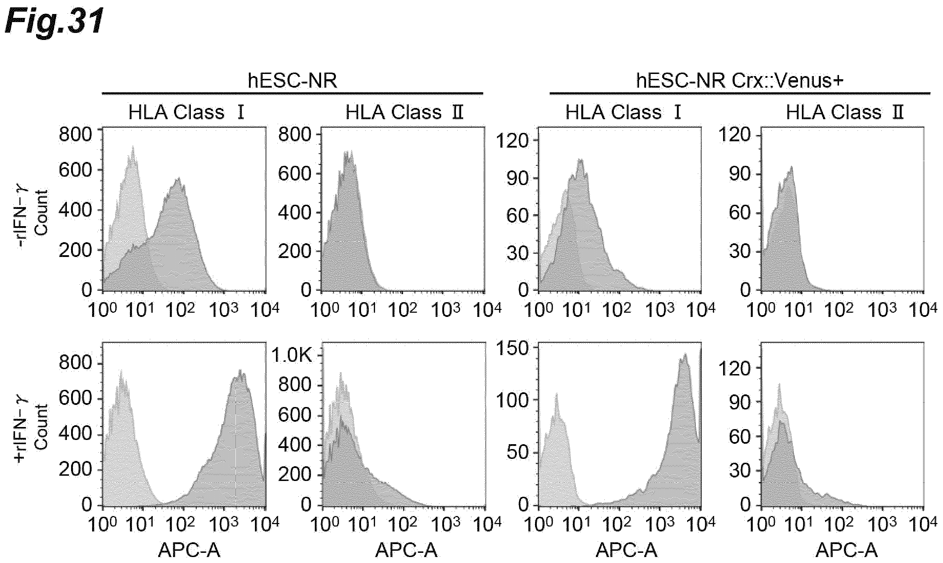

In order to evaluate the ability of neural retina (hES-NR/hiPSC-NR) derived from human ES/iPS cells to present an antigen, the expression of molecules involved in immunogenicity such as human leukocyte antigen (HLA) class I and II was examined by flow cytometry (Figure 30, Figure 31).

hES-NR/hiPSC-NR expressed HLA class I and β2-microglobulin (β2-MGat) at a low level in comparison with RPE cells, but no HLA class II. The inflammatory cytokine interferon-y (IFN-γ) stimulation increased the HLA class I expression.

The HLA class I expression level in hiPSC-NR was lower than that of hiPSC-RPE (Figure 30, Figure 31). Therefore, it was found that hESC-NR has lower immunogenicity than RPE cells.

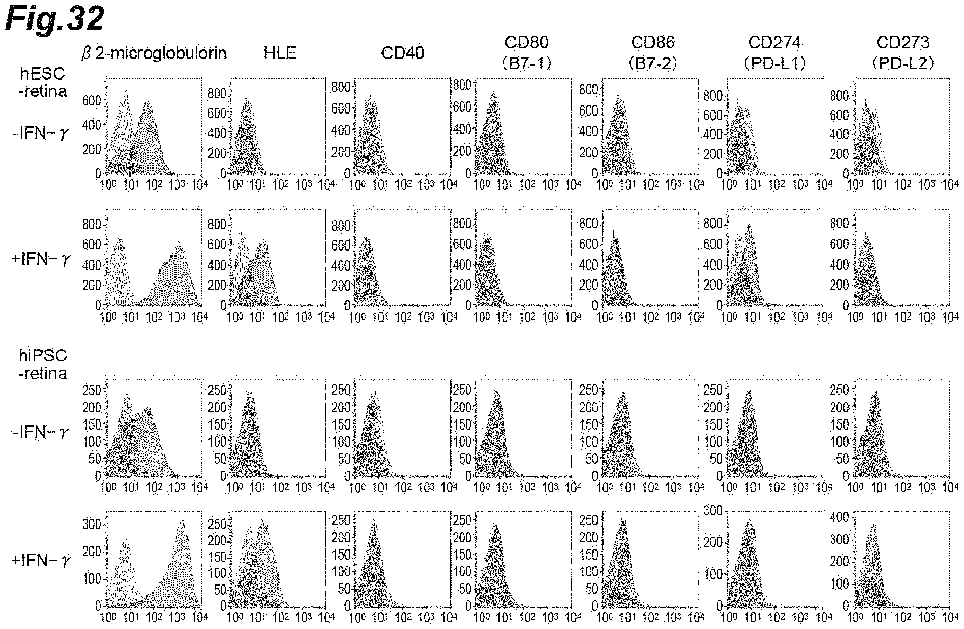

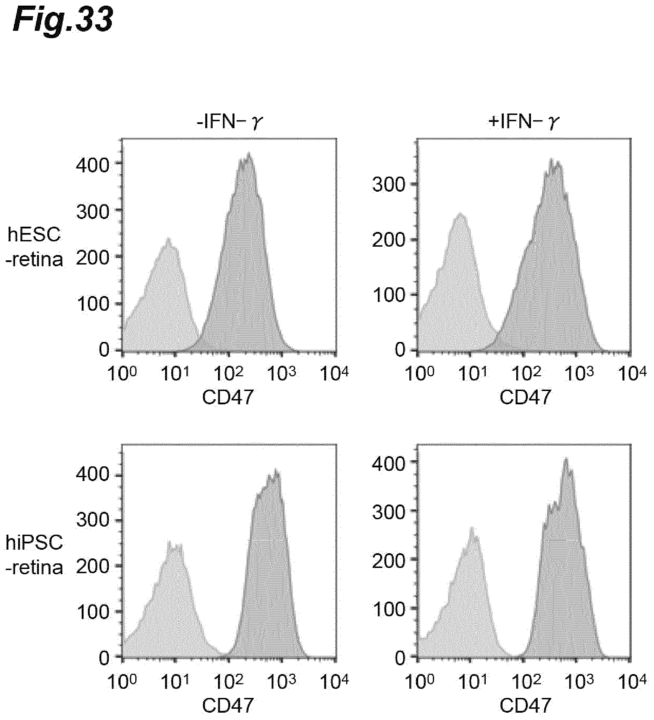

INF-γ also increased the expression of β2-microglobulin (β2-MG), HLA-E, which are proteins composing the MHC class I molecule, and PD-L1 in both hESC-NR and hiPSC-NR. On the other hand, no expression of the costimulatory factors CD40, 80, and 86 was confirmed with or without INF-γ. Furthermore, CD47, which is known as an immunosuppressive surface antigen, was expressed with or without INF-γ. The HLA class I expression level was lower in Crx::venus+ photoreceptor precursors than other populations (Figure 32, Figure 33).

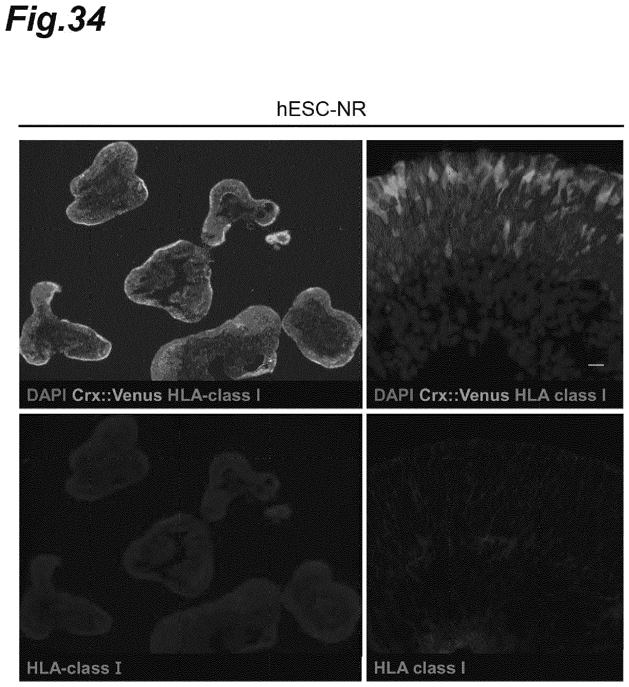

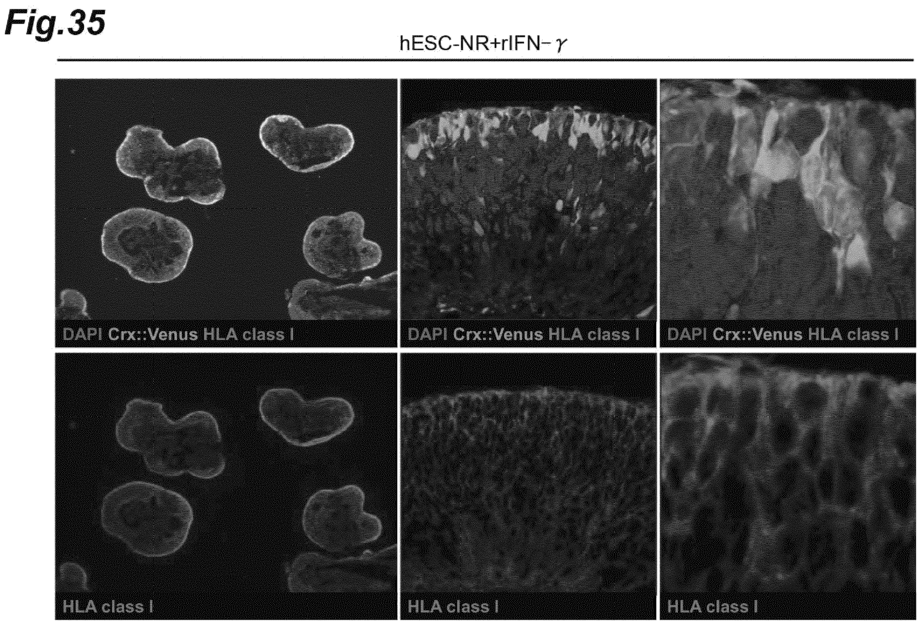

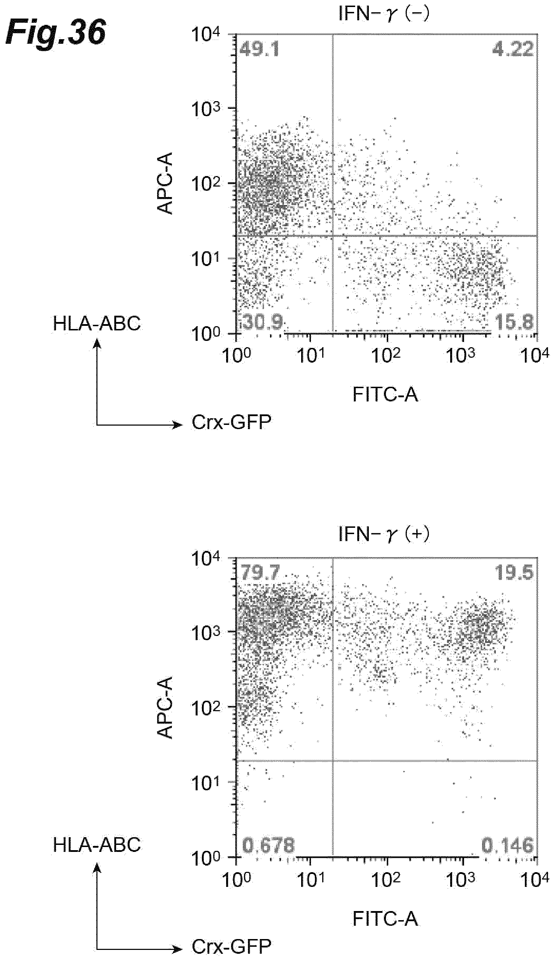

By the immunohistochemistry analysis, up-regulation of HLA class I in hESC-NR after the INF-γ stimulation was confirmed. On the other hand, it was hardly detected in Crx::Venus+ photoreceptor precursors with no INF-γ stimulation condition (Figures 34-36).

### <Example 19: HLA expression level and distribution in hESC-NR after graft>

Using a retinal degenerative Rho mutated nude rat model (lymphocyte-depleted nude rats), the HLA expression in neural retina (hES-NR) derived from human ES cells after graft was examined.

Rho mutated nude rats (SD-Foxn1 Tg (S334ter) 3LavRrrc nude rats, Japan SLC, Inc.) were prepared. As neural retina for graft preparation, the neural retina derived from human ES/iPS cells on Day 80 to Day 100 after starting the floating culture prepared in Example 1 were prepared.

A continuous epitheliums part of a neural retina for graft preparation was cut and prepared as a graft. Rats were anesthetized by ketamine hydrochloride (40-80 mg/kg) and xylazine (5-10 mg/kg) or inhalation of 3-5% isoflurane (Hung-ya et. al., 2018 EbioMedicine). The pupil was dilated with MydrinP (0.5% phenylephrine + 0.5% tropicamide, Santen Pharmaceutical Co., Ltd. (Osaka-shi)). The graft was inserted in the subretinal space in an SD-Foxn1Tg (from S334) 3LavRrc nude rat using a glass pipette.

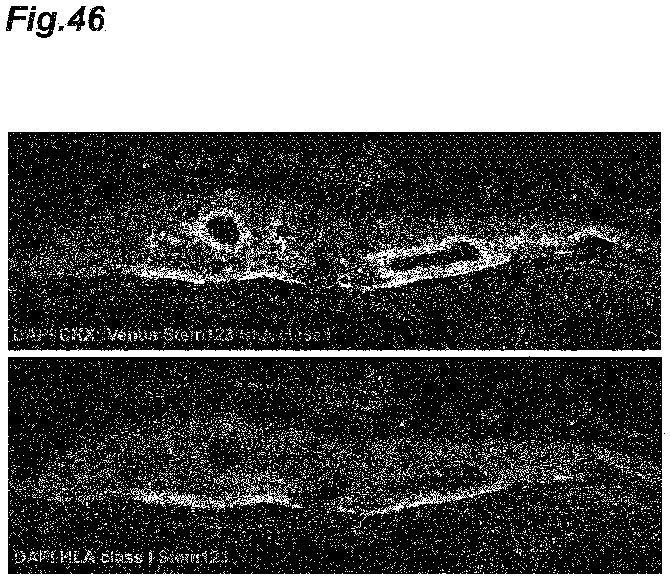

The HLA class I expression was not apparent on Day 1 after the insertion of graft. However, 5 months after the insertion of graft, cells expressing HLA class I were localized between the photoreceptor cell rosette and the host RPE. Furthermore, these cells expressing HLA class I were co-localized with human specific glial fibrillary acidic protein (GFAP) and graft-derived activated Muller glia exhibit HLA-Class I (Figure 46). Therefore, after graft, activated Muller glia expressed HLA-Class I with limitation and other photoreceptor cells did not express HLA-Class I.

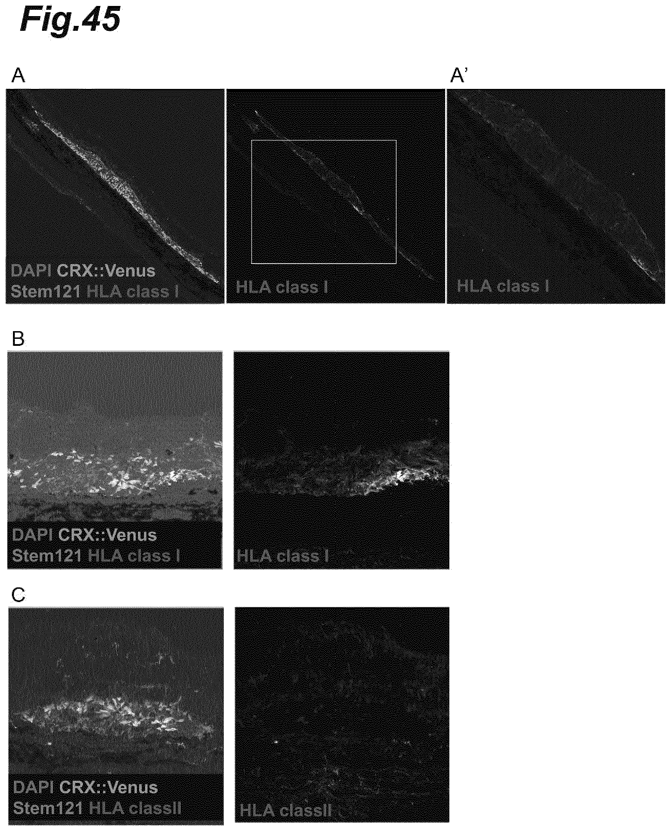

The expression of HLA class II was not confirmed in any part at any time point where and when examined (Figure 45C).

### <Example 20: About activation of immune cells by NR with increased HLA-Class I expression by INF-γ treatment>

To the culture liquid of the neural retina derived from human ES cells on Day 80 to Day 100 after starting the floating culture prepared in Example 1 was added recombinant IFN-γ (100 ng/mL) (manufactured by R & D Systems, Inc.) and the cultures were cultured for 2 days. Meanwhile, a group to which no recombinant IFN-γ was added was also prepared. The blood was collected from healthy subjects from which informed consent was obtained and mixed culture of the PBMCs and the neural retina was conducted using a 24-well plate (manufactured by Sumitomo Bakelite Co., Ltd.) for the collected PBMCs and the neural retina.

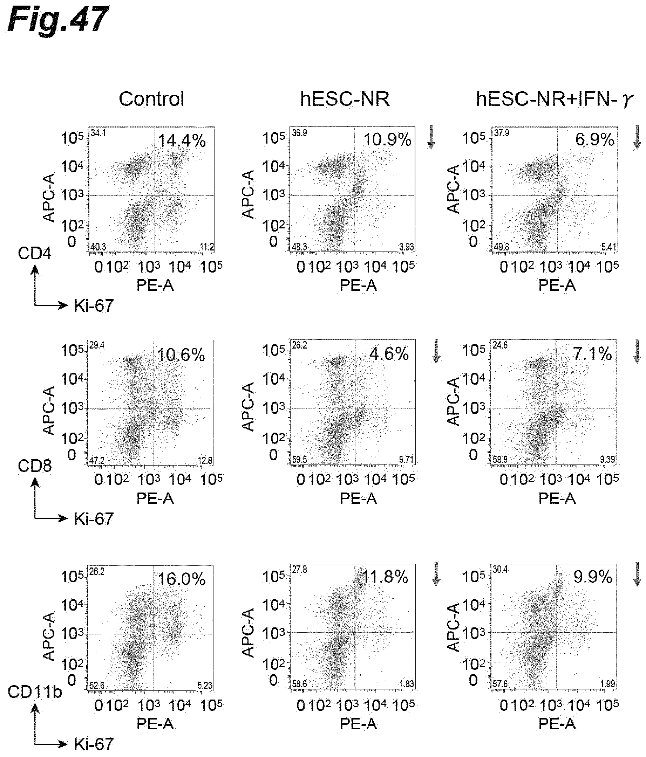

5 days later, the PBMCs were collected, washed with PBS, and then immunostained using an anti-human CD4 antibody (manufactured by BioLegend, #317416), an anti-CD8 antibody (manufactured by eBioscience, #17-0088), an anti-CD19 antibody, an anti-NKG2A antibody. Moreover, as isotypic antibodies, mouse IgG1 (BioLegend, #400122), κ isotype control phycoerythrin (PE) (BioLegend, #400112) were used. These immunostained cells were measured using a flow cytometry (FACSCanto flow cytometer, manufactured by BD Biosciences) and analyzed using FlowJo software. As a result, it was found that even hESC-NR treated with INF-γ leading to an increased HLA-Class I expression does not activate immune cells (Figure 47).

## Claims

1. A therapeutic drug for a disease accompanied by a disorder in retinal cells or a retinal tissue, wherein the drug comprises a retinal tissue being of an allogeneic origin and having a three-dimensional structure,
wherein an intended patient to be given the therapeutic drug is a patient affected by a disease accompanied by a disorder of retinal cells or a retinal tissue, to be given systemically no immunosuppressive agent aimed at preventing graft rejection for 1 month or more after the administration of the therapeutic drug.

2. The therapeutic drug according to claim 1, wherein the intended patient is a patient to be given systemically no immunosuppressive agent aimed at preventing graft rejection before, during, and/or after the administration of the therapeutic drug.

3. The therapeutic drug according to claim 1 or 2, wherein the intended patient is a patient to be given locally no immunosuppressive agent aimed at preventing graft rejection, other than a steroidal anti-inflammatory drug and a calcineurin inhibitor before, during, and/or after the administration of the therapeutic drug.

4. The therapeutic drug according to any one of claims 1 to 3, wherein the intended patient is a patient to be given locally one or more immunosuppressive agents selected from the group consisting of triamcinolone, fluocinolone, and a calcineurin inhibitor, aimed at preventing graft rejection before, during, and/or after the administration of the therapeutic drug,.

5. The therapeutic drug according to claim 3, wherein the intended patient is a patient to be given locally none of a steroidal anti-inflammatory drug and/or a calcineurin inhibitor, aimed at preventing graft rejection at least 1 month after the administration of the therapeutic drug.

6. The therapeutic drug according to claim 1, wherein the intended patient is a patient to be given no immunosuppressive agent aimed at preventing graft rejection before, during, and/or after the administration of the therapeutic drug.

7. The therapeutic drug according to any one of claims 1 to 6, wherein the therapeutic drug comprises substantially no retinal pigment epithelial cells.

8. The therapeutic drug according to any one of claims 1 to 7, wherein TGFβ is present at least on a surface of the retinal tissue.

9. The therapeutic drug according to any one of claims 1 to 8, wherein a diameter in the major axis direction of the retinal tissue is 0.2 mm or more.

10. The therapeutic drug according to any one of claims 1 to 9, wherein a total number of cells contained in a piece of the retinal tissue is 1 × 10⁴ cells or more.

11. The therapeutic drug according to any one of claims 1 to 10, wherein the surface of the retinal tissue has 50% or more by area of a continuous epithelial structure.

12. The therapeutic drug according to any one of claims 1 to 11, wherein 50% or more of the total number of cells contained in a piece of the retinal tissue are cells expressing at least one of PAX6, Chx10, and Crx.

13. The therapeutic drug according to any one of claims 1 to 12, wherein the retinal tissue is derived from a pluripotent stem cell.
